# EUROPEAN PATENT APPLICATION

(11) **EP 3 725 777 A1**
(43) Date of publication of application: **21.10.2020**
(21) Application number: 19169769.7
(22) Date of filing: 17.04.2019
(51) Int. Cl.: C07D 231/56, C07D 471/04, A61K 31/416, A61K 31/4162, A61P 35/00, C07D 401/10, C07D 401/14

(54) **BENZO- AND PYRIDO-PYRAZOLES AS PROTEIN KINASE INHIBITORS**

(71) Applicant: Rottapharm Biotech S.r.l., 20900 Monza (IT)
(72) Inventor: ROVATI, Lucio Claudio, 20900 MONZA (IT); ARTUSI, Roberto, 20017 RHO (IT); BOVINO, Clara, 20060 GESSATE (IT); MANDELLI, Stefano, 23880 CASATENOVO (IT); COLACE, Fabrizio, 20052 MONZA (IT); BUZZI, Benedetta, 20821 MEDA (IT); PIEPOLI, Tiziana, 20137 MILANO (IT)
(74) Representative: Cattaneo, Elisabetta

(57) **Abstract**

The invention relates to new chemical compounds of Formula (I), pharmaceutical compositions containing them, and their use for the pharmacological treatment of a cancer, preferably a glioblastoma.

## Description

### FIELD OF THE INVENTION

The present invention relates to new chemical compounds which modulates the activity of certain protein tyrosine kinases and methods of making and using the same. The compounds of this invention are therefore useful for treating diseases caused by deregulated protein tyrosine kinases, in general to the field of cancers, more particularly to CNS cancers, preferably glioblastoma.

### BACKGROUND OF THE INVENTION

The present invention relates to compounds which inhibit, regulate and/or modulate protein tyrosine kinases (PTKs) signal transduction, compositions which contain these compounds, and methods of using them to treat PTK-dependent diseases and conditions such as angiogenesis, tumour growth and migration.

PTKs are a class of proteins with tyrosine kinase activity that catalyses the transfer of phosphate groups on ATP to the tyrosine residues of many important proteins, the phosphorylated proteins transfer signals to regulate cell growth, differentiation, death and a series of biochemical processes. Tyrosine kinases abnormal expression/activation is also associated with tumour invasion and metastasis, tumour neovascularization and tumour resistance to chemotherapy (Jiao Q et al, Mol Cancer. 2018).

PTKs can be divided in two categories, as receptor type or non-receptor type.

Receptor type tyrosine kinases have an extracellular, a transmembrane, and an intracellular portion, while non-receptor type tyrosine kinases are wholly intracellular. In particular EGFR, a member of the Erb family (a group of four tyrosine kinases sharing similarities in structures and functions: ErbB1 (EGFR or HER1), ErbB2 (HER2), ErbB3 (HER3), and ErbB4 (HER4)), is a cell surface receptor and plays a pivotal role in regulating survival and apoptosis of tumours. Alteration or overexpression of EGFR and its ligands is present in a variety of epithelial tumour cells such as lung, breast, bladder and prostate cancer, squamous cell carcinoma of the head and neck and glioblastoma (Brennan CW et al. Cell 2013).

Another receptor tyrosine kinase with a key role in angiogenesis is KDR, which is also known as vascular endothelial growth factor 2 or VEGFR2, since it binds VEGF with high affinity. VEGF and KDR play an important role in the proliferation of vascular endothelial cells, and the formation and sprouting of blood vessels (vasculogenesis and angiogenesis). Inhibiting KDR modulates the level of mitogenic VEGF activity. In fact, tumour growth has been shown to be susceptible to the angiogenic effect of VEGF receptor antagonists.

The non-receptor type of tyrosine kinase is formed by numerous subfamilies, including Src. The Src subfamily is one of the largest and includes Src, Fyn, Yes, Lck, Lyn, Blk, Hck, Fgr, Frk, Srm and Yrk. Some Src subfamily members has been linked to oncogenesis. Fyn and Yes for example contributes to the development and progression of several cancer types through its involvement in the control of cell growth, death and cellular motility. Enhanced expression and/or activation is observed in different cancers, including melanoma, glioblastoma, squamous cell carcinoma of the head and neck, breast and prostate cancer. Moreover recently Fyn role in the resistance or susceptibility of cancer cells to some anti-cancer treatment was demonstrated (Elias D et al, Pharmacol Res. 2015).

Accordingly, the identification of small compounds which specifically inhibit, regulate and/or modulate the signal transduction of PTK is desirable and is an object of this invention.

### SUMMARY OF THE INVENTION

The objects above indicated have been achieved by a compound of Formula (I): 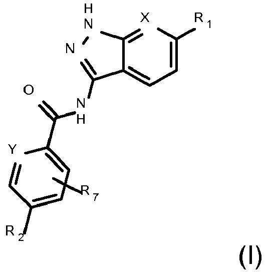 or a salt thereof,
wherein:
X is C or N;
Y is C or N;
R₁ is a substituent selected from the group consisting of: 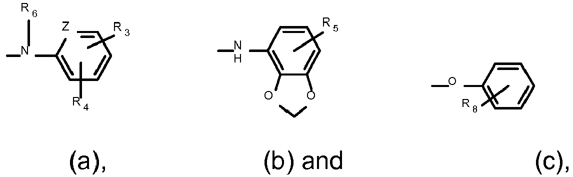
Where
Z is C or N;
R₃ and R₄ are, independently from each other, selected from the group consisting of hydrogen, fluorine, chlorine, cyano, (C₁-C₃)alkyl, (C₁-C₃)alkoxy and trifluoromethyl,
R₅ is hydrogen or fluorine,
R₆ is hydrogen or (C₁-C₃)alkyl,
R₈ is hydrogen or fluorine,
R₂ is a heterocyclic substituent selected from the group consisting of:
   piperidinyl, piperazinyl, morpholinyl, 4-piperidinyloxy and 3-piperidinyloxy wherein said heterocyclic substituent is
      optionally N-substituted with a substituent independently selected from (C₁-C₃) alkyl, acetyl, 2-methoxyethyl and 3-oxethanyl, and
   optionally C-substituted with one or more fluorine;
   R₇ is H, 4-tetrahydropyranylamino or 3-oxethanylmethylenoxy.

The present invention is further directed to a compound or a salt, particularly a pharmaceutically acceptable salt, thereof or the use in the inhibition of at least one PTK (protein tyrosine kinase). The compounds of the invention are inhibitors of PTKs and may be useful for the treatment of PTKs-mediated diseases and disorders. Therefore, in another aspect the invention relates to the compound of the invention for use in the treatment of PTKs-mediated diseases and disorders.

Among the mediated diseases and disorders the following can be cited:
- Primary brain tumors including, but not limited to, astrocytic and oligodendroglia tumors such as astrocytomas and glioblastomas;
- Nerve tissue tumors of infancy such as neuroblastomas;
- Metastatic brain tumors including, but not limited to, lung cancer, breast cancer, melanoma metastases, colon/colorectal metastases, kidney/renal metastases;
- Solid tumors including, but not limited to, primary, recurrent or metastatic, progressive thyroid, breast, liver, bladder and kidney cancers;
- Inflammatory diseases including, but not limited to, rheumatoid arthritis, conjunctivitis, rheumatoid spondylitis, osteoarthritis, gouty arthritis and other arthritic conditions;
- Central Nervous System degenerative diseases including, but not limited to, Alzheimer's Disease (AD), prodromal Alzheimer's Disease, amnestic mild cognitive impairment (MCI), Down syndrome dementia, traumatic brain injury, Lewy body dementia, Parkinson's Disease with dementia, frontotemporal dementia;
- Viral infection of the SNC including, but not limited to, cytomegalovirus, hepatitis virus, influenza virus, HIV1, West Nile virus infections.

The at least one PTK (protein tyrosine kinase) is preferably selected from the group consisting of FYN, EGFR, VEGFR2 (KDR) and YES1.

In a preferred aspect, the PTKs-mediated disease is a tumour, preferably glioblastoma. In a preferred and advantageous aspect the invention relates to a compound of Formula (I) or a salt thereof for use in treating glioblastoma.

Accordingly, the invention is further directed to a method of treating a PTKs-mediated disease or condition in a patient which comprises administering to the patient a therapeutically effective amount of a compound according to Formula (I), or a pharmaceutically acceptable salt thereof.

"Treating" or "treatment" is intended to mean at least the mitigation of a disease condition in a patient. The methods of treatment for mitigation of a disease condition include the use of the compounds in this invention in any conventionally acceptable manner, for example for prevention, retardation, prophylaxis, therapy or cure of a mediated disease.

The present invention is also directed to a pharmaceutical composition comprising a compound of the invention or its salt and a pharmaceutically acceptable carrier.

As used herein, "pharmaceutically acceptable excipient" means a material, composition or vehicle involved in giving form or consistency to the composition.

### DESCRIPTION OF THE FIGURES

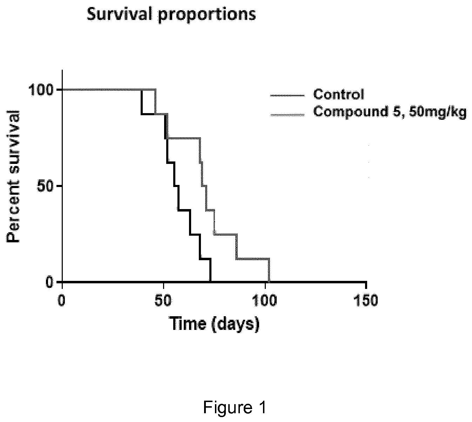

Figure 1 reports survival data in U87MG xenograft model after administration of compound 5.

### DETAILED DESCRIPTION OF THE INVENTION

The invention relates to a compound of Formula (I): 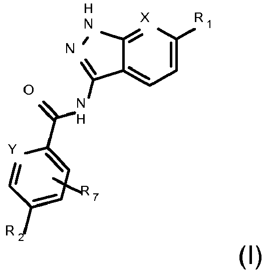 or a salt thereof,
wherein:
X is C or N;
Y is C or N;
R₁ is a substituent selected from the group consisting of: 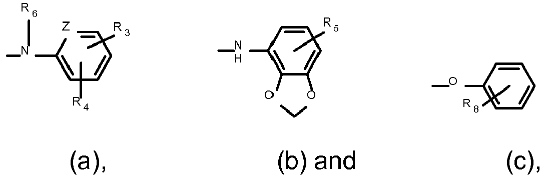
Where
Z is C or N;
R₃ and R₄ are, independently from each other, selected from the group consisting of hydrogen, fluorine, chlorine, cyano, (C₁-C₃)alkyl, (C₁-C₃)alkoxy and trifluoromethyl, R₅ is hydrogen or fluorine,
R₆ is hydrogen or (C₁-C₃)alkyl,
R₈ is hydrogen or fluorine,
R₂ is a heterocyclic substituent selected from the group consisting of:
   piperidinyl, piperazinyl, morpholinyl, 4-piperidinyloxy and 3-piperidinyloxy wherein said heterocyclic substituent is
      optionally N-substituted with a substituent independently selected from (C₁-C₃) alkyl, acetyl, 2-methoxyethyl and 3-oxethanyl, and
   optionally C-substituted with one or more fluorine;
   R₇ is H, 4-tetrahydropyranylamino or 3-oxethanylmethylenoxy.

The alternative definitions for the various groups and substituent groups of Formula (I) provided throughout the specification are intended to particularly describe each compound species disclosed herein, individually, as well as groups of one or more compound species. The scope of this invention includes any combination of these group and substituent group definitions.

The compounds according to Formula (I) may contain one or more asymmetric centre (also referred to as a chiral centre) and may, therefore, exist as individual enantiomers, diastereomers, or other stereoisomeric forms, or as mixtures thereof. Chiral centres, such as a chiral carbon may also be present in the compounds of this invention. Where the stereochemistry of a chiral centre present in a compound of this invention, or in any chemical structure illustrated herein, is not specified the structure is intended to encompass all individual stereoisomers and all mixtures thereof. Thus, compounds according to Formula (I) containing one or more chiral centre may be used as racemic mixtures, enantiomerically enriched mixtures, or as enantiomerically pure individual stereoisomers.

It is to be understood that a solid form of a compound of the invention may exist in crystalline forms, non-crystalline forms or a mixture thereof. Such crystalline forms may also exhibit polymorphism (i.e. the capacity to occur in different crystalline forms). These different crystalline forms are typically known as "polymorphs." Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, which may be used for identification. One of ordinary skill in the art will appreciate that different polymorphs may be produced, for example, by changing or adjusting the conditions used in crystallizing/re-crystallizing the compound.

In addition, the compounds of this invention, depending on further substitution, may exist in other tautomeric forms. All tautomeric forms of the compounds described herein are intended to be encompassed within the scope of the present invention. It is to be understood that any reference to a named compound of this invention is intended to encompass all tautomers of the named compound and any mixtures of tautomers of the named compound.

As used herein, the terms "compound(s) of the invention" or "compound(s) of this invention" mean a compound of Formula (I), as defined above, in any form, i.e., any salt or non-salt form (e.g., as a free acid or base form, or as a salt, particularly a pharmaceutically acceptable salt thereof) and any physical form thereof (e.g., including non-solid forms (e.g., liquid or semi-solid forms), and solid forms (e.g., amorphous or crystalline forms, specific polymorphic forms, solvate forms, including hydrate forms (e.g., mono-, di- and hemi- hydrates)), and mixtures of various forms.

As used herein, the term "optionally substituted" means unsubstituted groups or rings and groups or rings substituted with one or more specified substituents.

As used herein, the term "(C₁-C₃)alkyl" or "(C₁-C₃)alkoxy" refers to linear or branched (C₁-C₃)alkyl or "(C₁-C₃)alkoxy, respectively.

R₁ is a substituent selected from the group consisting of: 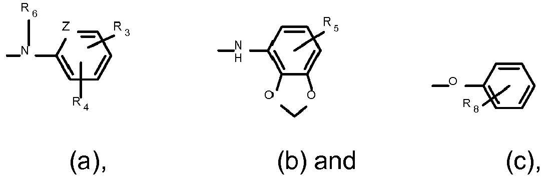 Where
Z is C or N;
R₃ and R₄ are, independently from each other, selected from the group consisting of hydrogen, fluorine, chlorine, cyano, (C₁-C₃)alkyl, (C₁-C₃)alkoxy and trifluoromethyl, R₅ is hydrogen or fluorine,
R₆ is hydrogen or (C₁-C₃)alkyl,
R₈ is hydrogen or fluorine,
R₁ is preferably the substituent (a) wherein Z is carbon or the substituent (b), more preferably the substituent (a) wherein Z is carbon.
When R₁ is the substituent (a), wherein Z is C, preferably R₃ and R₄ are fluorine or chlorine, more preferably fluorine.
When R₁ is the substituent (b) preferably R₅ is fluorine.
R₂ is a heterocyclic substituent selected from the group consisting of: piperidinyl, piperazinyl, morpholinyl, 4-piperidinyloxy and 3-piperidinyloxy, wherein said heterocyclic substituent is optionally N-substituted with a substituent independently selected from (C₁-C₃) alkyl, acetyl, 2-methoxyethyl and 3-oxethanyl, and optionally C-substituted with one or more fluorine.
R₂ is preferably piperidinyl, piperazinyl or 4-piperidinyloxy, more preferably piperidinyl. When R₂ is N-substituted, it is preferably substituted with (C₁-C₃)alkyl.

A compound of the invention includes a compound of Formula (I), or a salt thereof, particularly a pharmaceutically acceptable salt thereof.

Preferably the compound of the invention is selected from the group consisting of:
N-(6-((2-chloro-6-fluorophenyl)amino)-1H-indazol-3-yl)-4-(1-methylpiperidin-4-yl) benzamide;
N-(6-((2-ethyl-6-fluorophenyl)amino)-1H-indazol-3-yl)-4-(1-methylpiperidin-4-yl) benzamide;
4-((3S,4R)-3-fluoro-1-methylpiperidin-4-yl)-N-(6-((2-fluorophenyl)amino)-1H-indazol-3-yl)benzamide;
4-((3R,4S)-3-fluoro-1-methylpiperidin-4-yl)-N-(6-((2-fluorophenyl)amino)-1H-indazol-3-yl)benzamide;
N-(6-((2-fluorophenyl)amino)-1H-pyrazolo[3,4-b]pyridin-3-yl)-4-(1-methylpiperidin-4-yl)benzamide;
N-(6-((4-cyano-2-fluorophenyl)amino)-1H-indazol-3-yl)-4-(1-methylpiperidin-4-yl) benzamide;
N-(6-((2-fluorophenyl)amino)-1H-indazol-3-yl)-4-(1-(oxetan-3-yl)piperidin-4-yl) benzamide;
N-(6-((2-fluoro-4-methylphenyl)amino)-1H-indazol-3-yl)-4-(1-methylpiperidin-4-yl) benzamide;
N-(6-((2-chloro-4-fluorophenyl)amino)-1H-indazol-3-yl)-4-(1-methylpiperidin-4-yl) benzamide;
4-(1-methylpiperidin-4-yl)-N-(6-((2-(trifluoromethyl)phenyl)amino)-1H-indazol-3-yl) benzamide;
N-(6-((2-fluoro-6-methylphenyl)amino)-1H-indazol-3-yl)-4-(1-methylpiperidin-4-yl) benzamide;
N-(6-((4-chloro-2-fluorophenyl)amino)-1H-indazol-3-yl)-4-(1-methylpiperidin-4-yl) benzamide;
N-(6-((2-chloro-6-methylphenyl)amino)-1H-indazol-3-yl)-4-(1-methylpiperidin-4-yl) benzamide;
N-(6-((2-chlorophenyl)amino)-1H-indazol-3-yl)-4-(1-methylpiperidin-4-yl)benzamide;
N-(6-((5-fluorobenzo[d][1,3]dioxol-4-yl)amino)-1H-indazol-3-yl)-4-(1-methylpiperidin-4-yl)benzamide;
N-(6-((2,4-difluorophenyl)amino)-1H-indazol-3-yl)-4-(1-methylpiperidin-4-yl) benzamide;
N-(6-((2-fluorophenyl)amino)-1 H-indazol-3-yl)-4-(1-methylpiperidin-4-yl)benzamide; 4-(1-methylpiperidin-4-yl)-N-(6-(p-tolylamino)-1H-indazol-3-yl)benzamide;
N-(6-((3-fluorophenyl)amino)-1 H-indazol-3-yl)-4-(1-methylpiperidin-4-yl)benzamide;
N-(6-((2,3-difluorophenyl)amino)-1H-pyrazolo[3,4-b]pyridin-3-yl)-4-(1-methylpiperidin-4-yl)benzamide;
N-(6-((2-fluoro-6-methylphenyl)amino)-1H-pyrazolo[3,4-b]pyridin-3-yl)-4-(1-methylpiperidin-4-yl)benzamide;
N-(6-((2,4-difluorophenyl)amino)-1H-pyrazolo[3,4-b]pyridin-3-yl)-4-(1-methylpiperidin-4-yl)benzamide;
4-(1-methylpiperidin-4-yl)-N-(6-(p-tolylamino)-1H-pyrazolo[3,4-b]pyridin-3-yl) benzamide;
N-(6-((2-fluorophenyl)amino)-1H-pyrazolo[3,4-b]pyridin-3-yl)-4-(1-isopropylpiperidin-4-yl)benzamide;
N-(6-((2-fluorophenyl)amino)-1H-pyrazolo[3,4-b]pyridin-3-yl)-4-(1-(2-methoxyethyl) piperidin-4-yl)benzamide;
N-(6-((2-chlorophenyl)amino)-1 H-pyrazolo[3,4-b]pyridin-3-yl)-4-(1-methylpiperidin-4-yl)benzamide;
N-(6-((2-methoxyphenyl)amino)-1H-pyrazolo[3,4-b]pyridin-3-yl)-4-(1-methylpiperidin-4-yl)benzamide;
N-(6-(benzo[d][1,3]dioxol-5-ylamino)-1H-pyrazolo[3,4-b]pyridin-3-yl)-4-(1-methylpiperidin-4-yl)benzamide;
4-(1-methylpiperidin-4-yl)-N-(6-(phenylamino)-1H-pyrazolo[3,4-b]pyridin-3-yl) benzamide;
N-(6-((2-fluoro-4-methylphenyl)amino)-1H-pyrazolo[3,4-b]pyridin-3-yl)-4-(1-methylpiperidin-4-yl)benzamide;
N-(6-((4-methoxyphenyl)amino)-1H-pyrazolo[3,4-b]pyridin-3-yl)-4-(1-methylpiperidin-4-yl)benzamide;
N-(6-((4-cyanophenyl)amino)-1H-pyrazolo[3,4-b]pyridin-3-yl)-4-(1-methylpiperidin-4-yl)benzamide;
N-(6-((3-fluoro-4-methoxyphenyl)amino)-1H-pyrazolo[3,4-b]pyridin-3-yl)-4-(1-methylpiperidin-4-yl)benzamide;
N-(6-((2-fluorophenyl)amino)-1H-indazol-3-yl)-4-(4-methylpiperazin-1-yl)benzamide; N-(6-((2-fluorophenyl)amino)-1H-indazol-3-yl)-4-((1-methylpiperidin-4-yl)oxy) benzamide;
N-(6-((2-fluorophenyl)amino)-1H-indazol-3-yl)-4-morpholinobenzamide;
N-(6-(2-fluorophenoxy)-1H-indazol-3-yl)-4-((1-methylpiperidin-4-yl)oxy)benzamide;
N-(6-((2-fluorophenyl)amino)-1H-indazol-3-yl)-4-((1-methylpiperidin-3-yl)oxy)benzamide;
N-(6-((2-fluorophenyl)amino)-1H-indazol-3-yl)-5-(1-methylpiperidin-4-yl)picolinamide;
rel-(R)-N-(6-((2-fluorophenyl)amino)-1H-indazol-3-yl)-4-((1-methylpiperidin-3-yl)oxy) benzamide;
rel-(S)-N-(6-((2-fluorophenyl)amino)-1H-indazol-3-yl)-4-((1-methylpiperidin-3-yl)oxy) benzamide;
N-(6-((2-fluorophenyl)amino)-1H-indazol-3-yl)-4-(1-methylpiperidin-4-yl)-3-(oxetan-3-ylmethoxy)benzamide;
N-(6-((3,5-difluoropyridin-2-yl)amino)-1H-pyrazolo[3,4-b]pyridin-3-yl)-4-(1-methylpiperidin-4-yl)benzamide;
N-(6-((2,5-difluorophenyl)amino)-1H-pyrazolo[3,4-b]pyridin-3-yl)-4-(1-methylpiperidin-4-yl)benzamide;
rel-(R)-4-(3,3-difluoro-1-methylpiperidin-4-yl)-N-(6-((2-fluorophenyl)amino)-1 H-pyrazolo[3,4-b]pyridin-3-yl)benzamide;
rel-(S)-4-(3,3-difluoro-1-methylpiperidin-4-yl)-N-(6-((2-fluorophenyl)amino)-1 H-pyrazolo[3,4-b]pyridin-3-yl)benzamide;
N-(6-((2-fluorophenyl)amino)-1H-pyrazolo[3,4-b]pyridin-3-yl)-4-((1-methylpiperidin-4-yl)oxy)benzamide;
N-(6-((2-fluorophenyl)amino)-1H-pyrazolo[3,4-b]pyridin-3-yl)-4-(4-methylpiperazin-1-yl)benzamide;
N-(6-((5-fluorobenzo[d][1,3]dioxol-4-yl)amino)-1H-pyrazolo[3,4-b]pyridin-3-yl)-4-(1-methylpiperidin-4-yl)benzamide;
N-(6-((2-fluorophenyl)(methyl)amino)-1H-pyrazolo[3,4-b]pyridin-3-yl)-4-(1-methylpiperidin-4-yl)benzamide;
N-(6-((3,5-difluorophenyl)amino)-1H-pyrazolo[3,4-b]pyridin-3-yl)-4-(1-methylpiperidin-4-yl)benzamide;
4-(4-acetylpiperazin-1-yl)-N-(6-((2-fluorophenyl)amino)-1H-pyrazolo[3,4-b]pyridin-3-yl)benzamide, and
N-(6-((2-fluorophenyl)amino)-1H-indazol-3-yl)-4-(1-methylpiperidin-4-yl)-2-((tetrahydro-2H-pyran-4-yl)amino)benzamide.
Preferred compounds of the invention are selected from the group consisting of:
N-(6-((2-chloro-6-fluorophenyl)amino)-1H-indazol-3-yl)-4-(1-methylpiperidin-4-yl) benzamide,
N-(6-((2-fluorophenyl)amino)-1H-pyrazolo[3,4-b]pyridin-3-yl)-4-(1-methylpiperidin-4-yl)benzamide,
N-(6-((2,3-difluorophenyl)amino)-1H-pyrazolo[3,4-b]pyridin-3-yl)-4-(1-methylpiperidin-4-yl)benzamide,
N-(6-(benzo[d][1,3]dioxol-5-ylamino)-1H-pyrazolo[3,4-b]pyridin-3-yl)-4-(1-methylpiperidin-4-yl)benzamide,
N-(6-((2-fluoro-4-methylphenyl)amino)-1H-pyrazolo[3,4-b]pyridin-3-yl)-4-(1-methylpiperidin-4-yl)benzamide,
N-(6-((2-fluorophenyl)amino)-1H-pyrazolo[3,4-b]pyridin-3-yl)-4-((1-methylpiperidin-4-yl)oxy)benzamide, and
4-(1-methylpiperidin-4-yl)-N-(6-(p-tolylamino)-1H-pyrazolo[3,4-b]pyridin-3-yl) benzamide.

Because the compounds of the invention contain basic moieties, a desired salt form may be prepared by any suitable method known in the art, including treatment of the free base with an inorganic acid, selected from the group consisting of hydrochloric acid, hydrobromic acid, sulphuric acid, nitric acid, phosphoric acid, and the like, or with an organic acid selected from the group consisting of maleic acid, succinic acid, mandelic acid, fumaric acid, malonic acid, salicylic acid, citric acid, tartaric acid, p-toluensulfonic acid.

Because of their potential use in medicine, the salts of the compounds of Formula (I) are preferably pharmaceutically acceptable salts. Salts encompassed within the term "pharmaceutically acceptable salts" refer to non-toxic salts of the compounds of this invention.

The present invention is also directed to a pharmaceutical composition comprising a compound of the invention or its salt and a pharmaceutically acceptable carrier.

The invention relates also to a pharmaceutical compound of Formula (I) or its salt for use as a medicament.

The compounds of the invention may be administered by any suitable route of administration, including both systemic administration and topical administration. Systemic administration includes oral administration, parenteral administration, trans-dermal administration, rectal administration, and administration by inhalation.

The compounds of the invention may be administered once or according to a dosing regimen wherein a number of doses are administered at varying intervals of time for a given period of time. For example, doses may be administered one, two, three, or four times per day. Doses may be administered until the desired therapeutic effect is achieved or indefinitely to maintain the desired therapeutic effect. Suitable dosing regimens for a compound of the invention depend on the pharmacokinetic properties of that compound, such as absorption, distribution, and half-life, which can be determined by the skilled artisan. In addition, suitable dosing regimens, including the duration such regimens are administered, for a compound of the invention depend on the condition being treated, the severity of the condition being treated, the age and physical condition of the patient being treated, the medical history of the patient to be treated, the nature of concurrent therapy, the desired therapeutic effect, and like factors within the knowledge and expertise of the skilled artisan.

The compounds of the invention will be normally, but not necessarily, formulated into a pharmaceutical composition prior to administration to a patient. The pharmaceutical compositions of the invention are prepared using techniques and methods known to those skilled in the art.

The pharmaceutical compositions of the invention may be prepared and packaged in bulk form wherein an effective amount of a compound of the invention can be extracted and then given to the patient such as with powders, syrups, and solutions for injection. Alternatively, the pharmaceutical compositions of the invention may be prepared and packaged in unit dosage form. A dose of the pharmaceutical composition contains at least a therapeutically effective amount of a compound of this invention (i.e., a compound of Formula (I), or a salt, particularly a pharmaceutically acceptable salt, thereof). When prepared in unit dosage form, the pharmaceutical compositions may contain from 1 mg to 1000 mg of a compound of this invention.

The compounds of the invention and the pharmaceutically acceptable excipient or excipients will typically be formulated into a dosage form adapted for administration to the patient by the desired route of administration.

Conventional dosage forms include those adapted for (1) oral administration such as tablets, capsules, caplets, pills, troches, powders, syrups, elixirs, suspensions, solutions, emulsions, sachets, and cachets; (2) parenteral administration such as sterile solutions, suspensions, and powders for reconstitution; (3) trans-dermal administration such as trans-dermal patches; (4) rectal administration such as suppositories; (5) inhalation such as aerosols and solutions; and (6) topical administration such as creams, ointments, lotions, pastes, sprays and gels.

Suitable pharmaceutically acceptable excipients include the following types of excipients: diluents, fillers, binders, disintegrants, lubricants, granulating agents, coating agents, wetting agents, suspending agents, emulsifiers, sweeteners,, flavour masking agents, colouring agents, anti-caking agents, humectants, plasticizers, viscosity increasing agents, antioxidants, preservatives, stabilizers, surfactants, and buffering agents. Suitable diluents and fillers include lactose, sucrose, dextrose, mannitol, sorbitol, starch, cellulose, calcium sulphate, and dibasic calcium phosphate. The oral solid dosage form may further comprise a binder. Suitable binders include starch, gelatine, sodium alginate, alginic acid, guar gum, povidone, and cellulose and its derivatives (e.g. microcrystalline cellulose). The oral solid dosage form may further comprise a disintegrant. Suitable disintegrants include crospovidone, sodium starch glycolate, alginic acid, and sodium carboxymethyl cellulose. The oral solid dosage form may further comprise a lubricant. Suitable lubricants include stearic acid, magnesium stearate, calcium stearate, and talc. Suitable carriers for oral dosage forms include but are not limited to magnesium carbonate, magnesium stearate, talc, lactose, pectin, dextrin, starch, methylcellulose, sodium carboxymethyl cellulose, and the like. Techniques used to prepare oral formulations are the conventional mixing, granulation and compression or capsules filling.

The compounds of the present invention may be also formulated for parenteral administration with suitable carriers including aqueous vehicles solutions (i.e.: saline, dextrose) or and/or oily emulsions.

In a still further aspect the invention relates to a compound or a salt, particularly a pharmaceutically acceptable salt, for the use in the inhibition of at least one PTK (protein tyrosine kinase).

The at least one PTK (protein tyrosine kinase) is preferably selected from the group consisting of FYN, EGFR, VEGFR2 (KDR) and YES1.

The compounds of the invention are inhibitors of PTKs and may be useful for the treatment of PTKs-mediated diseases and disorders.

Therefore, in another aspect the invention relates to the compound of the invention for use in the treatment of PTKs-mediated diseases and disorders.

Among the mediated diseases and disorders the following can be cited:
- Primary brain tumors including, but not limited to, astrocytic and oligodendroglia tumors such as astrocytomas and glioblastomas;
- Nerve tissue tumors of infancy such as neuroblastomas;
- Metastatic brain tumors including, but not limited to, lung cancer, breast cancer, melanoma metastases, colon/colorectal metastases, kidney/renal metastases;
- Solid tumors including, but not limited to, primary, recurrent or metastatic, progressive thyroid, breast, liver, bladder and kidney cancers;
- Inflammatory diseases including, but not limited to, rheumatoid arthritis, conjunctivitis, rheumatoid spondylitis, osteoarthritis, gouty arthritis and other arthritic conditions;
- Central Nervous System degenerative diseases including, but not limited to, Alzheimer's Disease (AD), prodromal Alzheimer's Disease, amnestic mild cognitive impairment (MCI), Down syndrome dementia, traumatic brain injury, Lewy body dementia, Parkinson's Disease with dementia, frontotemporal dementia; and
- Viral infection of the SNC including, but not limited to, cytomegalovirus, hepatitis virus, influenza virus, HIV1, West Nile virus infections.

In a preferred aspect the PTKs-mediated disease is a tumour, preferably glioblastoma. In a preferred and advantageous aspect, the invention relates to a compound of Formula (I) or a salt thereof for use in treating glioblastoma.

Accordingly, the invention is further directed to a method of treating a PTKs-mediated disease or condition in a patient which comprises administering to the patient a therapeutically effective amount of a compound according to Formula (I), or a pharmaceutically acceptable salt thereof.

A therapeutically "effective amount" is intended to mean that amount of a compound that, when administered to a patient in need of such treatment, is sufficient to effect treatment, as defined herein. Thus, e.g., a therapeutically effective amount of a compound of Formula (I), or a pharmaceutically acceptable salt thereof, is a quantity of an inventive agent that, when administered to a human in need thereof, is sufficient to modulate or inhibit the activity of PTKs kinase such that a disease condition which is mediated by that activity is reduced, alleviated or prevented. The amount of a given compound that will correspond to such an amount will vary depending upon factors such as the particular compound (e.g., the potency (IC₅₀), efficacy (EC₅₀), and the biological half-life of the particular compound), disease condition and its severity, the identity (e.g., age, size and weight) of the patient in need of treatment, but can nevertheless be routinely determined by one skilled in the art. Likewise, the duration of treatment and the time period of administration (time period between dosages and the timing of the dosages, e.g., before/with/after meals) of the compound will vary according to the identity of the human in need of treatment (e.g., weight), the particular compound and its properties (e.g., pharmaceutical characteristics), disease or condition and its severity and the specific composition and method being used, but can nevertheless be determined by one of skill in the art.

As above stated, the compounds of the present invention may be administered orally or parenterally, in a pharmacological effective amount. For all methods of treatment herein discussed for the compounds of formula (I), the daily oral dosage regimen will preferably be from about 0.01 to about 1000 mg. It will also be recognized by one of skill in the art that the optimal quantity and spacing of individual dosages of a compound of formula (I) will be determined by the nature and extent of the condition being treated.

Accordingly, appropriate pharmaceutical composition of compounds of formula (I) or their pharmaceutically acceptable salts, optionally together with pharmaceutically acceptable carriers can be used for the treatment of tumours, specifically glioblastoma. General syntheses used for preparing compounds of Formula (I) are described in schemes 1, 2 and 3.

Steps of scheme 1 are reported below: 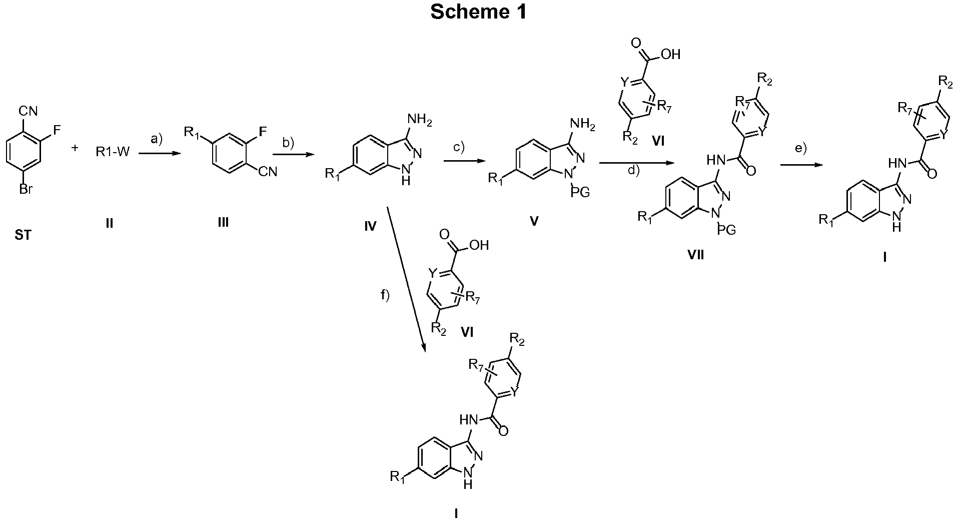 In the scheme 1 two synthetic pathways were followed to obtain compounds of formula (I) which are described in scheme 1.

### Pathway 1

a) Conversion of the starting material (ST) into compounds of formula (III), using compounds of formula (II), where R1-W is an aniline or phenol, in presence of organometallic catalyst, such as for example tris(dibenzylideneacetone) dipalladium(0);
b) Formation of the amino indazole derivatives (IV) using hydrazine in polar solvent such as for example ethanol;
c) Protection of the nitrogen on the ring with a known protecting group in presence of a catalytic amount of strong nucleophilic base such as for example DMAP;
d) Amide coupling between compounds of formula (V) and compounds of formula (VI) using either a coupling agent or via acid chloride formed *in situ;* and
e) Deprotection of the protecting group to obtain compounds of formula (I) where R₁, R₂, R₇, Y have the same meaning as in formula (I).

### Pathway 2

a) Conversion of the starting material (ST) into compounds of formula (III), using compounds of formula (II), where R1-W is an aniline or phenol, in presence of organometallic catalyst, such as for example tris(dibenzylideneacetone)-dipalladium(0);
b) Formation of the amino indazole derivatives (IV) using hydrazine in polar solvent such as for example ethanol; and
f) Amide coupling between compounds of formula (IV) and compounds of formula (VI) to obtain compounds of formula (I) where R₁, R₂, R₇, Y have the same meaning as in formula (I).

Steps of scheme 2 are reported below: 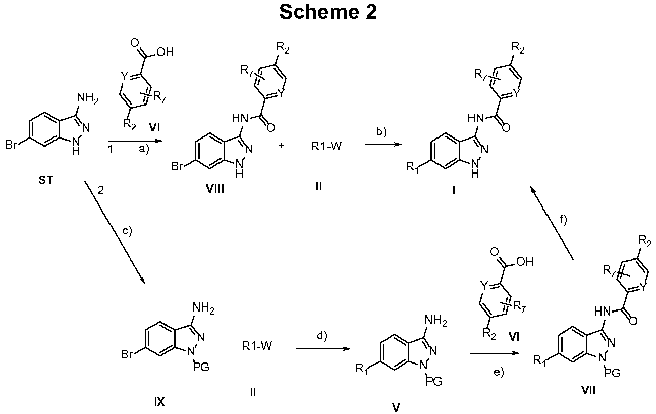 In scheme 2, two synthetic pathways are described from the starting (ST) bromo-indazole

### Pathway 1

a) Amide coupling between starting (ST) bromo-indazole and compounds of formula (VI) using either a coupling agent or via acid chloride formed *in situ*
b) Conversion compounds of formula (VIII) in compounds of formula (I), using compounds of formula (II) in presence of organometallic catalyst, such as for example tris(dibenzylideneacetone)dipalladium(0), where R₁, R₂, R₇, Y have the same meaning as in formula (I)

### Pathway 2

c) Protection of the nitrogen on the ring with a known protecting group in presence of a catalytic amount of strong nucleophilic base such as for example DMAP.
d) Conversion of compounds of formula (IX) in compounds of formula (V), using compounds of formula (II) in presence of organometallic catalyst, such as for example tris(dibenzylideneacetone)dipalladium(0)
e) Amide coupling between compounds of formula (V) and compounds of formula (VI) using either a coupling agent or via acid chloride formed *in situ*
f) Deprotection of the protecting group to obtain compounds of formula (I) where R₁, R₂, R₇, Y have the same meaning as in formula (I)

Another embodiment of the invention to obtain compound of formula (I) is described in scheme 3 below:

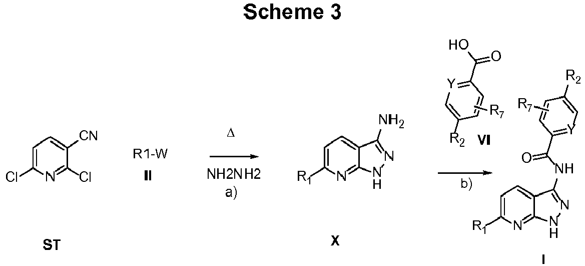

The process as outlined in scheme comprises the following steps.
a) Aromatic nucleophilic substitution of the starting material (ST) with a compound of formula (II) in presence of base at high temperature in a polar aprotic solvent as for example DMSO with subsequent condensation of the crude with hydrazine to obtain compounds of formula (X).
b) Amide coupling between compounds of formula (X) and compounds of formula (VI) to obtain compounds of formula (I) where R₁, R₂, R₇, Y have the same meaning as in formula (I).

The invention will be now detailed with reference to the preparative examples of the compounds of the invention and examples for testing the inhibitory activity with illustrative and not limitative purposes.

### Experimental part

Reagents used in the following examples were commercially available from various suppliers and used without further purifications. Solvents were used in dry form. Reactions in anhydrous environment were run under a positive pressure of dry N₂. Proton Nuclear Magnetic Resonance (¹H NMR) spectra were recorded on Bruker Avance 400 MHz instrument. Chemical shifts are reported in ppm (δ) using the residual solvent line as internal standard. Splitting patterns are designated as: s, singlet; d, doublet; t, triplet; q, quartet; m, multiplet; b, broad signal.

Mass spectra (MS) were run on a Ion Trap Thermo LCQ classic spectrometer, operating in positive ES(+) and negative ES(-) ionization mode.

UPLC spectra were performed on a Waters Acquity UPLC-SQD instrument using an Acquity UPLC-BEH C18 column (1.7µM, 50x2.1mm).

Chiral-HPLC spectra were performed using Agilent 1200 apparatus equipped UV-Vis detector.

Preparative HPLC was performed on Waters GX-281 HPLC system equipped with a UV-detector.

Flash silica gel chromatography was performed on Biotage automatic flash chromatography systems (Sp1 and Isolera systems) using Biotage SNAP HP silica cartridges or Biotage SNAP KP-NH cartridges.

Reverse phase chromatography was performed on Biotage automatic flash chromatography systems (Isolera systems) using RediSep Gold C-18Aq cartridges. Purifications of some basic compounds were performed using Phenomenex Strata SCX cartridges (55µm, 70A).

Thin layer chromatography was carried out using Merck TLC plates Kieselgel 60F-254, visualized with UV light, aqueous permanganate solution, iodine vapours.

The following abbreviations are used herein: AcOH: acetic acid; DIAD: diisopropyl (E)-diazene-1,2-dicarboxylate; Boc: terbutyloxycarbonyl; DCM: dichloromethane; DCE: 1,2-dichloroethane; TFA: trifluoroacetic acid; DMF: dimethylformamide; THF: tetrahydrofuran; RT: room temperature; DMAP: dimethylamino pyridine; AcOEt: ethyl acetate; NaOH: sodium hydroxyde; KOH: potassium hydroxyde; DIPEA: N,N-diisopropylethylamine; TEA: triethyl amine; NaHCO₃: sodium bicarbonate; Na₂SO₄: sodium sulphate; PdCl₂(PPh₃)₂: bis(triphenylphosphine)palladium(II)chloride. Cs₂CO₃: cesium carbonate

In the following schemes (4,5,6,7), the general synthetic pathways to obtain the intermediates (VI) of schemes 1, 2, 3 are described.

Scheme 4 for obtaining intermediates (VI) is reported below: 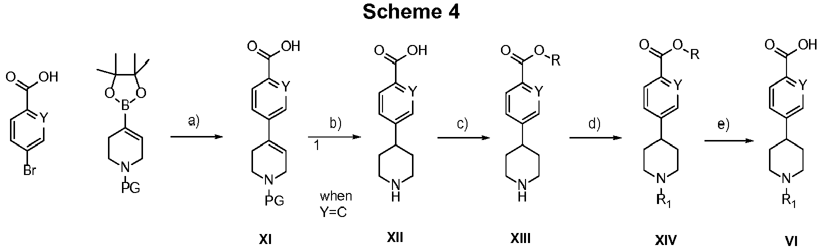 Scheme 4 provides for the following steps:
a) Conversion of starting materials in compounds of formula (XI) using organometallic catalyst such as bis(triphenylphosphine)palladium(II)chloride;
b) Reduction of double bond with a parallel removal of the protecting group of compounds (XI) to obtain compounds of formula (XII);
c) Protection of the carboxylic acid of compounds (XII) as ester such as for example methyl ester;
d) Alkylation of the nitrogen of compounds of formula (XIII) under reductive amination conditions using a known reducing agent such as for example sodium borohydride; and
e) Deprotection of the esters of compounds of formula (XIV) to obtain intermediates of formula (VI), using a strong base such as sodium hydroxide.

### Example 1:

### Preparation of Intermediate (XI) (intermediates 1 and 2) of scheme 4

### General procedure 1

A mixture of commercially available 4-bromobenzoate derivative (1 eq), benzyl 4-(4,4,5,5-tetramethyl-1,3,2-dioxaborolan-2-yl)-3,6-dihydropyridine-1(2H)-carboxylate (1.1 eq), PdCl₂(PPh₃)₂ (0.05 eq) and Na₂CO₃ (2 eq) in dioxane (ratio 6; 0.2 M) and water (ratio 1; 0.2M) was heated at 100 °C for 24h after that, the reaction was partitioned between water and AcOEt. The aqueous phase was separated, acidified with hydrochloric acid (2M) up to pH 2 and extracted with AcOEt (3 times), the organic phases were combined, dried over Na₂SO₄ and evaporated. The crude was purified by reverse phase chromatography eluted with water (0.1% of AcOH)/Acetonitrile (0.1% of AcOH) from 10/0 to 0/10 in gradient to afford title compounds.

**Table1**

| **Intermediates 1-2: Intermediate (XI) of scheme 4** | | | |
|---|---|---|---|
| **Intermediate** | **Structure** | **Analysis ¹HNMR/MS** | **Yields (%)** |
| 1 | 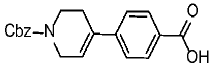 | ¹H NMR (400 MHz, *DMSO-d*6) δ ppm 12.88 (1 H, br. s.), 7.90 - 7.95 (2 H, m), 7.56 (2 H, d), 7.28 - 7.43 (5 H, m), 6.33 (1 H, br. s.), 5.13 (2 H, s), 4.13 (2 H, br. s.), 3.64 (2 H, br. s.), 2.52 - 2.57 (2 H, m) | 73 |
| | | ESI + m/z 338[M+H] | |
| 2 | 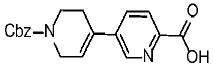 | ¹H NMR (400 MHz, *DMSO-d*6) δ ppm 13.11 (1 H, br. s.), 8.76 - 8.84 (1 H, m), 7.96 - 8.05 (2 H, m), 7.27- 7.44 (5 H, m), 6.46 (1 H, br. s.), 5.14 (2 H, s), 4.15 (2 H, br. s.), 3.66 (2 H, br. s.), 2.54 - 2.59 (2 H, m) | 84 |
| | | ESI + m/z 339[M+H] | |

### Example 2:

### Preparation of Intermediate (XII) (intermediates 3 and 4) of scheme 4

### General procedure 2

Examples **1 or 2** (1eq) and palladium hydroxide on carbon 20% in weight (0.2 eq) in MeOH (0.06M) was stirred under a constant pressure of H₂ at 2 bar upon completion. Water and HCl 2M were added and the catalyst filtered off, the filtrate was evaporated and the residue used as such without further purification.

**Table2**

| **Intermediates 3-4: Intermediate (XII) of scheme 4** | | | |
|---|---|---|---|
| **Intermediate** | **Structure** | **Analysis ¹HNMR/MS** | **Yields (%)** |
| 3 | 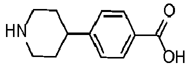 | ¹H NMR (400 MHz, *DMSO-d*6) δ ppm 12.83 (1 H, br. s.), 9.04 (1 H, br. s.), 7.89 - 7.94 (2 H, m), 7.36 (2H, d), 3.36 (2 H, d), 2.88 - 3.04 (3 H, m), 1.80 - 1.99 (4 H, m) | 78 |
| | | ESI + m/z 206[M+H] | |
| 4 | 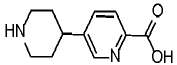 | ¹H NMR (400 MHz, *DMSO-d*6) δ ppm 8.72 (1 H, d), 8.09 (1 H, d), 7.85 (1 H, dd), 3.06 (2H, d), 2.70-2.80 (1 H, m), 2.64 (2 H, td), 1.68 - 1.75 (2 H, m), 1.48 (2 H, qd) | 80 |
| | | ESI + m/z 207[M+H] | |

### Example 3:

### Preparation of Intermediate (XIII) (intermediates 5 and 6) of scheme 4

### General procedure 3

Intermediate **3 or 4** (1 eq) and SOCl₂ (2-5 eq) in MeOH (0.05M) was stirred at RT upon completion. After that, volatiles were evaporated to afford title compounds

**Table3**

| **Intermediates 5-6:** | | | |
|---|---|---|---|
| **Intermediates** | **Structure** | **Analysis ¹HNMR/MS** | **Yields (%)** |
| 5 | 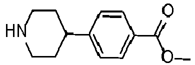 | ¹H NMR (400 MHz, *DMSO-d*6) δ ppm 8.75 - 9.14 (2 H, m), 7.90 - 8.00 (2 H, m), 7.39 (2 H, d), 3.85 (3H, s), 3.37 (2 H, d), 2.86 - 3.08 (3 H, m), 1.79 - 2.01 (4 H, m) | 100 |
| | | ESI + m/z 220[M+H] | |
| 6 | 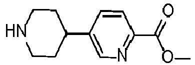 | ¹H NMR (400 MHz, *DMSO-d*6) δ ppm 8.61 (1 H, d), 8.00 (1 H, d), 7.84 (1 H, dd), 3.87 (3 H, s), 3.03 (2H, d), 2.69 - 2.78 (1 H, m), 2.59 (2 H, td), 1.68 - 1.75 (2 H, m), 1.54 (2 H, qd) | 89 |
| | | ESI + m/z 221 [M+H] | |

### Example 4:

### Preparation of Intermediate (XIV) (intermediates 7-10) of scheme 4

### General procedure 4

To a solution of intermediates **5 or 6** (1 eq) in DCM (0.05M), AcOH (3 eq), aldehydes or ketone (1.5) and sodium triacetoxyborohydride (2 eq) were added and the mixture was stirred at RT upon completion. NaHCO₃ was added and the phases separated, DCM was dried over Na₂SO₄, concentrated and the residue purified by flash chromatography eluting with a gradient DCM/MeOH to afford title compounds.

**Table 4**

| **Intermediates 7-10: Intermediate (XIV) of scheme 4** | | | |
|---|---|---|---|
| **Intermediates** | **Structure** | **Analysis ¹HNMR/MS** | **Yields (%)** |
| 7 | 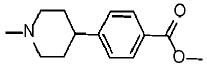 | ¹H NMR (400 MHz, *DMSO-d*6) δ ppm 7.89 (2 H, d), 7.40 (2 H, d), 3.84 (3 H, s), 2.83 - 2.97 (2 H, m),2.17 - 2.26 (3 H, m), 1.94 - 2.09 (2 H, m), 1.61 - 1.79 (4 H, m) | 90 |
| | | ESI + m/z 234 [M+H] | |
| 8 | 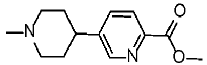 | ¹H NMR (400 MHz, *DMSO-d*6) δ ppm 8.63 (1 H, d), 8.00 (1 H, d), 7.84 (1 H, dd), 3.87 (3 H, s), 2.90 (2H, d), 2.69 - 2.78 (1 H, m), 2.21 (3 H, s), 2.00 - 1.98 (2 H, m), 1.68 (2 H, qd) | 94 |
| | | ESI + m/z 235 [M+H] | |
| 9 | 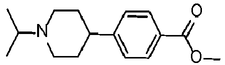 | ¹H NMR (400 MHz, *DMSO-d*6) δ ppm 7.85 - 7.92 (2 H, m), 7.40 (2 H, d), 3.81 - 3.86 (3 H, m), 2.89 (2H, d), 2.73 (1 H, dt), 2.54 - 2.60 (1 H, m), 2.23 (2 H, td), 1.73 -1.81 (2 H, m), 1.54- 1.69 (2 H, m), 1.00(6 H, d) | 71 |
| | | ESI + m/z 262 [M+H] | |
| 10 | 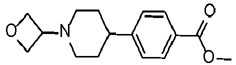 | ¹H NMR (400 MHz, *DMSO-d*6) δ ppm 7.87 - 7.93 (2 H, m), 7.42 (2 H, d), 4.52 - 4.58 (2 H, m), 4.42 -4.48 (2 H, m), 3.84 (3 H, s), 3.37 - 3.47 (1 H, m), 2.81 (2 H, d), 2.56 - 2.67 (1 H, m), 1.84 - 1.92 (2 H,m), 1.75 - 1.81 (2 H, m), 1.62 - 1.73 (2 H, m). | 79 |
| | | ESI + m/z 276 [M+H] | |

Scheme 5 for obtaining intermediate (VI) is reported below: 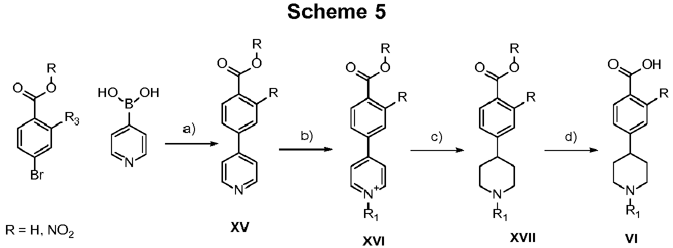 Scheme 5 provides for the following steps:
a) Conversion of starting materials in compounds of formula (XV) using an organometallic catalyst such as bis(triphenylphosphine)palladium(II)chloride;
b) Alkylation of the nitrogen of pyridine with an alkyl iodide as for example methyl iodide to obtain compounds of formula (XVI);
c) Reduction of compounds of formula (XVI) under a constant flow of hydrogen in presence of a catalyst as for example platinum (IV) oxide to give compounds of general formula (XVII); and
d) Ester-deprotection of compounds of formula (XVII) to give compounds of formula (VI).

### Example 5:

### Preparation of Intermediate (XV) (intermediate 11) of scheme 5

### tert-butyl 2-nitro-4-(pyridin-4-yl)benzoate

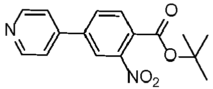

A mixture 4-bromo-2 nitrobenzoate (1g), 4 pyridinboronic acid (1.5 eq), Na₂CO₃ (4 eq) and tetrakis (0.04 eq) in 1,2-dimethoxyethane (40ml) and degassed water (20ml) was heated at 100 °C upon completion. The catalyst was filtered off and the filtrate was evaporated to dryness, the residue was taken in DCM and the solid filtered off, after that purification by flash chromatography with a gradient of DCM/AcOEt gave title compound. Yield 41%
1H NMR (400 MHz, DMSO-d6) δ ppm 8.70 - 8.76 (2 H, m), 8.44 (1 H, d), 8.24 (1 H, dd), 7.97 (1 H, d), 13, 7.83 - 7.88 (2 H, m), 1.53 (9 H, s)
ESI + m/z 301 [M+H]

### Example 6:

### Preparation of Intermediate (XVI) (intermediate 12) of scheme 5

### 4-(4-(tert-butoxycarbonyl)-3-nitrophenyl)-1-methylpyridin-1-ium iodide

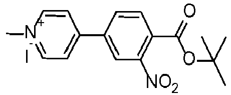

A mixture of intermediate 11 (1g) and iodomethane (2 eq.) in acetone (30ml) was heated at 60 °C for 1 h, and then overnight at rt. Volatiles were evaporated to afford title compound. Yield 100%.
1H NMR (400 MHz, DMSO-d6) δ ppm 9.15 (2 H, d), 8.71 (1 H, d), 8.64 (2 H, d), 8.45 - 8.50 (1 H, m),8.09 (1 H, d), 4.39 (3 H, s), 1.54 (9 H, s)
ESI + m/z 316 [M+H]

### Example 7:

### Preparation of Intermediate (XVII) (intermediate 13) of scheme 5

### tert-butyl 2-amino-4-(1-methylpiperidin-4-yl)benzoate

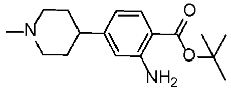

A mixture of intermediate 12 (1 eq) and platinium (IV) oxide (0.035 eq) in MeOH (100ml) was stirred under a constant pressure of H₂ at 5 bar upon completion. Catalyst was filtered off and solvent evaporated to afford title intermediate without further purification. Yield 99%
1H NMR (400 MHz, DMSO-d6) δ ppm 9.23 (1 H, d), 7.61 (1 H, d), 6.59 (3 H, s), 6.41 (1 H, dd), 3.47 (2H, d), 3.05 (2 H, t), 2.80 (3 H, s), 2.61 - 2.71 (1 H, m), 1.94 (2 H, d), 1.67 - 1.82 (2 H, m), 1.52 (9 H, s)
ESI + m/z 292 [M+H]

### Example 8

### Preparation of Intermediate (XVII) (intermediate 14) of scheme 5

### tert-butyl 4-(1-methylpiperidin-4-yl)-2-((tetrahydro-2H-pyran-4-yl)amino)benzoate

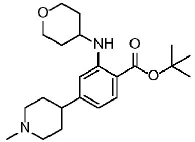

In a one neck round-bottomed flask, a solution of tert-butyl 2-amino-4- (1-methylpiperidin-4-yl)benzoate (485 mg, 1.67 mmol), tetrahydro-4H-pyran-4-one (209 mg, 2.088 mmol), trifluoroacetic acid (0.33 ml, 4.34 mmol) in DCE (25 ml) was stirred for 3 h prior addition of sodium triacetoxyborohydride (531 mg, 2.51 mmol). The mixture was stirred for 24 h at rt, then saturated aqueous solution of sodium bicarbonate was added and the organic layer was separated and concentrated. The residue was loaded onto a RediSep C18aq 50g column eluting with water + 0.1% AcOH and acetonitrile + 0.1% AcOH. Relevant fractions were collected and loaded on a SPE-XC cartridge. 2M Methanolic ammonia fractions were evaporated to give title compound (320 mg; 51% yield). ESI+ m/z 375 [M+H]+.

### Example 9

### Preparation of Intermediate (XVII) (intermediate 15) of scheme 5

### tert-butyl4-(1-methylpiperidin-4-yl)-2-(2,2,2-trifluoro-N-(tetrahydro-2H-pyran-4-yl)acetamido)benzoate

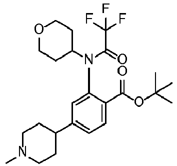

Trifluoroacetic anyhydride (0.154 ml, 1.111 mmol) was added to a solution of tert-butyl 4-(1-methylpiperidin-4-yl)-2-((tetrahydro-2H-pyran-4-yl)amino)benzoate (320 mg, 0.854 mmol) and TEA (0.179 ml, 1.282 mmol) in DCM (10 ml) at 0°C. The resulting mixture was stirred for 1h at rt. The reaction was washed with water, dried and evaporated to give title compound (400mg; 99% yield). ESI+ m/z 471 [M+H]+.

Scheme 6 for obtaining intermediates (VI) is below reported: 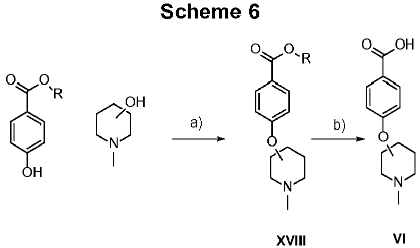 Scheme 6 provides for the following steps
a) Conversion of starting materials in compounds of formula (XVIII) under mitsunobu conditions; and
b) Ester-deprotection of compounds of formula (XVII) to obtain intermediates of formula (VI), using a strong base such as sodium hydroxide.

### Example 10:

### Preparation of Intermediate (XVIII) (intermediate 16-17) of scheme 6

### General procedure 5

A mixture of ethyl 4-hydroxybenzoate (1 eq), *N*-methyl piperidinol (1 eq), triphenylphosphine (1.1 eq) and DIAD (1.1 eq) in THF (0.2 M) was stirred at RT upon completion. Volatiles were evaporated and the reside was triturated in cyclohexane. The solid was filtered off and the filtrate was evaporated and loaded on a SPE-SCX cartridge. 2M Methanolic ammonia fraction were evaporated to afford title intermediates.

**Table 8**

| **Intermediates 16-17: Intermediate (XVIII) of scheme 6** | | | |
|---|---|---|---|
| **Intermediates** | **Structure** | **Analysis** | **Yields (%)** |
| 16 | 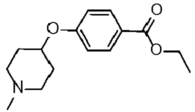 | ESI + m/z 264[M+H] | 72 |
| 17 | 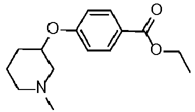 | ESI + m/z 264 [M+H] | 21 |

### Example 11

### Preparation of intermediate 18

### 4-(1-((benzyloxy)carbonyl)-1,2,3,6-tetrahydropyridin-4-yl)-2-hydroxybenzoic acid.

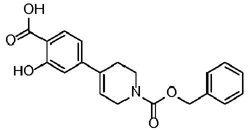

A mixture of 4-bromo-3-hydroxybenzoic acid (1 g, 4.61 mmol), benzyl 4- (4,4,5,5-tetramethyl-1,3,2-dioxaborolan-2-yl)-3,6-dihydropyridine-1(2H)-carboxylate (1.740 g, 5.07 mmol), PdCl₂(PPh₃)₂ (0.162 g, 0.230 mmol) and Na₂CO₃ (0.977 g, 9.22 mmol) in dioxane (Ratio: 4.00, Volume: 20 ml)/water (Ratio: 1.000, Volume: 5 ml) was heated at 100 °C overnight. The reaction was partioned between water and AcOEt. The aqueous layer was separated, acidified with HCl 2M and extracted with AcOEt, which was then dried over Na₂SO₄ and evaporated. The residue was triturated in diethylether to afford title compound (665 mg; 33.5 % yield).
¹H NMR (400 MHz, *DMSO-d*6) δ ppm 12.76 (1 H, br. s.), 9.83 (1 H, s), 7.43 (1 H, d), 7.38 - 7.42 (4 H,m), 7.30 - 7.37 (2 H, m), 7.19 (1 H, d), 5.97 (1 H, br. s.), 5.13 (2 H, s), 4.01 - 4.14 (2 H, m), 3.59 (2 H,br. s.), 2.45 - 2.49 (1 H, m). ESI + m/z 354 [M+H]+

### Example 12

### Preparation of intermediate 19

### benzyl 4-(2-hydroxy-4-(methoxycarbonyl)phenyl)-3,6-dihydropyridine-1(2H)-carboxylate

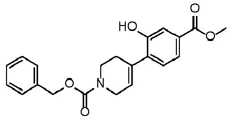

4-(1-((Benzyloxy)carbonyl)-1,2,3,6-tetrahydropyridin-4-yl)-3-hydroxybenzoic acid (660 mg, 1.868 mmol) was dissolved in MeOH (50ml) prior addition of sulfuric acid (500 µL, 9.38 mmol), the reaction was heated at 80 °C for 6h; volatiles were evaporated and the residue was partitioned between AcOEt and water, the organic phase was separated and washed with NaHCO₃ and water in sequence; dried over Na2SO4 and evaporated to afford title compound (588 mg; 68.6 % yield).
¹H NMR (400 MHz, *DMSO-d*6) δ ppm 9.92 (1 H, s), 7.46 (1 H, d), 7.39 (4 H, d), 7.31 - 7.37 (2 H, m),7.22 (1 H, d), 5.99 (1 H, br. s.), 5.13 (2 H, s), 4.03 - 4.13 (2 H, m), 3.82 (3 H, s), 3.59 (2 H, br. s.), 2.49(1 H, d). ESI + m/z 368 [M+H]+

### Example 13

### Preparation of intermediate 20

### Benzyl-4-(4-(methoxycarbonyl)-2-(oxetan-3-ylmethoxy)phenyl)-3,6-dihydropyridine-1(2H)-carboxylate

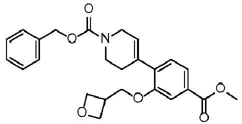

Benzyl-4-(2-hydroxy-4-(methoxycarbonyl)phenyl)-3,6-dihydropyridine-1(2H)-carboxylate (580 mg, 1.579 mmol), oxetan-3-ylmethanol (0.255 ml, 3.16 mmol), triphenylphosphine (828 mg, 3.16 mmol) and DIAD (0.932 ml, 4.74 mmol) in THF (40 ml) were stirred at RT upon completion. The solvent was evaporated and the residue was dissolved in EtOAc and the solution washed with HCl 1M, brine and NaOH 1M in sequence. The organic layer was dried over Na₂SO₄ and evaporated. The residue was purified by flash chromatography eluting with a gradient of cyclohexane/AcOEt to afford title compound (596 mg; 86 % yield).
ESI + m/z 438 [M+H]⁺

### Example 14

### Preparation of intermediate 21

### methyl 3-(oxetan-3-ylmethoxy)-4-(piperidin-4-yl)benzoate

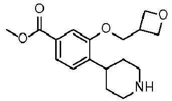

Benzyl4-(4-(methoxycarbonyl)-2-(oxetan-3-ylmethoxy)phenyl)-3,6-dihydropyridine-1(2H)-carboxylate (590 mg, 1.349 mmol) and palladium hydroxide on carbon, 20% loading dry basis (189 mg, 0.270 mmol) in MeOH (50 ml) was stirred under a constant pressure of H₂ at 2 bar for 2h. Catalyst was filtered off and the filtrate was evaporated. The residue was loaded on SPE-SCX cartridge (2g). 2M Methanolic ammonia fraction was evaporated to afford title compound (201 mg; 48.8 % yield).
ESI + m/z 306 [M+H]⁺

### Example 15

### Preparation of intermediate 22

### methyl 4-(1-methylpiperidin-4-yl)-3-(oxetan-3-ylmethoxy)benzoate

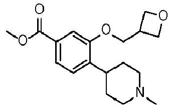

A mixture of methyl 3-(oxetan-3-ylmethoxy)-4-(piperidin-4-yl)benzoate (200 mg, 0.655 mmol), AcOH (0.075 ml, 1.310 mmol) and formaldehyde 38% in water (0.052 ml, 0.720 mmol) in DCM (10 ml) was stirred at RT, after 1h sodium triacetoxyborohydride (347 mg, 1.637 mmol) was added and the resulting mixture was stirred at RT upon completion. MeOH was added and volatiles were evaporated. The residue was purified by reverse phase chromatography to afford title compound (162 mg, 59.6 % yield).
ESI + m/z 320 [M+H]⁺

### Example 16

### Preparation of intermediate 23

### tert-butyl (3R,4R)-3-hydroxy-4-(4-(methoxycarbonyl)phenyl)piperidine-1-carboxylate

### and tert-butyl(3S,4S)-3-hydroxy-4-(4-(methoxycarbonyl)phenyl)piperidine-1-carboxylate (mixture of trans isomers)

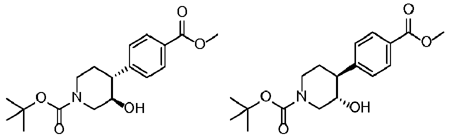

Borane dimethyl sulfide complex solution, 2.0 M in THF (2.95 ml, 5.89 mmol) was added dropwise to a stirred solution of tert-butyl 4-(4-(methoxycarbonyl)phenyl)- 3,6-dihydropyridine-1(2H)-carboxylate (1.7 g, 5.36 mmol) in THF (20 ml). The resulting mixture was stirred at RT for 16h, after that the reaction was cooled to 0°C and NaOH 1N (13.39 ml, 13.39 mmol) followed by hydrogen peroxide solution (1.172 ml, 13.39 mmol) were added. The mixture was then stirred at RT for 1h and then quenched with an aqueous saturated solution of sodium thiosulfate and extracted with AcOEt (3 x 10ml). Organic phases were dried, evaporated and then purified by flash chromatography eluting with a gradient of cyclohexane/AcOEt to afford title compound as mixture of *trans* isomers (1.34 g, 74.6 % yield).
¹H NMR (400 MHz, *DMSO-d*6) δ 7.88 (2 H, d), 7.41 (2 H, d), 4.89 (1 H, d), 4.15 (1 H, br. s.), 3.94 -4.04 (1 H, m), 3.84 (3 H, s), 3.52 (1 H, tt), 2.76 (1 H, br. s.), 2.53 - 2.69 (2 H, m), 1.71 (1 H, dd), 1.56 (1H, qd), 1.43 (9 H, s); ESI + m/z 336 [M+H]⁺

### Example 17

### Preparation of intermediate 24

### tert-butyl (3S,4R)-3-fluoro-4-(4-(methoxycarbonyl)phenyl)piperidine-1-carboxylate and tert-butyl (3R,4S)-3-fluoro-4-(4-(methoxycarbonyl)phenyl)piperidine-1-carboxylate (mixture of cis isomers)

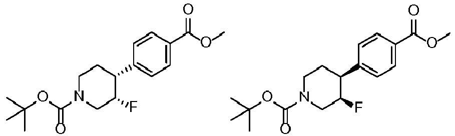

The mixture of trans diastereoisomers of **example 16** (700 mg, 2.087 mmol) in DCM (20 ml) was cooled at -78°C prior addition of Deoxo-Fluor® solution 50% in THF (0.986 ml, 2.296 mmol). After addition, the resulting mixture was gently warmed to RT over a period of 1h and then maintained at this temperature for an additional hour, after that the reaction was quenched with aqueous sodium bicarbonate, phases were separated and the organic layer was washed with citric acid (20ml) first and brine (20ml) after, dried over Na₂SO₄ and then evaporated. The residue was purified by flash chromatography eluting with a gradient of cyclohexane/AcOEt to afford title compound as a mixture of *cis* isomers (600 mg, 85 % yield).
¹H NMR (400 MHz, *DMSO-d*6) δ ppm 7.90 - 7.94 (2 H, m), 7.51 (2 H, d), 4.57 - 4.81 (1 H, m), 4.31 (1H, d), 3.97 (1 H, d), 3.85 (3 H, s), 2.93 - 3.04 (1 H, m), 2.86 (2 H, d), 1.77 - 1.87 (1 H, m), 1.59 - 1.72 (1H, m), 1.44 (9 H, s). ESI + m/z 338 [M+H]+

### Example 18

### Preparation of intermediate 25

### Methyl 4-((3S,4R)-3-fluoropiperidin-4-yl)benzoate and Methyl 4-((3R,4S)-3-fluoropiperidin-4-yl) benzoate (mixture of cis isomers)

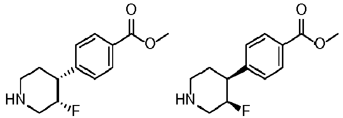

A mixture of *cis* isomers of **example 17** (600 mg, 1.778 mmol) was dissolved in DCM (15 ml) prior addition of TFA (5 ml, 64.9 mmol), the reaction was stirred at RT for 16h, after that volatiles were evaporated and the residue was loaded onto a SPE-SCX cartridge (5g); 2M methanolic ammonia fractions were evaporated to afford title compound as a mixture of *cis* isomers (374 mg, 89 % yield).
¹H NMR (400 MHz, *DMSO-d*6) δ ppm 7.90 - 7.95 (2 H, m), 7.46 (2 H, d), 4.52 - 4.72 (1 H, m), 3.82 -3.87 (3 H, m), 3.27 (1 H, dd), 2.80 - 2.94 (2 H, m), 1.77 (1 H, dt), 1.56
- 1.68 (1 H, m). ESI + m/z 238 [M+H]+

### Example 19

### Preparation of intermediate 26

### Methyl 4-((3S,4R)-3-fluoro-1-methyl-piperidin-4-yl) benzoate and Methyl 4-((3R,4S)-3-fluoro-1-methyl-piperidin-4-yl) benzoate (mixture of cis isomers)

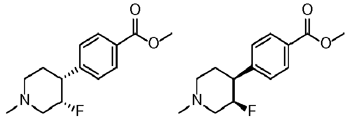

A mixture of *cis* isomers of **example 18** (370 mg, 1.559 mmol), AcOH (0.179 ml, 3.12 mmol) and formaldehyde 38% in water (0.124 ml, 1.715 mmol) in DCM (10 ml) was stirred at RT for 1h prior addition of sodium triacetoxyborohydride (826 mg, 3.90 mmol); the resulting mixture was stirred at RT for 3h, MeOH was added and volatiles evaporated. The residue was dissolved in AcOEt (20ml) and washed with NaHCO₃ (20ml), the organic phase was separated, washed with water, dried over Na₂SO₄ and evaporated to afford title compound (367 mg, 94 % yield).
¹H NMR (400 MHz, *DMSO-d*6) δ 7.89 - 7.94 (2 H, m), 7.45 - 7.51 (2 H, m), 4.65 - 4.86 (1 H, m),3.85 (3 H, s), 3.15 - 3.24 (1 H, m), 2.68 - 2.83 (2 H, m), 2.27 (3 H, s), 1.94 - 2.07 (2 H, m), 1.66 - 1.84 (2H, m). ESI + m/z 252 [M+H]⁺.

### Example 20

### Preparation of intermediate 27

### tert-butyl 4-(4-(methoxycarbonyl)phenyl)-3-oxopiperidine-1-carboxylate

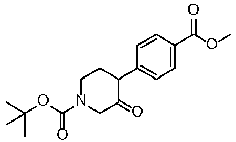

A mixture of trans isomers of **example 14** (600 mg, 1.789 mmol) and Dess-Martin periodinane (3.794 g, 8.94 mmol) in DCM (20 ml) was stirred at RT for 4h, the mixture was filtered through a pad of celite and the filtrate was washed with aqueous NaHCO₃, dried over Na₂SO₄ and evaporated. The residue was then purified by flash chromatography eluting with a gradient of cyclohexane/AcOEt to afford title compound (620 mg, 83 % yield).
¹H NMR (400 MHz, *DMSO-d*6) δ ppm 7.89 - 7.93 (2 H, m), 7.33 (2 H, d), 4.12 - 4.18 (1 H, m), 3.97 -4.05 (2 H, m), 3.80 - 3.92 (5 H, m), 3.48 (1 H, d), 2.15 - 2.26 (2 H, m), 1.44 (9 H, s). ESI + m/z 334 [M+H]⁺.

### Example 21

### Preparation of intermediate 28

### tert-butyl 3,3-difluoro-4-(4-(methoxycarbonyl)phenyl)piperidine-1-carboxylate

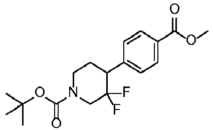

**Intermediate 27 of Example 20** (620 mg, 1.860 mmol) was dissolved in DCM (20 ml) and colled to 178°C prior addition of Deoxo-Fluor® solution 50% in toluene (1.758 ml, 4.09 mmol) dropwise. The resulting mixture was gently warmed to RT over a period of 1 hour and then maintained at this temperature for an additional hour. The reaction was quenched with aqueous sodium bicarbonate and the phases separated, the organic layer was washed with citric acid (20ml) first and water (20ml) then, dried over Na₂SO₄ and then evaporated. The residue was purified by flash chromatography eluting with a gradient of cyclohexane/AcOEt to afford title compound (402 mg, 60.8 % yield).
¹H NMR (400 MHz, *DMSO-d*6) δ ppm 7.94 (2 H, d), 7.48 (2 H, d), 4.25 (1 H, br. s.), 4.12 (1 H, d), 3.79 -3.89 (3 H, m), 3.40 - 3.57 (1 H, m), 2.98 (1 H, br. s.), 2.01 - 2.10 (1 H, m), 1.82 - 1.91 (1 H, m), 1.44 (9H, s). ESI + m/z 356 [M+H]⁺.

### Example 22

### Preparation of intermediate 29

### methyl 4-(3,3-difluoropiperidin-4-yl)benzoate

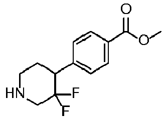

**Intermediate 28 of Example 21** (400 mg, 1.126 mmol) was dissolved in DCM (15 ml) prior addition of TFA (0.5 ml, 6.49 mmol) and the mixture stirred at RT for 16h. Volatiles were was evaporated and the residue loaded onto a SPE-SCX cartridge (5g) 2M methanolic ammonia fractions was evaporated to afford title compound (240 mg, 84 % yield).
¹H NMR (400 MHz, *DMSO-d*6) δ ppm 7.91 - 7.96 (2 H, m), 7.47 (2 H, d), 3.86 (3 H, s), 3.32 - 3.42 (1 H,m), 3.07 - 3.17 (1 H, m), 3.00 (1 H, d), 2.78 - 2.91 (1 H, m), 2.54 - 2.69 (2 H, m), 2.01 (1 H, qd), 1.72 -1.82 (1 H, m). ESI + m/z 256 [M+H]⁺.

### Example 23

### Preparation of intermediate 30

### methyl 4-(3,3-difluoro-1-methylpiperidin-4-yl)benzoate

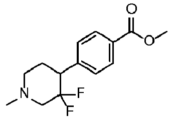

**Intermediate 29 of Example 22** (235 mg, 0.921 mmol), AcOH (0.105 ml, 1.841 mmol) and formaldehyde 38% in water (0.073 ml, 1.013 mmol) were dissolved in DCM (10 ml) and stirred at RT for 1 hour prior addition of sodium triacetoxyborohydride (488 mg, 2.302 mmol); the resulting mixture was stirred at RT for additional 3h and then MeOH was added and volatiles evaporated. The residue was re-dissolved in DCM and washed with NaHCO₃ (20 ml) first and water (20ml) after, dried over Na₂SO₄ and evaporated to afford title compound (231 mg, 93 % yield).
¹H NMR (400 MHz, *DMSO-d*6) δ ppm 7.94 (2 H, d), 7.48 (2 H, d), 3.86 (3 H, s), 3.06 - 3.28 (2 H, m), 2.91 (1 H, d), 2.31 - 2.40 (1 H, m), 2.29 (3 H, s), 2.05 - 2.20 (2 H, m), 1.76 - 1.87 (1 H, m). ESI + m/z 270 [M+H]⁺.

### Example 24

### Preparation of intermediate 31

### Ethyl (4-acetylpiperazin-1-yl)benzoate

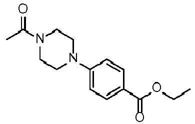

Ethyl 4-(piperazin-1-yl)benzoate (400 mg, 1.707 mmol), DIPEA (0.313 ml, 1.793 mmol) and acetyl chloride (0.127 ml, 1.793 mmol) in ethyl acetate (10 ml) was stirred at RT for 2 days; water was added and the phases were separated. The organic layer was evaporated to afford title compound (496 mg, 95 % yield).
¹H NMR (400 MHz, *DMSO-d6)* δ ppm 7.77 - 7.83 (2 H, m), 6.96 - 7.01 (2 H, m), 4.25 (2 H, q), 3.58 (4H, dd), 3.35 - 3.41 (2 H, m), 3.29 - 3.34 (2 H, m), 2.05 (3 H, s), 1.30 (4 H, t). ESI + m/z 277 [M+H]⁺.

### Example 25

### Preparation of Intermediate (VI) (intermediate 32-41) of schemes 4,5 or 6

### General procedure 6

The benzoate derivative (1eq) was dissolved in methanol (20ml) and water (20ml) prior addition of sodium hydroxide (3 eq), the reaction was left stirring upon completion. Volatiles were concentrated and the solid dissolved in water, acidified to pH 3-4 and purified by reverse phase chromatography eluting with water (0,1% of AcOH)/Acetonitrile (0.1% of AcOH) from 10/0 to 0/10 in gradient.

**Table 9**

| **Intermediates 32-41 (Intermediate VI, scheme 4,5,6)** | | | |
|---|---|---|---|
| **Intermediate** | **Structure** | **HNMR/MS** | **Yield %** |
| **32** | 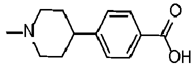 | ¹H NMR (400 MHz, *DMSO-d*6) δ ppm 10.01 - 12.33 (1 H, m), 7.91 (2 H, d), 7.38 (2 H, d), 3.31 - 3.41 (2H, m), 2.74 - 2.96 (3 H, m), 2.67 (3 H, s), 1.85 - 2.04 (4 H, m), 0.99 - 1.12 (1 H, m) | 100 |
| | | ESI + m/z 220 [M+H]⁺ | |
| **33** | 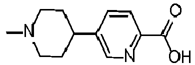 | ¹H NMR (400 MHz, *DMSO-d*6) δ ppm 8.29 - 8.33 (1 H, m), 7.82 (1 H, d), 7.60 (1 H, dd), 2.87 (2 H, d),2.20 (3 H, s), 1.97 (2 H, td), 1.58 - 1.80 (4 H, m) | 94 |
| | | ESI + m/z 221 [M+H]⁺ | |
| **34** | 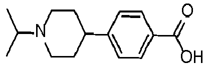 | ¹H NMR (400 MHz, *DMSO-d*6) δ ppm 7.85 (2 H, d), 7.33 (2 H, d), 2.90 (2 H, d), 2.70 - 2.78 (1 H, m), 2.24 (2 H, t), 1.77 (2 H, d), 1.56 - 1.69 (2 H, m), 1.00 (6 H, d) | 66 |
| | | ESI + m/z 248 [M+H]⁺ | |
| **35** | 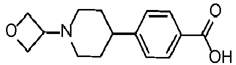 | ¹H NMR (400 MHz, *DMSO-d*6) δ ppm 12.61 (1 H, br. s.), 7.83 - 7.89 (2 H, m), 7.38 (2 H, d), 4.51 - 4.58 (2 H, m), 4.45 (2 H, t), 3.42 (1 H, quin), 2.81 (2 H, d), 2.59 (1 H, tt), 1.84 - 1.92 (2 H, m), 1.61 - 1.82 (4H, m) | 86 |
| | | ESI + m/z 262 [M+H]⁺ | |
| **36** | 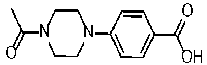 | ¹H NMR (400 MHz, *DMSO-d*6) δ ppm 12.26 (1 H, br. s.), 7.79 (2 H, d), 6.97 (2 H, d), 3.54 - 3.62 (4 H,m), 3.33 - 3.40 (2 H, m), 3.25 - 3.32 (2 H, m), 2.05 (3 H, s) | 68 |
| | | ESI + m/z 249 [M+H]⁺ | |
| **37** | 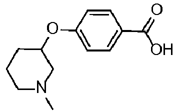 | ¹H NMR (400 MHz, *DMSO-d*6) δ ppm 7.85 - 7.91 (2 H, m), 6.97 - 7.06 (2 H, m), 4.05 (1 H, dd), 3.92 (1H, dd), 2.93 - 3.01 (1 H, m), 2.61 (1 H, dd), 2.38 (3 H, s), 2.18 - 2.27 (1 H, m), 1.97 (1 H, dd), 1.65 -1.76 (2 H, m), 1.60(1 H, d) | 90 |
| | | ESI + m/z 236 [M+H]⁺ | |
| **38** | 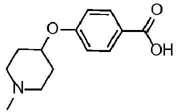 | ¹H NMR (400 MHz, *DMSO-d*6) δ ppm 7.82 - 7.91 (2 H, m), 6.97 - 7.07 (2 H, m), 4.49 (1 H, tt), 2.60 -2.68 (2 H, m), 2.16 - 2.30 (5 H, m), 1.96 (2 H, dd), 1.60 - 1.75 (2 H, m) | 80 |
| | | ESI + m/z 236 [M+H]⁺ | |
| **39** | 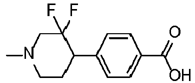 | ¹H NMR (400 MHz, *DMSO-d*6) δ ppm 12.85 (1 H, br. s.), 7.89 - 7.93 (2 H, m), 7.44 (2 H, d), 3.06 - 3.26 (2 H, m), 2.91 (1 H, d), 2.24 - 2.40 (4 H, m), 2.04 - 2.20 (2 H, m), 1.75 - 1.86 (1 H, m) | 81 |
| | | ESI + m/z 256 [M+H]⁺ | |
| **40** mixture of cis | 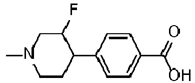 | ESI + m/z 238 [M+H]⁺ | 99 |
| **41** | 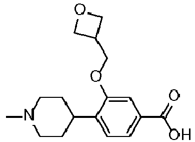 | ESI + m/z 306 [M+H]⁺ | 87 |

### Example 26

### Preparation of intermediate 42

### 4-(1-methylpiperidin-4-yl)-2-(2,2,2-trifluoro-N-(tetrahydro-2H-pyran-4-yl)acetamido) benzoic acid, trifluoroacetic acid salt

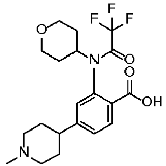

**Intermediate 15 of example 9** (400 mg, 0.850 mmol) was dissolved in DCM (5 ml) and 2,2,2-trifluoroacetic acid (1 ml, 12.98 mmol) was added, the reaction was stirred at RT overnight. Volatiles were evaporated and the compound used as such without further purification. ¹H NMR (400 MHz, *DMSO-d*6) δ ppm (1 H, s), 9.32 (1 H, br. S.), 7.94 - 8.10 (1 H, m), 7.44 - 7.55(1 H, m), 7.25 - 7.34 (1 H, m), 4.47 - 4.61 (1 H, m), 3.82 - 3.92 (1 H, m), 3.74 - 3.82 (1 H, m), 3.49 -3.61 (2 H, m), 3.39 - 3.48 (2 H, m), 3.02 - 3.14 (2 H, m), 2.91 - 3.02 (1 H, m), 2.83 (3 H, s), 2.05 - 2.17(2 H, m), 1.92 - 2.01 (1 H, m), 1.74 - 1.91 (2 H, m), 1.40 - 1.61 (2 H, m), 1.21 - 1.30 (1 H, m), 0.91 - 1.04 (1 H, m). ESI + m/z 416 [M+H]⁺

### Example 27

### Preparation of intermediate 43

### 4-(1-(2-methoxyethyl)piperidin-4-yl)benzoic acid

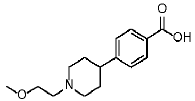

**Intermediate 3 of example 2** (100mg, 0,48mmol) was dissolved in acetonitrile (5 ml), then K₂CO₃ (119 mg, 0.860 mmol) and 1-chloro-2-methoxyethane (0.039 ml, 0.430 mmol) were added and the reaction was heated at 90°C in a sealed tube for 24h. Solvent was evaporated and the residue was dissolved in MeOH (Ratio: 1; 5.00 ml)/Water (Ratio: 1; 5.00 ml). After 4h volatiles were evaporated and the residue loaded onto a RediSep C18Aq 50g column primed with water + 0.1% AcOH only. The column was then eluted with water + 0.1% AcOH only and then gradually changed to acetonitrile + 0.1% AcOH. Relevant fractions were collected and evaporated to afford title compound (69 mg, 60.9 % yield).
¹H NMR (400 MHz, *DMSO-d*6) δ ppm 7.84 - 7.89 (2 H, m), 7.37 (2 H, d), 3.46 (2 H, t), 3.25 (3 H, s), 2.99 (2 H, d), 2.55 - 2.61 (1 H, m), 2.09 (2 H, td), 1.59 - 1.80 (4 H, m). ESI + m/z 264 [M+H]⁺

### Example 28

### Preparation of intermediate 44

### 5-fluoro[d][3,1]dioxol-4-amine

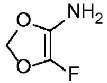

The compound was prepared according to procedure described in Journal of Medicinal Chemistry, 47(4), 871-887; 2004.

### Example 29

### Preparation of intermediate III (intermediates 45-55) of scheme 1

### General procedure 7

To a solution of the aniline (all commercially available; 1.40 g, 10.0 mmol) in toluene (60 mL), 4-bromo-2-fluorobenzonitrile (1.2 eq), Cs₂CO₃ (1.5 eq) and Pd(Oac)₂ / Xphos (0.01/0.06 eq) were added. After stirred at 90°C for 20 hours under N₂, toluene was removed under vacuum and the crude purified by column chromatography on silica gel eluting with petrol ether/ethyl acetate to afford title compounds.

**Table10**

| **Intermediates 45-55 (Intermediate III; scheme 1)** | | | |
|---|---|---|---|
| **intermediates** | **Structure** | **HNMR/MS** | **Yield %** |
| **45** | 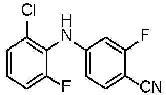 | ESI + m/z 265-267 [M+H]⁺ | 28 |
| **46** | 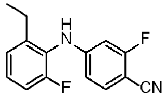 | ESI + m/z 259 [M+H]⁺ | 95 |
| **47** | 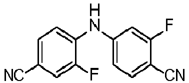 | ESI + m/z 256 [M+H]⁺ | 51 |
| **48** | 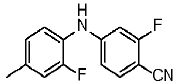 | ESI + m/z 245 [M+H]⁺ | 30 |
| **49** | 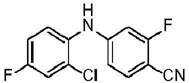 | ESI + m/z 265 [M+H]⁺ | 29 |
| **50** | 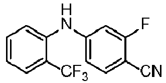 | ESI + m/z 281 [M+H]⁺ | 95 |
| **51** | 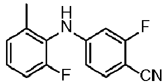 | ESI + m/z 245 [M+H]⁺ | 91 |
| **52** | 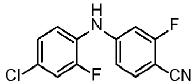 | ESI + m/z 265-267 [M+H]⁺ | 55 |
| **53** | 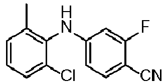 | ESI + m/z 261 [M+H]⁺ | 38 |
| **54** | 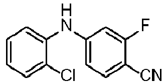 | ESI + m/z 247-249 [M+H]⁺ | 59 |
| **55** | 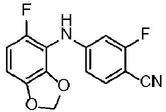 | ESI + m/z 275 [M+H]⁺ | 64 |

### Example 30

### Preparation of intermediate III (intermediate 56) of scheme 2

### 2-fluoro-4-((2-fluorophenyl)amino)benzonitrile

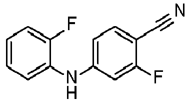

To a solution of 2-fluoroaniline (18.0 g, 0.163 mmol) in toluene (300 mL) 4-bromo-2-fluorobenzonitrile (25.0 g, 0.125 mol), t-BuONa (18.0 g, 0.188 mol) and Pd₂(dba)₃ /BINAP (1.00 g / 2.00 g) were added and the reaction stirred at 80°C for 48 hours under a constant flow of N₂. To the mixture, H₂O (300 mL) was added and then extracted with EtOAc (3 x 300 mL), the combined organic phases were collected, dried over Na₂SO₄ and evaporated. The crude was purified by flash chromatography eluting with a gradient of petrol ether/ethyl acetate, the obtained compound was re-crystallized in petrol ether/ethyl acetate in 3:1 ratio to afford title compound (3g;10% yield).
ESI + m/z 231 [M+H]⁺

### Example 31

### Preparation of intermediate IV (intermediates 57-68) of scheme 1

### General procedure 7

To a solution of **intermediate III (intermediates 45-56)** (1 eq) in n-butanol (0.25 M), NH₂NH₂.H₂O (20 eq), was added. After stirred at 120°C for 16 hours, n-butanol was removed under vacuum. To the mixture H₂O was added and the solution extracted with EtOAc (3 times), washed with water, brine, dried over Na₂SO₄ and concentrated to afford title compounds.

**Table 11**

| **Intermediates 57-68 (Intermediate IV; scheme 1)** | | | |
|---|---|---|---|
| **intermediates** | **Structure** | **MS** | **Yield %** |
| **57** | 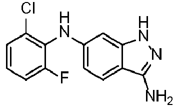 | ESI + m/z 277-279 [M+H]⁺ | 90 |
| **58** | 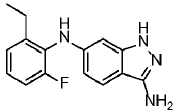 | ESI + m/z 271 [M+H]⁺ | 80 |
| **59** | 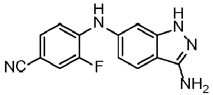 | ESI + m/z 268 [M+H]⁺ | 88 |
| **60** | 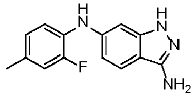 | ESI + m/z 257 [M+H]⁺ | 69 |
| **61** | 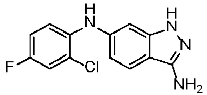 | ESI + m/z 277 [M+H]⁺ | 64 |
| **62** | 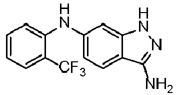 | ESI + m/z 293 [M+H]⁺ | 60 |
| **63** | 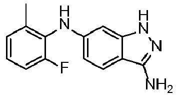 | ESI + m/z 257 [M+H]⁺ | 85 |
| **64** | 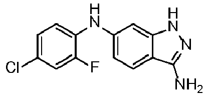 | ESI + m/z 277-279 [M+H]⁺ | 86 |
| **65** | 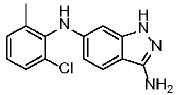 | ESI + m/z 273 [M+H]⁺ | 80 |
| **66** | 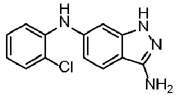 | ESI + m/z 259-261 [M+H]⁺ | 98 |
| **67** | 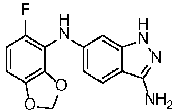 | ESI + m/z 287 [M+H]⁺ | 83 |
| **68** | 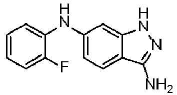 | ESI + m/z 243 [M+H]⁺ | 71 |

### Example 32

### Preparation of intermediate IX (intermediate 69) of scheme 2

### tert-butyl 3-amino-6-bromo-1H-indazole-1-carboxylate

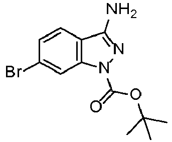

A mixture of 6-bromo-1H-indazol-3-amine (930 mg, 4.39 mmol), BOC₂O (1.018 ml, 4.39 mmol) and DMAP (26.8 mg, 0.219 mmol) in THF (20 ml) was stirred at 0 °C for 2h. The solvent was evaporated and the residue was loaded on a SNAP-Si cartridge (50g) eluted with DCM/AcOEt from 10/0 to 8/2 in gradient. Solvents were evaporated to afford title compound (1.36 g, 99 % yield).
¹H NMR (400 MHz, *DMSO-d6)* δ ppm 8.13 (1 H, s), 7.81 (1 H, d), 7.47 (1 H, dd), 6.42 (2 H, br. S.), 1.59(9 H, s). ESI + m/z 313 [M+H]⁺

### Example 33

### Preparation of intermediate V (intermediates 70-81) of schemes 1 and 2

### General procedure 8

To a solution of **Intermediate IV (intermediates 57-68)** (1 eq) in 1,4-dioxane (0.3 M), DMAP (0.1 eq), Et₃N (1.50 g, 2 eq) and (Boc)₂O (1.2 eq) were added and then stirred at room temperature upon completion. Solvent was removed under vacuum and the crude was purified by flash chromatography eluting with petrol ether/ethyl acetate to afford title compounds.

**Table 12**

| **intermediates 70-81 (Intermediate V; scheme 1 and 2)** | | | |
|---|---|---|---|
| **intermediates** | **Structure** | **MS** | **Yield %** |
| **70** | 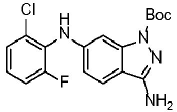 | ESI + m/z 377-379 [M+H]⁺ | 62 |
| **71** | 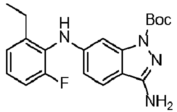 | ESI + m/z 371 [M+H]⁺ | 49 |
| **72** | 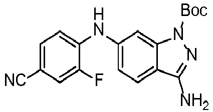 | ESI + m/z 368 [M+H]⁺ | 57 |
| **73** | 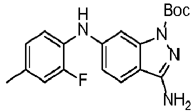 | ESI + m/z 357 [M+H]⁺ | 59 |
| **74** | 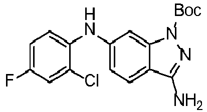 | ESI + m/z 377 [M+H]⁺ | 72 |
| **75** | 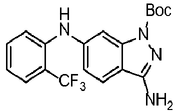 | ESI + m/z 393 [M+H]⁺ | 41 |
| **76** | 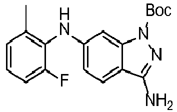 | ESI + m/z 357 [M+H]⁺ | 46 |
| **77** | 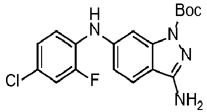 | ESI + m/z 377-379 [M+H]⁺ | 49 |
| **78** | 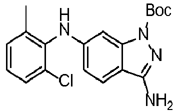 | ESI + m/z 373 [M+H]⁺ | 44 |
| **79** | 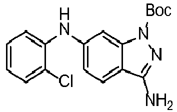 | ESI + m/z 359-361 [M+H]⁺ | 64 |
| **80** | 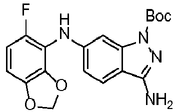 | ESI + m/z 387 [M+H]⁺ | 53 |
| **81** | 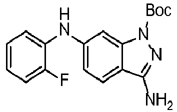 | ESI + m/z 343 [M+H]⁺ | 43 |

### Example 34

### Preparation of intermediate V (intermediate 82) of scheme 2

### tert-butyl 3-amino-6-(2-fluorophenoxy)-1H-indazole-1-carboxylate

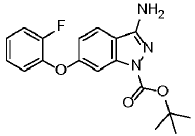

A mixture of intermediate 69 (300 mg, 0.961 mmol), 2-fluorophenol (0.259 ml, 2.88 mmol), N,N-Dimethylglycine HCl (107 mg, 0.769 mmol) and Cul (99 mg, 0.519 mmol) in dioxane (5 ml) was heated with stirring at 100 °C for 48 hours. The reaction was cooled, diluted with AcOEt and filtered through a pad of celite. Volatiles were evaporated and the residue loaded onto a RediSep C18aq (3x50g) column eluting with water + 0.1% AcOH and acetonitrile + 0.1% AcOH. Relevant fractions were collected and evaporated to give title compound. (150 mg; 38.6% yield) ¹H NMR (400 MHz, DMSO-d₆) δ ppm 7.80 - 7.88 (1 H, m), 7.43 - 7.51 (1 H, m), 7.22 - 7.40 (4 H, m), 7.01 - 7.12 (1 H, m), 6.23 - 6.33 (2 H, m), 1.45 (9 H, s). ESI + m/z 344 [M+H]⁺

### Example 35:

### Preparation of Intermediate VII (intermediates 83- 97) of scheme 1 and 2

### General procedure 9

To a solution of **Intermediate V (intermediates 70-82) (**1 eq) in DCM (0.1M), DIPEA (10 eq) and acid chloride (1.1 eq) (prepared *in situ* from **intermediates 32, 37 and 38** by standard procedures) were added. After that the reaction was stirred at room temperature for 16 hours. DCM was removed under vacuum and the crude was purified by reverse phase column eluting with water + 0.1% AcOH and acetonitrile + 0.1% AcOH.

**Table 13**

| **Intermediates 83- 97 (Intermediate VII; scheme 1 and 2)** | | | |
|---|---|---|---|
| **Intermediates** | **Structure** | **MS** | **Yield %** |
| **83** | 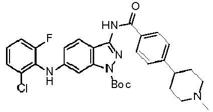 | ESI + m/z 578-580 [M+H]⁺ | 35 |
| **84** | 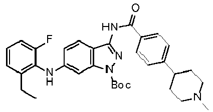 | ESI + m/z 572 [M+H]⁺ | 23 |
| **85** | 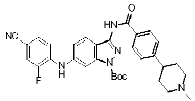 | ESI + m/z 569 [M+H]⁺ | 27 |
| **86** | 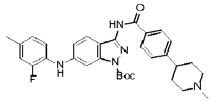 | ESI + m/z 558 [M+H]⁺ | 16 |
| **87** | 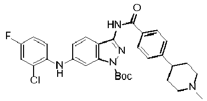 | ESI + m/z 578 [M+H]⁺ | 15 |
| **88** | 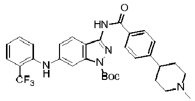 | ESI + m/z 594 [M+H]⁺ | 38 |
| **89** | 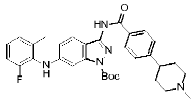 | ESI + m/z 558 [M+H]⁺ | 19 |
| **90** | 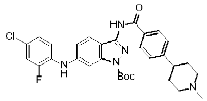 | ESI + m/z 577-579 [M+H]⁺ | 19 |
| **91** | 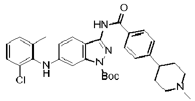 | ESI + m/z 574 [M+H]⁺ | 28 |
| **92** | 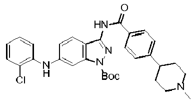 | ESI + m/z 560-562 [M+H]⁺ | 25 |
| **93** | 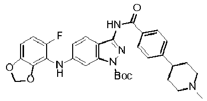 | ESI + m/z 588 [M+H]⁺ | 29 |
| **94** | 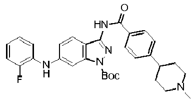 | ESI + m/z 544 [M+H]⁺ | 83 |
| **95** | 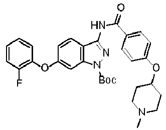 | ESI + m/z 561 [M+H]⁺ | 98 |
| **96** | 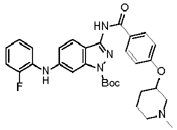 | ESI + m/z 561 [M+H]⁺ | 98 |

### Example 36

### Preparation of Intermediates VIII (Intermediates 97-100) of scheme 2

### General procedure 10

**Intermediate VI (intermediates 32, 38 and Ethyl 4-(piperazin-1-yl)benzoic acid)** (1.3 eq) was suspended in toluene (0.4 M), thionyl chloride (20 eq) was added and the mixture was stirred at 90°C for 60 min. The solvent was evaporated and the residue added to a solution of 6-bromo-1H-indazol-3-amine (1 eq) in pyridine (0.4 M). The resulting mixture was stirred at RT for 4 hours. Volatiles were evaporated and the crude purified by reverse phase column eluted with water (0.1% AcOH)/Acetonitrile (0.1% AcOH) from 10/0 to 0/10 in gradient.

**Table 14**

| **Intermediates 97-100 (Intermediate VIII; scheme 2)** | | | |
|---|---|---|---|
| **intermediates** | **Structure** | **HNMR/MS** | **Yield %** |
| **97** | 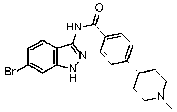 | ¹H NMR (400 MHz, *DMSO-d*6) δ ppm 12.90 (1 H, br. S), 10.78 (1 H, s), 7.98 - 8.04 (2 H, m), 7.72 (2 H, s), 7.38 - 7.44 (2 H, m), 7.19 - 7.27 (1 H, m), 2.89 (2 H,d), 2.21 (3 H, s), 1.93 - 2.07 (2 H, m), 1.65 - 1.81 (4 H, m) | 49 |
| | | ESI + m/z 421 [M+H]⁺ | |
| **98** | 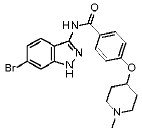 | ¹H NMR (400 MHz, *DMSO-d*6) δ ppm 12.88 (1 H, br. S.), 10.70 (1 H, s), 8.01 - 8.07 (2 H, m), 7.66 - 7.75 (2 H, m), 7.18 - 7.24 (1 H, m), 7.05 - 7.12 (2 H, m), 2.66 - 2.75 (2 H, m), 2.23 - 2.36 (5 H, m), 1.99 (2 H,d), 1.92 (3 H, s), 1.64 - 1.76 (2 H, m) ESI + m/z 431 [M+H]⁺ | 45 |
| **99** | 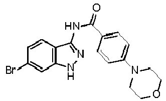 | ¹H NMR (400 MHz, *DMSO-d*6) δ ppm 12.84 (1 H, s), 10.57 (1 H, s), 7.99 (2 H, d), 7.68 - 7.73 (2 H, m),7.20 (1 H, dd), 7.03 (2 H, d), 3.72 - 3.81 (4 H, m), 3.23 - 3.30 (4 H, m) | 58 |
| | | ESI + m/z 402 [M+H]⁺ | |
| **100** | 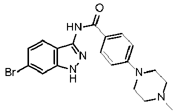 | ¹H NMR (400 MHz, *DMSO-d*6) δ ppm 12.86 (1 H, br. S.), 10.58 (1 H, s), 7.99 (2 H, d), 7.67 - 7.73 (2 H,m), 7.20 (1 H, dd), 7.06 (2 H, d), 3.44 (4 H, br. S.), 2.73 - 2.86 (3 H, m) | 55 |
| | | ESI + m/z 414 [M+H]⁺ | |

### Example 37:

### Preparation of intermediates X (Intermediates 101- 117) of scheme 3

### General procedure 11

A mixture of the aniline (1.1 eq), 2,6-dichloronicotinonitrile (1 eq) and DIPEA (2 eq) in DMSO (0.15 M) were heated at 100 °C upon completion. The crude was loaded on SNAP-C18 gold (50g) cartridges eluting with H₂O-AcOH (0.1%) / CH3CN-AcOH (0.1%). Relevant fractions were collected and evaporated. The residue was dissolved in Ethanol (0.15 M), hydrazine monohydrate (10 eq) was added and the mixture was reflux overnight. Volatiles were evaporated and the residue was purified by reverse phase column eluted with water (0.1% of AcOH)/Acetonitrile (0.1 %of AcOH) from 10/0 to 0/10 in gradient.

### General procedure 12

A mixture of 2,6-dichloronicotinonitrile (51.9 g, 0.30 mol) in DMSO (0.2 M mL), the aniline (1 eq) and DIPEA (1.1 eq) was stirred at 110°C upon completion. The mixture was poured in water and extracted with EtOAc (3 times), the organic phases were combined, dried over Na2SO4 and evaporated. The crude was dissolved in EtOH (0.15 M), hydrazine hydrate (12 eq) was added. The mixture was stirred at 90 °C upon completion. The reaction solution was concentrated in vacuum. The crude was purified by reverse phase column eluted with water (0.1 % of AcOH)/Acetonitrile (0.1% of AcOH) from 10/0 to 0/10 in gradient.

**Table 15**

| **Intermediates 101- 117 (Intermediate X; scheme 3)** | | | | |
|---|---|---|---|---|
| **Intermediate** | **Structure** | **HNMR/MS** | **Procedure** | **Yield %** |
| **101** | 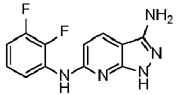 | ¹H NMR (400 MHz, *DMSO-d*6) δ ppm 11.30 - 11.58 (1 H, m), 8.91 - 9.10(1 H, m), 8.03 - 8.14 (1 H, m), 7.81 - 7.90 (1 H, m), 7.09 - 7.20 (1 H, m), 6.94 - 7.07 (1 H, m), 6.54 - 6.73 (1 H, m), 5.14 - 5.37 (2 H, m) | 11 | 26 |
| | | ESI + m/z 262 [M+H]⁺ | | |
| **102** | 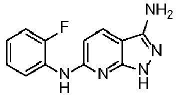 | ¹H NMR (400 MHz, *DMSO-d*6) δ ppm 11.41 (1 H, br. S.), 8.76 (1 H, s), 8.28 (1 H, td), 7.82 (1 H, d), 7.22 (1 H, ddd), 7.12 - 7.17 (1 H, m), 6.96 - 7.05 (1 H, m), 6.62 (1 H, d), 5.23 (2 H, br. S.) | 12 | 40 |
| | | ESI + m/z 244 [M+H]⁺ | | |
| **103** | 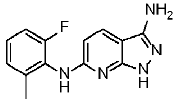 | ¹H NMR (400 MHz, *DMSO-d*6) δ ppm 11.21 (1 H, br. S.), 8.33 (1 H, s), 7.76 (1 H, d), 7.00 - 7.21 (3 H,m), 6.32 (1 H, d), 5.76 (1 H, s), 5.15 (2 H, s), 2.21 (3 H, s) | 11 | 26 |
| | | ESI + m/z 258 [M+H]⁺ | | |
| **104** | 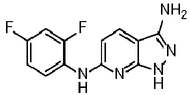 | ¹H NMR (400 MHz, *DMSO-d*6) δ ppm 11.30 - 11.46 (1 H, m), 8.73 (1 H, s), 8.08 - 8.22 (1 H, m), 7.82 (1H, d), 7.28 (1 H, s), 7.00 - 7.11 (1 H, m), 6.55 (1 H, d), 5.21 (2 H, s), 1.75 (1 H, s) | 12 | 30 |
| | | ESI + m/z 262 [M+H]⁺ | | |
| **105** | 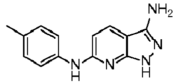 | ¹H NMR (400 MHz, *DMSO-d*6) δ ppm 11.25 - 11.57 (1 H, m), 9.04 (1 H, s), 7.78 (1 H, d), 7.60 - 7.71 (2H, m), 7.09 (2 H, d), 6.42 (1 H, d), 5.27 (2 H, br. S.), 2.26 (3 H, s) | 12 | 38 |
| | | ESI + m/z 240 [M+H]⁺ | | |
| **106** | 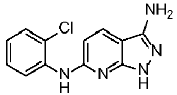 | ¹H NMR (400 MHz, *DMSO-d*6) δ ppm 11.32 - 11.49 (1 H, m), 8.80 - 8.89 (1 H, m), 8.40 - 8.49 (1 H, m),8.07 - 8.13 (1 H, m), 7.84 (1 H, d), 7.45-7.50(2 H, m), 7.27 - 7.35 (2 H, m), 7.03 - 7.09 (1 H, m), 6.63(1 H, d), 5.23 (2 H, s) | 12 | 24 |
| | | ESI + m/z 240 [M+H]⁺ | | |
| **107** | 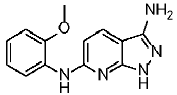 | ¹H NMR (400 MHz, *DMSO-d*6) δ ppm 11.38 (1 H, br. S.), 8.33 - 8.43 (1 H, m), 8.12 - 8.20 (1 H, m), 7.78(1 H, d), 7.00 - 7.06 (1 H, m), 6.87 - 6.99 (2 H, m), 6.66 (1 H, d), 5.22 (2 H, br. S.), 3.86 (3 H, s) | 12 | 74 |
| | | ESI + m/z 256 [M+H]⁺ | | |
| **108** | 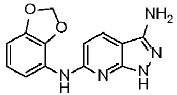 | ¹H NMR (400 MHz, *DMSO-d*6) δ ppm 11.37 (1 H, s), 9.03 (1 H, s), 7.77 (1 H, d), 7.64 (1 H, d), 7.00 -7.12 (1 H, m), 6.84 (1 H, d), 6.37 (1 H, d), 5.96 (2 H, s), 5.18 (2 H, s) | 12 | 45 |
| | | ESI + m/z 270 [M+H]⁺ | | |
| **109** | 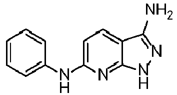 | ¹H NMR (400 MHz, *DMSO-d*6) δ ppm 11.40 (1 H, s), 9.15 (1 H, s), 7.77-7.83 (3 H, m), 7.24 - 7.31 (2H, m), 6.87 - 6.94 (1 H, m), 6.45 (1 H, d), 5.20 (2 H, s) | 12 | 50 |
| | | ESI + m/z 226 [M+H]⁺ | | |
| **110** | 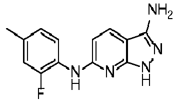 | ESI + m/z 258 [M+H]⁺ | 12 | 74 |
| **111** | 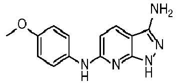 | ¹H NMR (400 MHz, *DMSO-d*6) δ ppm 11.31 (1 H, s), 8.94 (1 H, s), 7.75 (1 H, d), 7.67 (2 H, d), 6.88 (2H, d), 6.37 (1 H, d), 5.16 (2 H, s), 3.73 (3 H, s) | 12 | 68 |
| | | ESI + m/z 256 [M+H]⁺ | | |
| **112** | 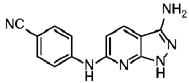 | ¹H NMR (400 MHz, *DMSO-d*6) δ ppm 11.62 (1 H, br. S.), 9.75 (1 H, s), 7.96-8.06 (2 H, m), 7.91 (1 H,d), 7.66 - 7.76 (2 H, m), 6.54 (1 H, d), 5.32 (2 H, s) | 12 | 42 |
| | | ESI + m/z 251 [M+H]⁺ | | |
| **113** | 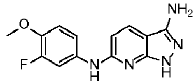 | ¹H NMR (400 MHz, *DMSO-d*6) δ ppm 11.42 (1 H, br. S.), 9.17 (1 H, s), 8.00 (1 H, dd), 7.79 (1 H, d), 7.25 - 7.35 (1 H, m), 7.09 (1 H, t), 6.39 (1 H, d), 5.20 (2 H, s), 3.80 (3 H, s) | 12 | 57 |
| | | ESI + m/z 274 [M+H]⁺ | | |
| **114** | 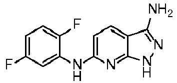 | ¹H NMR (400 MHz, *DMSO-d*6) δ ppm 11.62 (2 H, br. S.), 9.01 (1 H, s), 8.52 (1 H, ddd), 8.07 - 8.13 (1 H,m), 7.87 (1 H, d), 7.26 (1 H, ddd), 6.70 - 6.81 (2 H, m), 5.28 (2 H, br. S.),1.92 (2 H, s). | 12 | 5 |
| | | ESI + m/z 262 [M+H]⁺ | | |
| **115** | 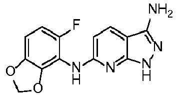 | ESI + m/z 288 [M+H]⁺ | 12 | 6 |
| **116** | 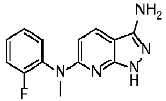 | ESI + m/z 258 [M+H]⁺ | 12 | 62 |
| **117** | 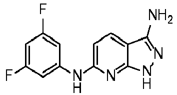 | ¹H NMR (400 MHz, *DMSO-d*6) δ ppm 9.64 - 9.79 (1 H, m), 8.16 - 8.30 (1 H, m), 7.59 (3 H, s), 6.65 -6.80 (1 H, m), 6.09 (1 H, d), 4.32 - 4.52 (2 H, m) | 12 | 16 |
| | | ESI + m/z 262 [M+H]⁺ | | |

### Example 38

### Preparation of intermediate 118

### N-(6-((2-fluorophenyl)amino)-1H-indazol-3-yl)-4-(1-methylpiperidin-4-yl)-2-(2,2,2-trifluoro-N-(tetrahydro-2H-pyran-4-yl)acetamido)benzamide

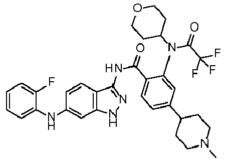

Oxalyl chloride (200 µL, 2.285 mmol) and 1 drop of anhydrous dimethylformamide were added to a solution of **intermediate 42** in DCM (6 ml). The reaction mixture was stirred for 2 hours at room temperature. Volatiles were evaporated and added to a solution of **intermediate 102** (92 mg, 0.378 mmol) and DIPEA (264 µL, 1.514 mmol) in THF (5 ml) at -20 °C. The reaction mixture was stirred for 2 hours at -20 °C. THF was evaporated and the residue was loaded onto a RediSep C18 30g column eluting with water + 0.1% AcOH only to acetonitrile + 0.1% AcOH only. Relevant fractions were collected and evaporated to give title compound (45 mg; 16% yield). ESI + m/z 639 [M+H]⁺.

### Example 39

### General procedures used to prepare compounds of inventions (formula I):

### General procedure 13:

To a solution of **Intermediates VII** (1 eq) in DCM (ratio 1: 0.03 M) was added TFA (ratio 1, 0.03 M). After stirred at room temperature for 3-5 hours, DCM was removed under vacuum. The residue was purified by reverse phase column eluted with water (0.1% of AcOH)/Acetonitrile (0.1% of AcOH) from 10/0 to 0/10 in gradient

### General procedure 14:

Benzoic acid **intermediates VI (intermediates 32-41 and 43)** (2eq) was suspended in DCM (0.1M), oxalyl chloride (6 eq) was added and the mixture was stirred at rt upon completion. Volatiles were evaporated and the residue added to a solution of **intermediate IV (intermediates 57-68) or intermediate X (intermediates 101-117)** (1 eq) in pyridine (0.1M) and the resulting mixture stirred at RT overnight. The solvent was evaporated and the residue was suspended in EtOH (0.1M) and NaOH 2 N (0.02M) and stirred for 30 min at 50°C. The solution was acidified with AcOH, the EtOH evaporated and the residue purified by reverse phase chromatography eluted with water (0.1 %of AcOH)/Acetonitrile(0.1 %of AcOH) from 10/0 to 0/10 in gradient.

### General procedure 15:

Commercially available aniline (4 eq) was added to a mixture of **Intermediate VIII (intermediates 97-100)** (1 eq), CS₂CO₃ (2 eq), Tris(dibenzylideneacetone)dipalladium (0) (0.14 eq), tBuXPhos (0.35) in dry tert-butanol (0.08 M). The reaction mixture was heated at 110°C upon completion, then cooled to RT and filtered through a pad of celite, which was washed with AcOEt (2 times). All the organic layers were evaporated and the residue was purified by reverse phase chromatography eluted with water (0.1 % AcOH)/Acetonitrile(0.1% AcOH) from 10/0 to 0/10 in gradient.

With reference to the below table 16, following the procedures above indicated (3^{rd} column of the table) by starting from intermediates above prepared (and indicated in the 2^{nd} column of the below table) the compounds 1-52 have been prepared:

**Table 16**

| **Compounds 1- 52** | | | |
|---|---|---|---|
| **Compound** | **Intermediate** | **Procedure** | **Yield** |
| **1** | **83** | 13 | 72 |
| 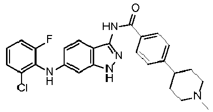 | ¹H NMR (400 MHz, *DMSO-d*6) δ ppm δ11.00 (s, 1H), 10.86 (s, 1H), 8.05 (d, 2H, *J*₁ = 7.5 Hz), 7.61 (d, 1H, *J*₁ = 9.0 Hz), 7.46-7.28 (m, 5H), 6.71 (d, 3H, *J*₁ = 9.0 Hz), 6.30 (s, 1H), 3.48 (d, 2H, *J*₁ = 11.4 Hz), 3.09-3.05 (m, 2H), 2.90-2.86 (m, 1H), 2.75 (d, 3H, *J*₁ = 4.2 Hz), 2.13-1.96 (m, 4H) | | |
| | ESI+ m/z 478-480[M+H]+ | | |
| N-(6-((2-chloro-6-fluorophenyl)amino)-1H-indazol-3-yl)-4-(1-methylpiperidin-4-yl)benzamide | | | |

| **2** | **84** | 13 | 85 |
|---|---|---|---|
| 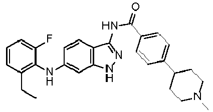 | ¹H NMR (400 MHz, *DMSO-d*6) δ ppm 610.9-10.8 (ss, 2H), 8.06-8.03 (d, 2H), 7.63-7.60 (d, 1H), 7.41-7.38 (d, 2H), 7.23-7.15 (m, 3H), 6.71-6.88 (d, 1H), 6.13 (s, 1H), 3.49-3.45 (d, 2H), 3.13-3.04 (q, 2H), 2.89-2.85 (m, 1H), 2.75-2.74 (s, 3H), 2.64-2.62 (q, 2H), 2.13-2.01 (m, 4H), 1.15-1.06 (t, 3H) | | |
| | ESI+ m/z 472[M+H]+ | | |
| N-(6-((2-ethyl-6-fluorophenyl)amino)-1H-indazol-3-yl)-4-(1-methylpiperidin-4-yl)benzamide | | | |

| **3** single unknown enantiomer | **68 and 40** | 14 | 11 |
|---|---|---|---|
| 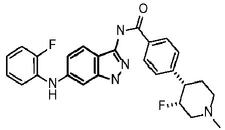 | ¹H NMR (400 MHz, *DMSO-d*6) δ ppm 12.27 (1 H, s), 10.62 (1 H, s), 8.06 - 8.13 (1 H, m), 8.02 (2 H, d),7.54 - 7.62 (1 H, m), 7.49 (2 H, d), 7.34 - 7.43 (1 H, m), 7.20 - 7.30 (1 H, m), 7.10 - 7.19 (1 H, m), 6.95 -7.06 (1 H, m), 6.79 - 6.89 (2 H, m), 4.63 - 4.92 (1 H, m), 3.17 - 3.26 (1 H, m), 2.70 - 2.86 (2 H, m), 2.29(3 H, s), 1.94 - 2.11 (2 H, m), 1.67 - 1.89 (2 H, m) | | |
| | ESI+ m/z 462[M+H]+ | | |
| *rel*((3S,4R)-3-fluoro-1-methylpiperidin-4-yl)-N-(6-((2-fluorophenyl)amino)-1H-indazol-3-yl)benzamide | | | |

| **4** single unknown enantiomer | **68 and 40** | 14 | 12 |
|---|---|---|---|
| 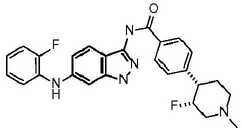 | ¹H NMR (400 MHz, *DMSO-d*6) δ ppm 12.27 (1 H, s), 10.62 (1 H, s), 8.06 - 8.13 (1 H, m), 8.02 (2 H, d),7.54 - 7.63 (1 H, m), 7.50 (2 H, s), 7.36 - 7.43 (1 H, m), 7.20 - 7.30 (1 H, m), 7.10 - 7.19 (1 H, m), 6.96 -7.05 (1 H, m), 6.78 - 6.90 (2 H, m), 4.66 - 4.91 (1 H, m), 3.19 - 3.26 (1 H, m), 2.70 - 2.89 (2 H, m), 2.29 (3H, s), 1.97-2.09 (2H,m), 1.72-1.85 (2H,m). | | |
| | ESI+ m/z 462[M+H]+ | | |
| *rel*((3R,4S)-3-fluoro-1-methylpiperidin-4-yl)-N-(6-((2-fluorophenyl)amino)-1H-indazol-3-yl)benzamide | | | |

| **5** | **102 and 32** | 14 | 66 |
|---|---|---|---|
| 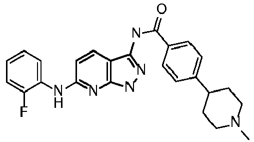 | ¹H NMR (400 MHz, *DMSO-d*6) δ ppm 12.70 (1 H, s), 10.74 (1 H, s), 9.01 (1 H, s), 8.28 (1 H, d), 7.95 -8.10 (3 H, m), 7.40 (2 H, d), 7.23 - 7.30 (1 H, m), 7.19 (1 H, s), 7.02 - 7.12 (1 H, m), 6.82 (1 H, d), 3.15 -3.20 (0 H, m), 2.89 (2 H, d), 2.54 - 2.61 (1 H, m), 1.99 (2 H, d), 1.64 - 1.82 (4 H, m) | | |
| | ESI+ m/z 445[M+H]+ | | |
| N-(6-((2-fluorophenyl)amino)-1H-pyrazolo[3,4-b]pyridin-3-yl)-4-(1-methylpiperidin-4-yl)benzamide | | | |

| **6** | **85** | 13 | 82 |
|---|---|---|---|
| 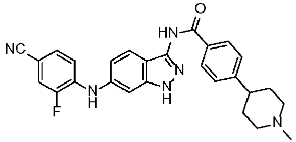 | ¹H-NMR (300 MHz, DMSO-*d*₆): δ ppm 10.9-10.8 (m, 2H), 8.89-8.86 (m, 1H), 8.08-8.05 (m, 2H), 7.80-7.68 (m, 2H), 7.52-7.36 (m, 4H), 7.22 (s, 1H), 7.01-6.98 (m, 1H), 6.04-5.91 (m, 3H), 3.52-3.47 (m, 2H), 3.10-3.06 (m, 2H), 2.93-2.88 (m, 1H), 2.77-2.75 (m, 3H), 2.13-1.99 (m, 4H) | | |
| | ESI+ m/z 469[M+H]+ | | |
| N-(6-((4-cyano-2-fluorophenyl)amino)-1H-indazol-3-yl)-4-(1-methylpiperidin-4-yl)benzamide | | | |

| **7** | **68 and 35** | 14 | 4 |
|---|---|---|---|
| 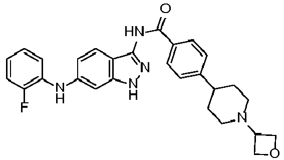 | ¹H NMR (400 MHz, *DMSO-d*6) δ ppm 12.26 (1 H, s), 10.59 (1 H, s), 8.09 (1 H, s), 8.01 (2 H, d), 7.54 -7.60 (1 H, m), 7.35 - 7.45 (3 H, m), 7.26 (1 H, ddd), 7.12 - 7.18 (1 H, m), 6.96 - 7.04 (1 H, m), 6.81 -6.87 (2 H, m), 4.53 - 4.60 (2 H, m), 4.47 (2 H, br. S.), 3.44 (1 H, br. S.), 2.84 (2 H, d), 2.63 (1 H, br. S.),1.65 - 1.95 (6 H, m) | | |
| | ESI+ m/z 486[M+H]+ | | |
| N-(6-((2-fluorophenyl)amino)-1H-indazol-3-yl)-4-(1-(oxetan-3-yl)piperidin-4-yl)benzamide | | | |

| **8** | **86** | 13 | 66 |
|---|---|---|---|
| 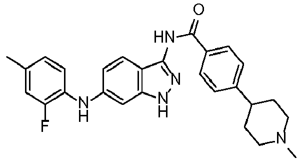 | ¹H-NMR (300 MHz, DMSO-*d*₆): δ ppm: 10.98 (s, 1H), 10.89 (s, 1H), 8.05 (d, 2H, *J*₁ = 7.8 Hz), 7.63 (d, 1H, *J*₁ = 9.0 Hz), 7.40 (d, 2H, *J*₁ = 8.1 Hz), 7.26 (t, 1H, *J*₁ = 8.4 Hz), 7.10 (d, 1H, *J*₁ = 11.1 Hz), 6.98 (d, 1H, *J*₁ = 7.8 Hz), 6.80 (d, 1H, *J*₁ = 9.3 Hz), 6.67 (s, 1H), 4.60-4.45 (m, 8H), 3.48 (d, 2H, *J*₁ = 11.7 Hz), 3.09-3.05 (m, 2H), 2.90-2.84 (m, 1H), 2.75 (d, 3H, *J*₁ = 4.2 Hz), 2.99 (s, 3H), 2.13-1.94 (m, 4H). | | |
| | ESI+ m/z 458[M+H]+ | | |
| N-(6-((2-fluoro-4-methylphenyl)amino)-1H-indazol-3-yl)-4-(1-methylpiperidin-4-yl)benzamide | | | |

| **9** | **87** | 13 | <5 |
|---|---|---|---|
| 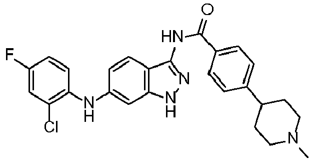 | ¹H-NMR (300 MHz, DMSO-*d*₆): δ ppm: 12.3 (s, 1H), 10.6 (s, 1H), 8.01-7.98 (d, 2H), 7.87 (s, 1H), 7.57-7.50 (dd, 2H), 7.48-7.37 (m, 3H), 7.22-7.18 (m, 1H), 6.81-6.78 (d, 1H), 6.72 (s, 1H), 2.90-2.88 (d, 2H), 2.58-2.53 (m, 1H), 2.20 (s, 3H), 2.03-1.95 (m, 2H), 1.77-1.67 (m, 4H) | | |
| | ESI+ m/z 477-479 [M+H]+ | | |
| N-(6-((2-chloro-4-fluorophenyl)amino)-1H-indazol-3-yl)-4-(1-methylpiperidin-4-yl)benzamide | | | |

| **10** | **88** | 13 | 75 |
|---|---|---|---|
| | ¹H-NMR (300 MHz, DMSO-*d*₆): δ ppm: 10.96 (s, 1H), 10.83 (s, 1H), 8.06 (d, 2H, *J*₁ = 7.5 Hz), 7.72-7.56 (m, 4H), 7.45-7.39 (m, 3H), 7.21 (t, 1H, *J*₁ = 4.5 Hz), 6.87-6.84 (m, 2H), 5.18-5.10 (m, 6H), 3.50-3.46 (m, 2H), 3.09-3.05 (m, 2H), 2.90-2.86 (m, 1H), 2.75 (d, 3H, *J*₁ = 3.9 Hz), 2.12-1.97 (m, 5H). | | |
| 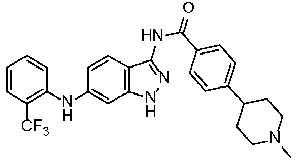 | ESI+ m/z 494[M+H]+ | | |
| 4-(1-methylpiperidin-4-yl)-N-(6-((2-(trifluoromethyl)phenyl)amino)-1H-indazol-3-yl)benzamide | | | |

| **11** | **89** | 13 | 99 |
|---|---|---|---|
| 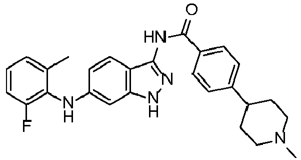 | ¹H-NMR (300 MHz, DMSO-*d*₆): δ ppm: 12.13 (s, 1H), 10.67 (s, 1H), 8.05-8.03 (d, 2H), 7.51-7.49 (m, 1H), 7.40-7.14 (m, 5H), 6.66-6.64 (d, 1H), 6.13 (d, 1H), 4.77-3.99 (m 6H), 3.50-3.48 (d, 1H), 3.08 (s, 1H), 2.87 (s, 1H), 2.77 (s, 2H), 2.23 (s, 3H), 2.02-2.01 (s, 4H) | | |
| | ESI+ m/z 458[M+H]+ | | |
| N-(6-((2-fluoro-6-methylphenyl)amino)-1H-indazol-3-yl)-4-(1-methylpiperidin-4-yl)benzamide | | | |

| **12** | **90** | 13 | 64 |
|---|---|---|---|
| 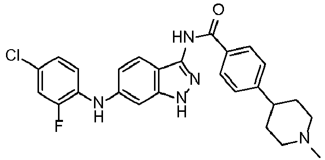 | ¹H-NMR (300 MHz, DMSO-*d*₆): δ ppm: 10.95 (s, 1H), 10.81 (s, 1H), 8.06 (d, 2H, *J*₁ = 8.1 Hz), 7.64 (d, 1H, *J*₁ = 9.3 Hz), 7.48-7.35 (m, 4H), 7.22-7.19 (m, 1H), 6.85-6.87 (m, 2H), 5.32-5.22 (m, 7H), 3.51-3.46 (m, 2H), 3.12-3.03 (m, 2H), 2.90-2.88 (m, 1H), 2.76-2.74 (m, 3H), 2.17-1.98 (m, 4H) | | |
| | ESI+ m/z 478[M+H]+ | | |
| N-(6-((4-chloro-2-fluorophenyl)amino)-1H-indazol-3-yl)-4-(1-methylpiperidin-4-yl)benzamide | | | |

| **13** | **91** | 13 | 71 |
|---|---|---|---|
| 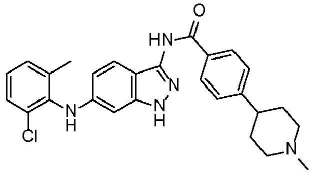 | ¹H NMR (400 MHz, *DMSO-d*6) δ ppm: 11.0 (s, 1H), 10.9 (s, 1H), 8.06-8.04 (d, 2H), 7.62-7.59 (d, 2H), 7.44-7.22 (m, 5H), 6.69-6.66 (d, 1H), 6.01 (s, 1H), 5.38 (s, 4H), 3.50-3.45 (d, 2H),3.09-3.05 (q, 2H), 2.93-2.86 (m, 1H), 2.76-2.74 (d, 3H), 2.22 (s, 3H), 2.12-1.97 (m, 4H). | | |
| | ESI+ m/z 474-476[M+H]+ | | |
| N-(6-((2-chloro-6-methylphenyl)amino)-1H-indazol-3-yl)-4-(1-methylpiperidin-4-yl)benzamide | | | |

| **14** | **92** | 13 | 29 |
|---|---|---|---|
| 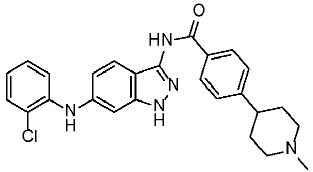 | ¹H NMR (400 MHz, *DMSO-d*6) δ ppm: 612.32 (s, 1H), 10.60 (s, 1H), 8.00-7.98 (d, 2H), 7.59-7.56 (d, 1H), 7.48-7.24 (m, 5H), 7.00-6.86 (m, 3H), 2.92-2.89 (d, 2H), 2.57-2.53 (m, 1H), 2.22 (s, 3H), 2.05-1.99 (m, 2H), 1.79-1.68 (m, 4H) | | |
| | ESI+ m/z 460-462[M+H]+ | | |
| N-(6-((2-chlorophenyl)amino)-1H-indazol-3-yl)-4-(1-methylpiperidin-4-yl)benzamide | | | |

| **15** | **93** | 13 | 71 |
|---|---|---|---|
| 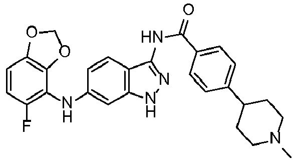 | ¹H NMR (400 MHz, *DMSO-d*6) δ ppm 11.1 (s, 1H), 11.0 (s, 1H), 8.08-8.05 (d, 2H), 7.68-7.65 (d, 1H), 7.41-7.39 (d, 2H),6.80-6.72 (m, 4H), 6.48 (s, 1H), 6.06 (s, 2H), 3.50-3.45 (d, 2H), 3.09-3.05 (q, 2H), 2.89-2.84 (m, 1H), 2.75-2.73 (d, 3H), 2.15-1.96 (m, 4H). | | |
| | ESI+ m/z 488[M+H]+ | | |
| N-(6-((5-fluorobenzo[d][1,3]dioxol-4-yl)amino)-1H-indazol-3-yl)-4-(1-methylpiperidin-4-yl)benzamide | | | |

| **16** | **99** | 15 | 13 |
|---|---|---|---|
| 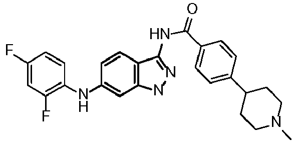 | ¹H NMR (400 MHz, *DMSO-d*6) δ ppm 12.21 (1 H, s), 10.57 (1 H, s), 7.96 - 8.04 (3 H, m), 7.52 - 7.58 (1 H, m), 7.40 (4 H, d), 7.02 - 7.11 (1 H, m), 6.72 - 6.81(1 H, m), 6.68 (1 H, s), 2.83 - 2.96 (2 H, m), 2.54 - 2.61 (1 H, m), 2.22 (3 H, s), 1.94 - 2.05 (2 H, m), 1.64 - 1.82 (4 H, m) | | |
| | ESI+ m/z 462 [M+H]+ | | |
| N-(6-((2,4-difluorophenyl)amino)-1H-indazol-3-yl)-4-(1-methylpiperidin-4-yl)benzamide | | | |

| **17** | **97** | 15 | 12 |
|---|---|---|---|
| 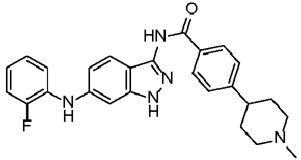 | ¹H NMR (400 MHz, *DMSO-d*6) δ ppm :12.26 (1 H, s), 10.58 (1 H, s), 8.09 (1 H, s), 8.00 (2 H, d), 7.57 (1H, d), 7.35 - 7.43 (3 H, m), 7.26 (1 H, ddd), 7.14 (1 H, td), 6.97 - 7.04 (1 H, m), 6.81 - 6.88 (2 H, m), 2.90 (2 H, d), 2.54 - 2.61 (1 H, m), 2.22 (3 H, s), 1.95 - 2.06 (2 H, m), 1.61 - 1.84 (4 H, m | | |
| | ESI+ m/z 444 [M+H]+ | | |
| N-(6-((2-fluorophenyl)amino)-1H-indazol-3-yl)-4-(1-methylpiperidin-4-yl)benzamide | | | |

| **18** | **97** | 15 | 21 |
|---|---|---|---|
| 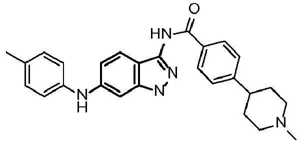 | ¹H NMR (400 MHz, *DMSO-d*6) δ ppm 12.20 (1 H, s), 10.56 (1 H, s), 8.16 (1 H, s), 8.00 (2 H, d), 7.54 (1 H, d), 7.40 (2 H, d), 7.04 - 7.15 (4 H, m), 6.94 (1 H, d),6.72 - 6.81 (1 H, m), 2.89 (2 H, d), 2.55 - 2.61 (1 H, m), 2.26 (3 H, s), 2.21 (3 H, s), 1.99 (2 H, td), 1.61 - 1.83 (4 H, m) | | |
| | ESI+ m/z 440 [M+H]+ | | |
| 4-(1-methylpiperidin-4-yl)-N-(6-(p-tolylamino)-1H-indazol-3-yl)benzamide | | | |

| **19** | **97** | 15 | 36 |
|---|---|---|---|
| 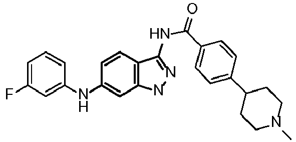 | ¹H NMR (400 MHz, *DMSO-d*6) δ ppm 12.33 (1 H, s), 10.61 (1 H, s), 8.57 (1 H, s), 8.00 (2 H, d), 7.62 (1 H, d), 7.40 (2 H, d), 7.24 - 7.33 (1 H, m), 7.11 (1 H, d),6.94 - 6.98 (1 H, m), 6.82 - 6.92 (2 H, m), 6.63 (1 H, d), 2.85 - 2.99 (2 H, m), 2.54 - 2.60 (1 H, m), 2.22 (3 H, s), 1.96 - 2.07 (2 H, m), 1.64 - 1.83 (4 H, m) | | |
| | ESI+ m/z 444 [M+H]+ | | |
| N-(6-((3-fluorophenyl)amino)-1H-indazol-3-yl)-4-(1-methylpiperidin-4-yl)benzamide | | | |

| **20** | **101 and 32** | 14 | 20 |
|---|---|---|---|
| 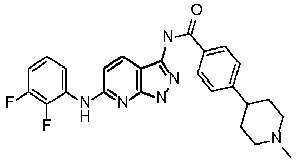 | ¹H NMR (400 MHz, DMSO-d₆) δ ppm 12.76 (1 H, br. S.), 10.77 (1 H, s), 9.24 (1 H, s), 8.11 - 8.11 (1 H, m), 8.04 - 8.12 (2 H, m), 8.01 (2 H, d), 7.40 (2 H, d), 7.14 - 7.24 (1 H, m), 7.02 - 7.13 (1 H, m), 6.85 (1 H, d), 2.86 - 2.99 (2 H, m), 2.55 -2.65(1 H, m), 2.20 - 2.31 (3 H, m), 1.95 - 2.14 (2 H, m), 1.92 (3 H, s), 1.63 - 1.84 (4 H, m) | | |
| | ESI+ m/z 463 [M+H]+ | | |
| N-(6-((2,3-difluorophenyl)amino)-1H-pyrazolo[3,4-b]pyridin-3-yl)-4-(1-methylpiperidin-4-yl)benzamide | | | |

| **21** | **103 and 32** | 14 | 9 |
|---|---|---|---|
| 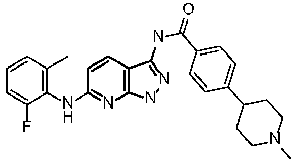 | ¹H NMR (400 MHz, *DMSO-d*6) δ ppm 12.52 (1 H, br. S.), 10.69 (1 H, s), 8.59 (1 H, s), 7.92 - 8.06 (3 H,m), 7.40 (2 H, d), 7.16 - 7.24 (1 H, m), 7.06 - 7.15 (2 H, m), 6.55 (1 H, d), 2.85 - 3.05 (2 H, m), 2.55 -2.64 (2 H, m), 2.24 (5 H, s), 2.00 - 2.18 (2 H, m), 1.92 (3 H, s), 1.62 - 1.85 (4 H, m) | | |
| | ESI+ m/z 459 [M+H]+ | | |
| N-(6-((2-fluoro-6-methylphenyl)amino)-1H-pyrazolo[3,4-b]pyridin-3-yl)-4-(1-methylpiperidin-4-yl)benzamide, Acetic acid salt | | | |

| **22** | **104 and 32** | 14 | 30 |
|---|---|---|---|
| 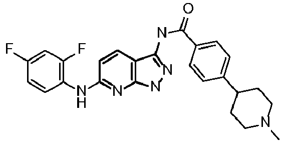 | ¹H NMR (400 MHz, *DMSO-d*6) δppm 12.68 (1H, br. S.), 11.47 - 12.30 (1H, m), 10.74 (1H, s), 8.98 (1H, s), 8.14 (1 H, td), 8.04 (1 H, d), 8.00 (2 H, d), 7.40 (2 H, d), 7.28 - 7.36 (1 H, m), 7.06 - 7.14 (1 H, m),6.75 (1 H, d), 2.89 (2 H, d), 2.53 - 2.61 (2 H, m), 2.21 (3 H, s), 1.94 - 2.04 (2 H, m), 1.91 (3 H, s), 1.64 -1.82 (4 H, m) | | |
| | ESI+ m/z 463 [M+H]+ | | |
| N-(6-((2,4-difluorophenyl)amino)-1H-pyrazolo[3,4-b]pyridin-3-yl)-4-(1-methylpiperidin-4-yl)benzamide, Acetic acid salt | | | |

| **23** | **105 and 32** | 14 | 41 |
|---|---|---|---|
| 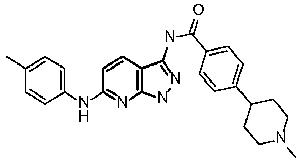 | ¹H NMR (400 MHz, *DMSO-d*6) δ ppm 12.55 - 12.77 (1 H, m), 10.72 (1 H, s), 9.26 (1 H, s), 7.96 - 8.05 (3H, m), 7.73 (2 H, d), 7.41 (2 H, s), 7.07 - 7.16 (2 H, m), 6.57 - 6.67 (1 H, m), 2.85 - 2.96 (2 H, m), 2.54 -2.64 (1 H, m), 2.28 (3 H, s), 2.22 (3 H, s), 1.94 - 2.06 (2 H, m), 1.91 (2 H, s), 1.63 - 1.83 (4 H, m) | | |
| | ESI+ m/z 442 [M+H]+ | | |
| 4-(1-methylpiperidin-4-yl)-N-(6-(p-tolylamino)-1H-pyrazolo[3,4-b]pyridin-3-yl)benzamide | | | |

| **24** | **102 and 34** | 14 | 20 |
|---|---|---|---|
| 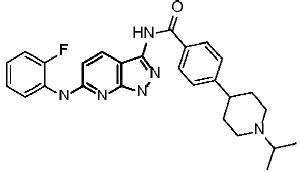 | ¹H NMR (400 MHz, *DMSO-d*6) δ ppm 12.73 (1 H, br. S.), 10.80 (1 H, s), 9.41 (1 H, br. S.), 9.02 (1 H, s),8.27 (1 H, td), 8.06 (3 H, dd), 7.41 (2 H, d), 7.26 (1 H, ddd), 7.20 (1 H, t), 7.03 - 7.12 (1 H, m), 6.83 (1H, d), 3.53 - 3.60 (1 H, m), 3.49 (2 H, d), 3.06 - 3.19 (2 H, m), 2.94 - 3.03 (1 H, m), 1.91 - 2.14 (4 H, m),1.24 - 1.36 (6 H, m) | | |
| | ESI+ m/z 473 [M+H]+ | | |
| N-(6-((2-fluorophenyl)amino)-1H-pyrazolo[3,4-b]pyridin-3-yl)-4-(1-isopropylpiperidin-4-yl)benzamide, Hydrochloride salt | | | |

| **25** | **102 and 43** | 14 | 11 |
|---|---|---|---|
| 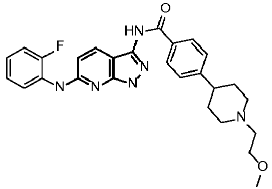 | ¹H NMR (400 MHz, *DMSO-d*6) δ ppm 12.71 (1 H, br. S.), 10.80 (1 H, s), 10.02 (1 H, br. S.), 9.03 (1 H,s), 8.27 (1 H, td), 8.05 (3 H, dd), 7.41 (2 H, d), 7.16 - 7.32 (2 H, m), 7.04 - 7.12 (1 H, m), 6.83 (1 H, d),3.71 - 3.80 (2 H, m), 3.61 (2 H, d), 3.34 - 3.38 (3 H, m), 3.32 (2 H, d), 3.11 (2 H, d), 2.88 - 2.97 (1 H,m), 1.92 - 2.17 (4 H, m) | | |
| | ESI+ m/z 489 [M+H]+ | | |
| N-(6-((2-fluorophenyl)amino)-1H-pyrazolo[3,4-b]pyridin-3-yl)-4-(1-(2-methoxyethyl)piperidin-4-yl)benzamide, Hydrochloride salt | | | |

| **26** | **106 and 32** | 14 | 11 |
|---|---|---|---|
| 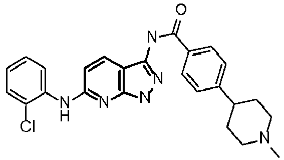 | ¹H NMR (400 MHz, *DMSO-d*6) δ ppm 12.68 (1 H, br. S.), 10.74 (1 H, s), 8.74 (1 H, s), 8.03 - 8.11 (2 H,m), 8.00 (2 H, d), 7.51 (1 H, dd), 7.40 (2 H, d), 7.32 - 7.38 (1 H, m), 7.08 - 7.16 (1 H, m), 6.81 (1 H, d),2.83 - 2.95 (2 H, m), 2.54 - 2.62 (1 H, m), 2.23 (3 H, s), 1.96 - 2.07 (2 H, m), 1.92 (3 H, s), 1.63 - 1.83 (4H, m) | | |
| | ESI+ m/z 461 [M+H]+ | | |
| N-(6-((2-chlorophenyl)amino)-1H-pyrazolo[3,4-b]pyridin-3-yl)-4-(1-methylpiperidin-4-yl)benzamide, Acetic acid salt | | | |

| **27** | **107 and 32** | 14 | 32 |
|---|---|---|---|
| 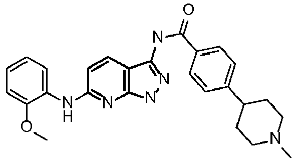 | ¹H NMR (400 MHz, *DMSO-d*6) δ ppm 12.65 (1 H, s), 10.72 (1 H, s), 8.43 (1 H, s), 8.40 (1 H, dd), 8.00(3 H, dd), 7.40 (2 H, d), 6.92 - 7.08 (3 H, m), 6.86 (1 H, d), 3.87 (3 H, s), 3.16 - 3.20 (1 H, m), 2.86 -2.96 (2 H, m), 2.55 - 2.62 (1 H, m), 2.20 - 2.27 (4 H, m), 1.97 - 2.09 (3 H, m), 1.65 - 1.82 (4 H, m) | | |
| | ESI+ m/z 457 [M+H]+ | | |
| N-(6-((2-methoxyphenyl)amino)-1H-pyrazolo[3,4-b]pyridin-3-yl)-4-(1-methylpiperidin-4-yl)benzamide | | | |

| **28** | **108 and 32** | 14 | 54 |
|---|---|---|---|
| 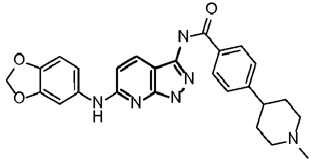 | ¹H NMR (400 MHz, *DMSO-d*6) δ ppm 12.65 (1 H, br. S.), 10.72 (1 H, s), 9.26 (1 H, s), 8.00 (3 H, d),7.69 (1 H, d), 7.40 (2 H, d), 7.11 (1 H, dd), 6.88 (1 H, d), 6.58 (1 H, d), 5.99 (2 H, s), 2.91 (2 H, d), 2.55- 2.62 (1 H, m), 2.23 (3 H, s), 2.02 (2 H, t), 1.66 - 1.82 (4 H, m) | | |
| | ESI+ m/z 471 [M+H]+ | | |
| N-(6-(benzo[d][1,3]dioxol-5-ylamino)-1H-pyrazolo[3,4-b]pyridin-3-yl)-4-(1-methylpiperidin-4-yl)benzamide | | | |

| **29** | **109 and 32** | 14 | 43 |
|---|---|---|---|
| 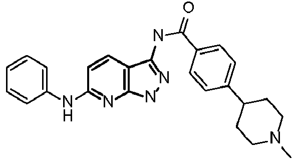 | ¹H NMR (400 MHz, DMSO-d₆) δ ppm 12.70 (1 H, s), 10.75 (1 H, s), 9.38 (1 H, s), 8.02 (3 H, dd), 7.86 (2 H, d), 7.40 (2 H, d), 7.32 (2 H, t), 6.96 (1 H, t), 6.66 (1 H, d), 2.92 (2 H, d), 2.54 - 2.64 (1 H, m), 2.24 (3 H, s), 2.04 (2 H, t), 1.65 - 1.84 (4 H, m) | | |
| | ESI+ m/z 427 [M+H]+ | | |
| 4-(1-methylpiperidin-4-yl)-N-(6-(phenylamino)-1H-pyrazolo[3,4-b]pyridin-3-yl)benzamide | | | |

| **30** | **110 and 32** | 14 | 40 |
|---|---|---|---|
| 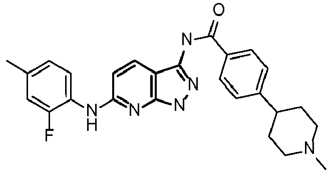 | ¹H NMR (400 MHz, *DMSO-d*6) δ ppm 12.65 (1 H, br. S.), 10.73 (1 H, s), 8.88 (1 H, s), 8.01 (4 H, dd),7.40 (2 H, d), 7.04 - 7.14 (1 H, m), 6.95 - 7.03 (1 H, m), 6.74 (1 H, d), 2.86 - 3.03 (2 H, m), 2.54 - 2.65(1 H, m), 2.22 - 2.37 (6 H, m), 1.99 - 2.18 (2 H, m), 1.92 (3 H, s), 1.62 - 1.84 (4 H, m) | | |
| | ESI+ m/z 459 [M+H]+ | | |
| N-(6-((2-fluoro-4-methylphenyl)amino)-1H-pyrazolo[3,4-b]pyridin-3-yl)-4-(1-methylpiperidin-4-yl)benzamide, Acetic acid salt | | | |

| **31** | **111 and 32** | 14 | 22 |
|---|---|---|---|
| 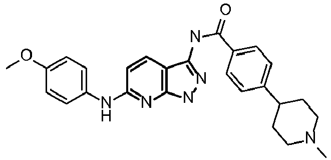 | 1H NMR (400 MHz, *DMSO-d*6) δ ppm 12.60 (1 H, s), 10.71 (1 H, s), 9.17 (1 H, s), 7.94 - 8.05 (3 H, m), 7.72 (2 H, d), 7.40 (2 H, d), 6.91 (2 H, d), 6.57 (1 H, d), 3.75 (3 H, s), 2.87 - 3.00 (2 H, m), 2.56 - 2.65 (1H, m), 2.20 - 2.31 (3 H, m), 1.99 - 2.15 (2 H, m), 1.65 - 1.85 (4 H, m) | | |
| | ESI+ m/z 457 [M+H]+ | | |
| N-(6-((4-methoxyphenyl)amino)-1H-pyrazolo[3,4-b]pyridin-3-yl)-4-(1-methylpiperidin-4-yl)benzamide | | | |

| **32** | **112 and 32** | 14 | 25 |
|---|---|---|---|
| 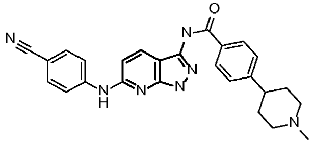 | ¹H NMR (400 MHz, *DMSO-d*6) δ ppm 12.92 (1 H, br. S.), 10.83 (1 H, s), 9.96 (1 H, s), 8.16 (1 H, d),8.04 (4 H, dd), 7.76 (2 H, d), 7.41 (2 H, d), 6.75 (1 H, d), 3.14 - 3.23 (1 H, m), 2.92 - 3.09 (2 H, m), 2.56- 2.70 (1 H, m), 2.04 - 2.41 (4 H, m), 1.92 (2 H, s), 1.64 - 1.87 (4 H, m) | | |
| | ESI+ m/z 452 [M+H]+ | | |
| N-(6-((4-cyanophenyl)amino)-1H-pyrazolo[3,4-b]pyridin-3-yl)-4-(1-methylpiperidin-4-yl)benzamide, Acetic acid salt | | | |

| **33** | **113 and 32** | 14 | 34 |
|---|---|---|---|
| 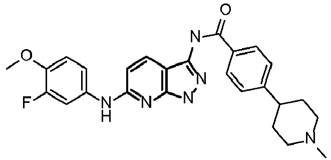 | ¹H NMR (400 MHz, *DMSO-d*6) δ ppm 12.71 (1 H, br. S.), 10.75 (1 H, s), 9.40 (1 H, s), 7.97 - 8.10 (4 H,m), 7.40 (2 H, d), 7.32 - 7.37 (1 H, m), 7.13 (1 H, t), 6.60 (1 H, d), 3.82 (3 H, s), 2.93 - 3.08 (2 H, m),2.56 - 2.67 (1 H, m), 2.33 (4 H, br. S.), 1.92 (2 H, s), 1.67 - 1.87 (4 H, m) | | |
| | ESI+ m/z 475 [M+H]+ | | |
| N-(6-((3-fluoro-4-methoxyphenyl)amino)-1H-pyrazolo[3,4-b]pyridin-3-yl)-4-(1-methylpiperidin-4-yl)benzamide, Acetic acid salt | | | |

| **34** | **100** | 15 | 29 |
|---|---|---|---|
| 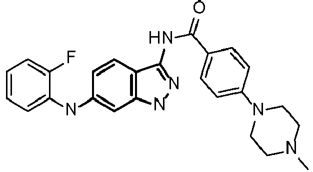 | ¹H NMR (400 MHz, *DMSO-d*6) δ ppm 10.35 (1 H, s), 8.07 (1 H, s), 7.96 (2 H, d), 7.53 - 7.59 (1 H, m), 267.38 (1 H, td), 7.25 (1 H, ddd), 7.11 - 7.17 (1 H, m), 6.97 - 7.06 (3 H, m), 6.81 - 6.86 (2 H, m), 3.28 -3.33 (4 H, m), 2.44 - 2.49 (4 H, m), 2.24 (3 H, s) | | |
| | ESI+ m/z 445 [M+H]+ | | |
| N-(6-((2-fluorophenyl)amino)-1H-indazol-3-yl)-4-(4-methylpiperazin-1-yl)benzamide, Acetic acid salt | | | |

| **35** | **68 and 38** | 14 | 11 |
|---|---|---|---|
| 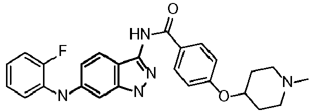 | ¹H NMR (400 MHz, *DMSO-d*6) δ ppm 12.24 (1 H, s), 10.49 (1 H, s), 8.01 - 8.10 (3 H, m), 7.53 - 7.60 (1H, m), 7.38 (1 H, td), 7.25 (1 H, ddd), 7.12 - 7.17 (1 H, m), 7.07 (2 H, d), 6.97 - 7.04 (1 H, m), 6.80 -6.86 (2 H, m), 4.53 (1 H, br. S.), 2.68 (2 H, dt), 2.24 (5 H, br. S.), 1.98 (2 H, d), 1.69 (2 H, d) | | |
| | ESI+ m/z 460 [M+H]+ | | |
| N-(6-((2-fluorophenyl)amino)-1H-indazol-3-yl)-4-((1-methylpiperidin-4-yl)oxy)benzamide | | | |

| **36** | **99** | 15 | 10 |
|---|---|---|---|
| 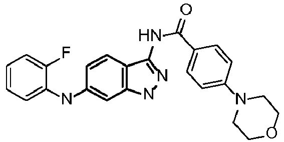 | ¹H NMR (400 MHz, *DMSO-d*6) δ ppm 12.21 (1 H, br. s.), 10.38 (1 H, s), 8.07 (1 H, s), 7.99 (2 H, d),7.56 (1 H, d), 7.38 (1 H, td), 7.25 (1 H, ddd), 7.14 (1 H, t), 6.95 - 7.06 (3 H, m), 6.78 - 6.88 (2 H, m),3.67 - 3.80 (4 H, m), 3.21 - 3.29 (4 H, m) | | |
| | ESI+ m/z 432 [M+H]+ | | |
| N-(6-((2-fluorophenyl)amino)-1H-indazol-3-yl)-4-morpholinobenzamide | | | |

| **37** | **95** | 13 | 78 |
|---|---|---|---|
| 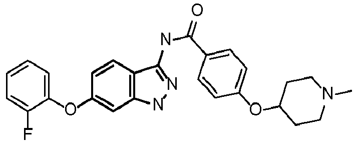 | ¹H NMR (400 MHz, DMSO-d₆) δ ppm 7.04 - 7.15 (2 H, m), 6.87 - 6.98 (2 H, m), 3.76 (2 H, d), 2.96 (2 H, d), 2.42 - 2.49 (2 H, m), 1.80 (1 H, br. s.), 1.68 (2 H, d), 1.09 - 1.22 (2 H, m) | | |
| | ESI+ m/z 461 [M+H]+ | | |
| N-(6-(2-fluorophenoxy)-1H-indazol-3-yl)-4-((1-methylpiperidin-4-yl)oxy)benzamide | | | |

| **38** | **96** | 13 | 22 |
|---|---|---|---|
| 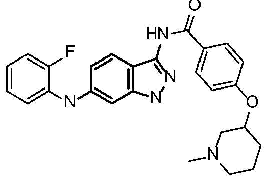 | ¹H NMR (400 MHz, *DMSO-d*6) δ ppm 12.24 (1 H, s), 10.50 (1 H, s), 8.02 - 8.10 (3 H, m), 7.53 - 7.59 (1H, m), 7.38 (1 H, td), 7.25 (1 H, ddd), 7.12 - 7.18 (1 H, m), 7.07 (2 H, d), 6.96 - 7.03 (1 H, m), 6.81 -6.86 (2 H, m), 4.08 (1 H, dd), 3.94 (1 H, dd), 2.98 (1 H, br. S.), 2.57 - 2.65 (1 H, m), 2.39 (3 H, s), 2.17 -2.27 (1 H, m), 1.92 - 2.04 (1 H, m), 1.56 - 1.78 (3 H, m) | | |
| | ESI+ m/z 460 [M+H]+ | | |
| N-(6-((2-fluorophenyl)amino)-1H-indazol-3-yl)-4-((1-methylpiperidin-3-yl)oxy)benzamide | | | |

| **39** | **68 and 33** | 14 | 20 |
|---|---|---|---|
| 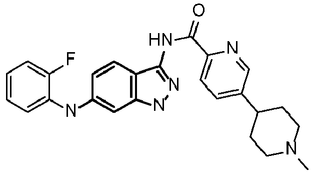 | ¹H NMR (400 MHz, *DMSO-d*6) δ ppm 12.30 (1 H, s), 10.53 (1 H, s), 8.66 (1 H, d), 8.09 - 8.14 (2 H, m),7.97 (1 H, dd), 7.79 (1 H, d), 7.39 (1 H, td), 7.26 (1 H, ddd), 7.10 - 7.18 (1 H, m), 6.97 - 7.05 (1 H, m),6.80 - 6.89 (2 H, m), 2.93 (2 H, d), 2.66 - 2.74 (1 H, m), 2.21 - 2.30 (3 H, m), 2.05 (2 H, t), 1.68 - 1.85 (4H, m) | | |
| | ESI+ m/z 445 [M+H]+ | | |
| N-(6-((2-fluorophenyl)amino)-1H-indazol-3-yl)-5-(1-methylpiperidin-4-yl)picolinamide | | | |

| **40 single unknown enantiomer** | **96** | 13 | 10 |
|---|---|---|---|
| 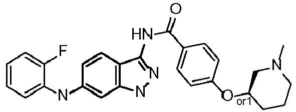 | ¹H NMR (400 MHz, *DMSO-d*6) δ ppm 12.24 (1 H, s), 10.50 (1 H, s), 8.02 - 8.10 (3 H, m), 7.53 - 7.59 (1H, m), 7.38 (1 H, td), 7.25 (1 H, ddd), 7.12 - 7.18 (1 H, m), 7.07 (2 H, d), 6.96 - 7.03 (1 H, m), 6.81 -6.86 (2 H, m), 4.08 (1 H, dd), 3.94 (1 H, dd), 2.98 (1 H, br. S.), 2.57 - 2.65 (1 H, m), 2.39 (3 H, s), 2.17 -2.27 (1 H, m), 1.92 - 2.04 (1 H, m), 1.56 - 1.78 (3 H, m) | | |
| | ESI+ m/z 460 [M+H]+ | | |
| *rel*-(R)-N-(6-((2-fluorophenyl)amino)-1H-indazol-3-yl)-4-((1-methylpiperidin-3-yl)oxy)benzamide | | | |

| **41 single unknown enantiomer** | **96** | 13 | 8 |
|---|---|---|---|
| 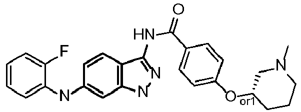 | ¹H NMR (400 MHz, *DMSO-d*6) δ ppm 12.24 (1 H, s), 10.50 (1 H, s), 8.02 - 8.10 (3 H, m), 7.53 - 7.59 (1H, m), 7.38 (1 H, td), 7.25 (1 H, ddd), 7.12 - 7.18 (1 H, m), 7.07 (2 H, d), 6.96 - 7.03 (1 H, m), 6.81 -6.86 (2 H, m), 4.08 (1 H, dd), 3.94 (1 H, dd), 2.98 (1 H, br. S.), 2.57 - 2.65 (1 H, m), 2.39 (3 H, s), 2.17 -2.27 (1 H, m), 1.92 - 2.04 (1 H, m), 1.56 - 1.78 (3 H, m) | | |
| | ESI+ m/z 460 [M+H]+ | | |
| *rel*-(S)-N-(6-((2-fluorophenyl)amino)-1H-indazol-3-yl)-4-((1-methylpiperidin-3-yl)oxy)benzamide | | | |

| **42** | **68 and 41** | 14 | 3 |
|---|---|---|---|
| 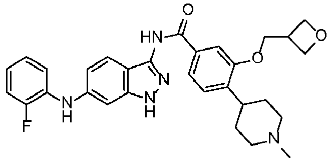 | ¹H NMR (400 MHz, *CHLOROFORM-d*) δ ppm 8.76 (1 H, br. s.), 8.09 (1 H, d), 7.58 (1 H, s), 7.50 (1 H,d), 7.44 (1 H, t), 7.34 (1 H, d), 7.04 - 7.18 (3 H, m), 6.86 - 6.99 (2 H, m), 6.03 (1 H, br. s.), 4.92 (2 H,dd), 4.64 (2 H, t), 4.29 (2 H, d), 3.47 - 3.57 (1 H, m), 3.10 (2 H, d), 2.98 - 3.06 (1 H, m), 2.41 (3 H, s),2.17 - 2.26 (2 H, m), 1.81 -1.92 (4 H, m) | | |
| | ESI+ m/z 530 [M+H]+ | | |
| N-(6-((2-fluorophenyl)amino)-1H-indazol-3-yl)-4-(1-methylpiperidin-4-yl)-3-(oxetan-3-ylmethoxy)benzamide | | | |

| **43** | **97** | 15 | **7** |
|---|---|---|---|
| 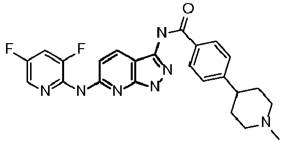 | ¹H NMR (400 MHz, *DMSO-d*6) δ ppm 9.72 - 10.78 (1 H, m), 9.24 - 9.45 (1 H, m), 9.04 - 9.23 (1 H, m),8.06 - 8.25 (1 H, m), 7.71 (3 H, d), 7.35 (2 H, d), 6.14 - 6.28 (1 H, m), 3.71 - 3.88 (1 H, m), 2.89 (2 H,d), 2.56 (1 H, br. S.), 2.21 (3 H, s), 1.93 - 2.04 (2 H, m), 1.74 (4 H, br. S.), 1.05 (2 H, d) | | |
| | ESI+ m/z 464 [M+H]+ | | |
| N-(6-((3,5-difluoropyridin-2-yl)amino)-1H-pyrazolo[3,4-b]pyridin-3-yl)-4-(1-methylpiperidin-4-yl)benzamide | | | |

| **44** | **114 and 32** | 14 | 21 |
|---|---|---|---|
| 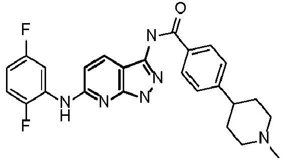 | ¹H NMR (400 MHz, *DMSO-d*6) δ ppm 12.84 (1 H, br. S.), 10.79 (1 H, s), 9.16 - 9.34 (1 H, m), 8.49 - 8.58(1 H, m), 8.10 (1 H, d), 8.01 (2 H, d), 7.41 (2 H, d), 7.25 - 7.34 (1 H, m), 6.96 (1 H, d), 6.77 - 6.87 (1 H,m), 2.84 - 2.96 (2 H, m), 2.55 - 2.63 (1 H, m), 2.23 (3 H, s), 1.97 - 2.07 (2 H, m), 1.91 (3 H, s), 1.63 - 1.83 (4 H, m) | | |
| | ESI+ m/z 463 [M+H]+ | | |
| N-(6-((2,5-difluorophenyl)amino)-1H-pyrazolo[3,4-b]pyridin-3-yl)-4-(1-methylpiperidin-4-yl)benzamide, Acetic acid salt | | | |

| **45 single unknown enantiomer** | **102 and 39** | 14 | 6 |
|---|---|---|---|
| 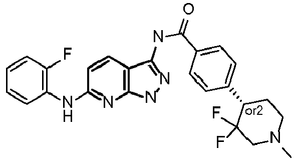 | ¹H NMR (400 MHz, *DMSO-d*6) δ ppm 12.71 (1 H, s), 10.82 (1 H, s), 9.01 (1 H, s), 8.23 - 8.34 (1 H, m),7.97 - 8.11 (3 H, m), 7.42 - 7.52 (2 H, m), 7.15 - 7.32 (2 H, m), 7.01 - 7.13 (1 H, m), 6.78 - 6.87 (1 H,m), 3.07 - 3.21 (2 H, m), 2.87 - 3.00 (1 H, m), 2.30 (3 H, s), 2.06 - 2.22 (2 H, m), 1.76 - 1.89 (1 H, m) | | |
| | ESI+ m/z 481[M+H]+ | | |
| *rel*-(R)-4-(3,3-difluoro-1-methylpiperidin-4-yl)-N-(6-((2-fluorophenyl)amino)-1H-pyrazolo[3,4-b]pyridin-3-yl)benzamide | | | |

| **46 single unknown enantiomer** | **102 and 39** | 14 | 6 |
|---|---|---|---|
| 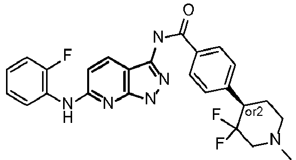 | ¹H NMR (400 MHz, *DMSO-d*6) δ ppm 12.71 (1 H, s), 10.82 (1 H, s), 9.01 (1 H, s), 8.23 - 8.34 (1 H, m),7.97 - 8.11 (3 H, m), 7.42 - 7.52 (2 H, m), 7.15 - 7.32 (2 H, m), 7.01 - 7.13 (1 H, m), 6.78 - 6.87 (1 H,m), 3.07 - 3.21 (2 H, m), 2.87 - 3.00 (1 H, m), 2.30 (3 H, s), 2.06 - 2.22 (2 H, m), 1.76 - 1.89 (1 H, m) | | |
| | ESI+ m/z 481[M+H]+ | | |
| *rel*-(S)-4-(3,3-difluoro-1-methylpiperidin-4-yl)-N-(6-((2-fluorophenyl)amino)-1H-pyrazolo[3,4-b]pyridin-3-yl)benzamide | | | |

| **47** | **102 and 38** | 14 | 50 |
|---|---|---|---|
| 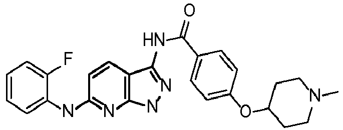 | ¹H NMR (400 MHz, *DMSO-d*6) δ ppm 12.67 (1 H, br. S.), 10.65 (1 H, s), 9.00 (1 H, s), 8.28 (1 H, td),8.04 (3 H, d), 7.26 (1 H, ddd), 7.16 - 7.22(1 H, m), 7.03 - 7.11 (3 H, m), 6.81 (1 H, d), 4.51 (1 H, dt),2.58 - 2.66 (2 H, m), 2.15 - 2.26 (5 H, m), 1.97 (2 H, d), 1.91 (3 H, s), 1.61 - 1.73 (2 H, m) | | |
| | ESI+ m/z 461[M+H]+ | | |
| N-(6-((2-fluorophenyl)amino)-1H-pyrazolo[3,4-b]pyridin-3-yl)-4-((1-methylpiperidin-4-yl)oxy)benzamide, Acetic acid salt | | | |

| **48** | **102 and 4-(4-methylpiperazin-1-yl)benzoic acid** | 14 | 49 |
|---|---|---|---|
| 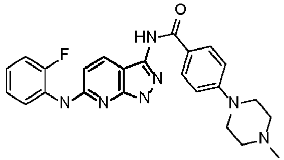 | ¹H NMR (400 MHz, *DMSO-d*6) δ ppm 12.64 (1 H, br. S.), 10.50 (1 H, s), 8.99 (1 H, s), 8.28 (1 H, td),8.04 (1 H, d), 7.97 (2 H, d), 7.26 (1 H, ddd), 7.16 - 7.22 (1 H, m), 7.03 - 7.10 (1 H, m), 7.01 (2 H, d), 6.81 (1 H, d), 3.30 - 3.32 (4 H, m), 2.41 -2.48 (4 H, m), 2.24 (3 H, s) | | |
| | ESI+ m/z 446[M+H]+ | | |
| N-(6-((2-fluorophenyl)amino)-1H-pyrazolo[3,4-b]pyridin-3-yl)-4-(4-methylpiperazin-1-yl)benzamide | | | |

| **49** | **115 and 32** | 14 | **7** |
|---|---|---|---|
| 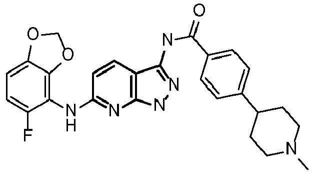 | ¹H NMR (400 MHz, *DMSO-d*6) δ ppm 12.60 (1 H, s), 10.71 (1 H, s), 8.81 (1 H, s), 7.90 - 8.08 (3 H, m),7.36 - 7.44 (2 H, m), 6.71 - 6.81 (2 H, m), 6.55 - 6.61 (1 H, m), 6.03 - 6.08 (2 H, m), 2.85 - 2.93 (2 H,m), 2.56 - 2.62 (2 H, m), 2.18 - 2.25 (3 H, m), 1.94 - 2.03 (2 H, m), 1.91 (2 H, s), 1.66 - 1.81 (4 H, m) | | |
| | ESI+ m/z 489[M+H]+ | | |
| N-(6-((5-fluorobenzo[d][1,3]dioxol-4-yl)amino)-1H-pyrazolo[3,4-b]pyridin-3-yl)-4-(1-methylpiperidin-4-yl)benzamide, Acetic acid salt | | | |

| **50** | **116 and 32** | 14 | 26 |
|---|---|---|---|
| 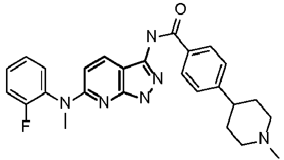 | ¹H NMR (400 MHz, *DMSO-d*6) δ ppm 12.64 - 12.79 (1 H, m), 11.59 - 12.18 (1 H, m), 10.66 - 10.80 (1H, m), 7.97 (3 H, s), 7.46 - 7.56 (1 H, m), 7.38 (5 H, d), 6.17 - 6.28 (1 H, m), 3.42 (3 H, s), 2.82 - 2.95 (2H, m), 2.53 - 2.61 (1 H, m), 2.21 (3 H, s), 1.95 - 2.05 (2 H, m), 1.92 (3 H, s), 1.61 - 1.82 (4 H, m) | | |
| | ESI+ m/z 459[M+H]+ | | |
| N-(6-((2-fluorophenyl)(methyl)amino)-1H-pyrazolo[3,4-b]pyridin-3-yl)-4-(1-methylpiperidin-4-yl)benzamide, Acetic acid salt | | | |

| **51** | **117 and 32** | 14 | 3 |
|---|---|---|---|
| 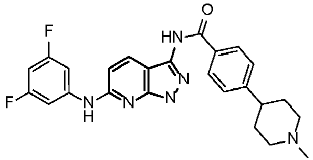 | ¹H NMR (400 MHz, *DMSO-d*6) δ ppm 12.80 - 12.97 (1 H, m), 10.81 (1 H, s), 9.84 (1 H, s), 8.14 (1 H, d),8.01 (2 H, d), 7.64 (2 H, dd), 7.41 (2 H, d), 6.69 - 6.80 (1 H, m), 6.67 (1 H, d), 2.84 - 2.98 (2 H, m), 2.55(3 H, s), 2.22 (3 H, s), 1.95 - 2.06 (2 H, m), 1.91 (1 H, s), 1.63 - 1.82 (4 H, m) | | |
| | ESI+ m/z 463[M+H]+ | | |
| N-(6-((3,5-difluorophenyl)amino)-1H-pyrazolo[3,4-b]pyridin-3-yl)-4-(1-methylpiperidin-4-yl)benzamide | | | |

| **52** | **102 and 36** | 14 | 13 |
|---|---|---|---|
| 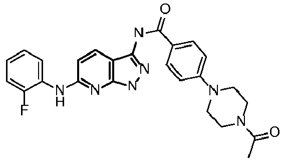 | ¹H NMR (400 MHz, *DMSO-d*6) δ ppm 12.64 (1 H, s), 10.53 (1 H, s), 8.99 (1 H, s), 8.28 (1 H, td), 8.04 (1H, d), 7.99 (2 H, d), 7.26 (1 H, ddd), 7.15 - 7.22 (1 H, m), 7.00 - 7.10 (3 H, m), 6.81 (1 H, d), 3.55 - 3.65(4 H, m), 3.33 - 3.42 (2 H, m), 2.06 (3 H, s) | | |
| | ESI+ m/z 474[M+H]+ | | |
| 4-(4-acetylpiperazin-1-yl)-N-(6-((2-fluorophenyl)amino)-1H-pyrazolo[3,4-b]pyridin-3-yl)benzamide | | | |

**Example 40: Preparation of Compound 53**

### N-(6-((2-fluorophenyl)amino)-1H-indazol-3-yl)-4-(1-methylpiperidin-4-yl)-2-((tetrahydro-2H-pyran-4-yl)amino)benzamide

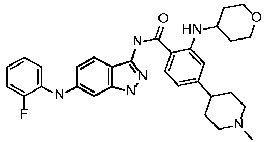

Triethylamine (0.216 ml, 1.550 mmol) was added to a solution of **intermediate 118 (example 38)** (45 mg, 0.070 mmol) in MeOH (2 ml). The reaction medium was heated at 65 °C for 2 hours.

Solvent was evaporated and the residue was loaded onto a RediSep C18aq 30g column eluting with water + 0.1% AcOH only to acetonitrile + 0.1% AcOH only. Relevant fractions were loaded on XC (0.5 g) cartridge washing with MeOH and eluting with NH3/MeOH. The ammonia fractions were evaporated to give title compound (9 mg; 24 % yield). ¹H NMR (400 MHz, *DMSO-d*6) δ ppm: 12.24 (1 H, s), 10.31 (1 H, s), 8.08 (1 H, s), 7.91 (1 H, d), 7.81 (1H, d), 7.48 (1 H, d), 7.38 (1 H, d), 7.23 (1 H, s), 7.14 (1 H, d), 6.93 - 7.05 (1 H, m), 6.81 - 6.90 (2 H, m), 6.68 (1 H, d), 6.50 (1 H, dd), 3.77 - 3.90 (2 H, m), 3.62 - 3.75 (1 H, m), 3.44 - 3.56 (2 H, m), 2.88 (2 H,d), 2.38 - 2.48 (1 H, m), 2.20 (3 H, s), 1.95 (4 H, dd), 1.72 (4 H, dd), 1.37 (2 H, d). ESI+ m/z 543 [M+H]+

### Pharmacological evaluation of the compounds of the invention

Compounds of Formula (I), according to the invention, have been studied in vitro in order to establish their potential FYN, EGFR and VEGFR2 kinases inhibitory activity, and their efficacy in a cellular model for testing kinase inhibition, as well as to establish the efficacy of the best compounds, resulting from the screening in vitro, in experiments of glioblastoma

The obtained results are summarized in the following Tables

### Example 41

### In vitro kinase assay

Assays to determine the kinase inhibitory activity of compounds were performed using an automatic liquid handling device (Microlab STAR Hamilton) and Z'-LYTE™ Kinase Assay Platform (Invitrogen), a Fluorescence Resonance Energy Transfer (FRET)-based assay platform compatible with high-throughput screening (HTS) applications. The assay employs a fluorescence-based, coupled-enzyme format and utilizes the differential sensitivity of phosphorylated and non-phosphorylated peptides to proteolytic cleavage.

Test compounds were evaluated towards active FYN (CarnaBioscience). The enzyme was incubated in a 384 low-volume microplate with a synthetic peptide-substrate, ATP and different inhibitor concentrations, ranging from 10⁻¹⁰ M up to 10⁻⁵ M final concentration. Samples representing the 0% inhibition (or total enzymatic activity) were in the presence of compound diluent (1% DMSO final) in Reaction Buffer (50 mMHepes, 10 mM MgCl2, 1 mM EGTA, 0.01% Brij-35, pH 7.5).

A near-Km ATP concentrations for the kinase and an optimal enzyme concentration that phosphorylates 20-40% of the Z'-LYTE™ Tyr 2 Peptide in a one-hour incubation, were selected. All reagents were diluted in reaction buffer and the kinase reaction was carried out in a total volume of 10 µl, for 60 minutes at 25 °C. The 0% Phosphorylation (i.e. no ATP) and 100% Phosphorylation (i.e. synthetically phosphorylated peptide supplied) Assay Controls, included in each plate, allowed to calculate the percent phosphorylation achieved in the specific reaction well. The 0% Inhibition and 0% Phosphorylation (i.e. 100% Inhibition) Controls define the dynamic range in the screen. At the end of incubation, a secondary reaction (the Development Reaction) started by adding 5 µl of Development Reagent, containing a site-specific protease that recognizes and cleaves non-phosphorylated peptides, and was interrupted after 60 minutes with 5 µl/sample of Stop Reagent. Measurement of the Coumarin (Ex. 400 nm, Em. 460 nm) and Fluorescein (Ex. 400 nm, Em. 535 nm) emission signals were performed by a fluorescence plate reader (Envision, PerkinElmer).

Results are expressed as percentage of inhibition and the IC₅₀ values were calculated by non- linear curve fitting using GraphPadTM Prism software.

The FYN kinase inhibitory activity of representative compounds of Formula (I) is reported in the following table.

**Table 17: FYN KINASE INHIBITION**

| **Compound** | **IC₅₀(nM)** | **Compound** | **IC₅₀(nM)** | **Compound** | **IC₅₀(nM)** |
|---|---|---|---|---|---|
| 1 | 104 | 23 | 111 | 45 | 1000 |
| 2 | 315 | 24 | 209 | 46 | 1000 |
| 3 | 307 | 25 | 238 | 47 | 513 |
| 4 | 203 | 26 | 260 | 48 | 212 |
| 5 | 69 | 27 | 538 | 49 | 831 |
| 6 | 1000 | 28 | 72 | 50 | 1000 |
| 7 | 468 | 29 | 95 | 51 | 1000 |
| 8 | 99 | 30 | 115 | 52 | 168 |
| 9 | 388 | 31 | 589 | 53 | 201 |
| 10 | 650 | 32 | 356 | | |
| 11 | 47 | 33 | 103 | | |
| 12 | 220 | 34 | 147 | | |
| 13 | 207 | 35 | 145 | | |
| 14 | 82 | 36 | 370 | | |
| 15 | 19 | 37 | 388 | | |
| 16 | 97 | 38 | 283 | | |
| 17 | 87 | 39 | 181 | | |
| 18 | 105 | 40 | 548 | | |
| 19 | 258 | 41 | 374 | | |
| 20 | 209 | 42 | 204 | | |
| 21 | 442 | 43 | 1000 | | |
| 22 | 283 | 44 | 1000 | | |

In this assay, several compounds emerged as inhibitors with IC50 in the nanomolar concentration range. In particular, compounds 5, 11, 15 and 28 were the more active.

### Example 42

### In vitro kinase assay

The ADP-Glo™ Kinase Assay (Promega), monitoring ADP produced in the kinase reaction, was performed in two steps. In the first one, after the kinase reaction, the ADP-Glo™ Reagent is added to terminate the kinase reaction and deplete the remaining ATP. In the second step, the Kinase Detection Reagent is added to simultaneously convert ADP to ATP (ATPn or newly synthesized ATP) and introduce luciferase and luciferin, allowing the ATPn to be measured (luciferase/luciferin reaction). The amount of light generated correlates with the amount of ATPn (indirectly with the ADP produced in kinase reaction).

Kinase reaction was performed in 10 µl in a white 384-well plate, inhibitor solutions in DMSO / Kinase buffer (40 mM Tris-CI, 20mM MgCl2, 0.1mg/ml BSA, 50 µM DTT, pH 7.5) and EGFR kinase in Kinase buffer (SignalChem, 2.5 ng/µl final concentration in assay) were pre-incubate 15 minutes at room temperature. At the kinase concentration selected a signal-to negative control ratio > 10 was calculated, according to supplier indication. In samples representing the Total enzymatic activity (no inhibitor) or the negative control (no enzyme), inhibitor solution was replaced by diluents (1% DMSO in kinase buffer, final concentration) or ultra-pure water, respectively.

The range of inhibitor concentrations was from 10⁻¹¹ M up to 3 x 10⁻⁶ M (final concentration in assay) and each experimental point was performed in duplicate.

To start the reaction, a substrate/ATP mix (ATP 10 µM final concentration, near to ATP Km value) was added to each sample and after 30 minutes incubation at room temperature, ADP-Glo™ Reagent and Kinase Detection Reagent, incubated in sequence according to the respective incubation time and supplier indication, allowed to stop and develop the kinase reaction. The luminescence was measured with a plate-reading luminometer (Envision 2104 Multilabel reader, PerkinElmer). The percentage of inhibition was calculated towards the Total enzymatic activity and the corresponding curves were analyzed by non linear curve fitting (GraphPad software, version 7 for Windows), allowing to calculate the IC₅₀ value ± standard error of the estimate.

**Table 18: EGFR KINASE INHIBITION**

| **Compound** | **IC₅₀(nM)** | **Compound** | **IC₅₀(nM)** |
|---|---|---|---|
| 1 | 102 | 29 | 1000 |
| 5 | 6 | 30 | 155 |
| 8 | 1000 | 32 | 1000 |
| 14 | 1000 | 37 | 1000 |
| 17 | 225 | 39 | 1000 |
| 20 | 9 | 47 | 24 |
| 23 | 1000 | 48 | 15 |
| 28 | 1000 | | |

In this assay, compounds 1, 5, 20, 47 and 48 emerged as the more active.

### Example 43

### In vitro kinase assay

The ADP-Glo™ Kinase Assay (Promega), monitoring ADP produced in the kinase reaction, was performed in two steps. In the first one, after the kinase reaction, the ADP-Glo™ Reagent is added to terminate the kinase reaction and deplete the remaining ATP. In the second step, the Kinase Detection Reagent is added to simultaneously convert ADP to ATP (ATPn or newly synthesized ATP) and introduce luciferase and luciferin, allowing the ATPn to be measured (luciferase/luciferin reaction). The amount of light generated correlates with the amount of ATPn (indirectly with the ADP produced in kinase reaction).

Kinase reaction was performed in 10 µl in a white 384-well plate, inhibitor solutions in DMSO / Kinase buffer (40 mM Tris-CI, 20mM MgCl2, 0.1mg/ml BSA, 50 µM DTT, pH 7.5) and VEGFR2 (KDR) kinase in Kinase buffer (SignalChem, 0.5 ng/µl final concentration in assay) were pre-incubate 15 minutes at room temperature. At the kinase concentration selected, a signal-to negative control ratio > 10 was calculated, according to supplier indication. In samples representing the Total enzymatic activity or the negative control (no enzyme), inhibitor solution was replaced by diluents (1% DMSO in kinase buffer, final concentration) or ultra-pure water, respectively.

The range of inhibitor concentrations was from 10⁻¹¹ M up to 3 x 10⁻⁶ M (final concentration in assay) and each experimental point was performed in duplicate. To start the reaction, a substrate/ATP mix (50 µM final concentration, near to ATP Km value) was added to each sample and after 30 minutes incubation at room temperature, ADP-Glo™ Reagent and Kinase Detection Reagent, incubated in sequence according to the respective incubation time and supplier indication, allowed to stop and develop the kinase reaction. The luminescence was measured with a plate-reading luminometer (Envision 2104 Multilabel reader, PerkinElmer). The percentage of inhibition was calculated towards the Total enzymatic activity and the corresponding curves were analyzed by non linear curve fitting (GraphPad software, version 7 for Windows), allowing to calculate the IC₅₀ value ± standard error of the value

**Table 19: VEGFR2 KINASE INHIBITION**

| **Compound** | **IC₅₀(nM)** | **Compound** | **IC₅₀(nM)** |
|---|---|---|---|
| 1 | 105 | 29 | 1 |
| 5 | 82 | 30 | 1 |
| 8 | 126 | 32 | 3 |
| 14 | 58 | 37 | 437 |
| 17 | 222 | 39 | 533 |
| 20 | 105 | 47 | 12 |
| 23 | 2.6 | 48 | 1 |
| 28 | 1 | | |

As Tables 19 shows, the more potent VEGFR2 kinase inhibitors of Formula (I) resulted to be compounds 23, 28, 29, 30, 32, 47 and 48. Advantageous compounds hence are those with pyrazolo-pyridinic core.

### Example 44

### In vitro kinase assay

The ADP-Glo™ Kinase Assay (Promega), monitoring ADP produced in the kinase reaction, was performed in two steps. In the first one, after the kinase reaction, the ADP-Glo™ Reagent is added to terminate the kinase reaction and deplete the remaining ATP. In the second step, the Kinase Detection Reagent is added to simultaneously convert ADP to ATP (ATPn or newly synthesized ATP) and introduce luciferase and luciferin, allowing the ATPn to be measured (luciferase/luciferin reaction). The amount of light generated correlates with the amount of ATPn (indirectly with the ADP produced in kinase reaction).

Kinase reaction was performed in 10 µl in a white 384-well plate, inhibitor solutions in DMSO / Kinase buffer (40 mM Tris-CI, 20mM MgCl2, 0.1mg/ml BSA, 50 µM DTT, pH 7.5) and Yes1 kinase in Kinase buffer (Thermo Fisher, 0.5 ng/µl final concentration in assay) were pre-incubate 15 minutes at room temperature. At the kinase concentration selected, a signal-to negative control ratio > 10 was calculated, according to supplier indication. In samples representing the Total enzymatic activity or the negative control (no enzyme), inhibitor solution was replaced by diluents (1% DMSO in kinase buffer, final concentration) or ultra-pure water, respectively.

The range of inhibitor concentrations was from 10⁻¹⁰ M up to 10⁻⁵ M (final concentration in assay) and each experimental point was performed in duplicate. To start the reaction, a substrate / ATP (50 µM final concentration, near to ATP Km value) mix was added to each sample and after 30 minutes incubation at room temperature, ADP-Glo™ Reagent and Kinase Detection Reagent, incubated in sequence according to the respective incubation time and supplier indication, allowed to stop and develop the kinase reaction. The luminescence was measured with a plate-reading luminometer (Envision 2104 Multilabel reader, PerkinElmer). The percentage of inhibition was calculated towards the Total enzymatic activity and the corresponding curves were analyzed by non linear curve fitting (GraphPad software, version 7 for Windows), allowing to calculate the IC₅₀ value. Compound 5 inhibited Yes1 in a concentration dependent way (IC₅₀ 3.6 nM).

### Example 45:

### Inhibition of FynB-induced Tau(Y18)-phosphorylation (cell-based assay)

HEK293 cells (Human Embryonic Kidney 293) were seeded at 6x10⁵ in poly-D-lysine 12w microplates and grown adherent in medium DMEM/10%FBS at 37°C with 5% CO₂. After 24h, cells were transfected in order to over-express either constitutively active form of human FynB and Tau protein (isoform 0N4R), through an optimized Lipofectamine®3000 Transfection protocol (Life Technologies™). A non-transfected sample (negative control) was included in every experiment.

Twenty-four hours from transfection, treatments of cells were performed by adding fresh medium containing diluent (DMSO 0.1% final concentration) or test compounds. The incubation was carried out for 6 or 24 hours at 37°C with 5% CO₂. All experimental conditions were performed in duplicate.

At the end of the incubation, the medium was removed and cellular lysates were obtained by adding M-PER lysis buffer containing a 1x Protease/Phosphatase inhibitors cocktail. Then, lysates were transferred into Eppendorf tubes, sonicated and stored at -80°C.

Protein content was measured by Bradford method. Lysates were analyzed by a customized ELISA assay for Tau phosphorylation (Tyrosine 18 residue - Fyn mediated) and Total amount determination. The net OD values were used to calculate the inhibitory effect of compounds.

**Table 20: Inhibition of FynB-induced Tau(Y18)-phosphorylation**

| **Compound** | **IC₅₀(nM)** | **Compound** | **IC₅₀(nM)** | **Compound** | **IC₅₀(nM)** |
|---|---|---|---|---|---|
| 1 | 1000 | 22 | 1000 | 36 | 1000 |
| 4 | 1000 | 23 | 230 | 39 | 1000 |
| 5 | 300 | 24 | 1000 | 42 | 1000 |
| 7 | 1000 | 25 | 1770 | 47 | 1000 |
| 8 | 1000 | 26 | 1000 | 48 | 259 |
| 11 | 1000 | 28 | 100 | 52 | 646 |
| 14 | 1000 | 29 | 1000 | 53 | 1000 |
| 15 | 1000 | 30 | 669 | | |
| 17 | 1000 | 33 | 1851 | | |
| 20 | 234 | 34 | 1000 | | |
| 21 | 1000 | 35 | 1000 | | |

In this cell test, the compounds 5, 20, 23, 28 and 48 emerged as the best inhibitors.

### Example 46:

### Inhibition of EGF-induced EGFR (Y1068)-phosphorylation (cell-based assay)

U87MG cells (Uppsala 87 Malignant Glioma, a human glioblastoma cell line from ATCC) were plated at 3x10⁵ cells/well in 12w microplates and grown adherent in medium DMEM + 10% FBS at 37°C with 5% CO₂. After 24h, medium was removed and starvation was induced overnight in medium DMEM + 0.1%FBS (medium starvation). At the end of incubation, treatment of cells was performed by medium replacement and by adding fresh medium containing or not diluent or test compounds (DMSO 0.1% final concentration) diluted in medium starvation. After 1 hour, EGF stimuli (diluted in medium starvation, 10 ng/ml final concentration) was added to each sample, with the exception of basal sample; plate was then incubated for 15 min at 37°C with 5% CO₂. At the end of the incubation, the medium was removed and cellular lysates were obtained by adding M-PER lysis buffer containing 1x Protease/Phosphatase inhibitors cocktail. Then, lysates were transferred into Eppendorf tubes, sonicated and stored at -80°C.

Protein content was measured by Bradford method. Lysates were analyzed by Western blot for EGFR phosphorylation (Tyrosine 1068) and total receptor amount determination. Densitometric analysis of sample lanes were performed by ImageQuant TL software (GE Healthcare Life Sciences). Each sample value was normalized by respective actin; resulting data were used to calculate inhibitory effect of compounds with respect to stimuli control (absence of inhibitor). Compound 5 inhibited EGFR-phosphorylation in a concentration dependent way (IC₅₀ of 3 µM).

### Example 47:

### Inhibition of VEGF-induced VEGFR (Y1175)-phosphorylation (cell-based assay)

HUVEC-C cells (Human Umbilical Vein Endothelial Cells from ATCC) were plated at 6x10⁴ cells/well in 24w microplates and grown adherent in medium F12K + 0.1 mg/ml Heparin + 0.05 mg/ml ECGS + 10% FBS at 37°C with 5% CO₂. After 24h, medium was removed and starvation was induced overnight in medium F12K + 0.1 mg/ml Heparin + 0.5% FBS (medium starvation). At the end of incubation, treatment of cells was performed by medium replacement and by adding fresh medium containing or not diluent or test compounds (DMSO 0.1% final concentration) diluted in medium starvation. After 1 hour, VEGF stimuli (diluted in medium starvation, 50 ng/ml final concentration) was added to each sample, with the exception of basal sample; plate was then incubated for 5 min at 37°C with 5% CO₂. At the end of the incubation, the medium was removed and cellular lysates were obtained by adding M-PER lysis buffer containing 1x Protease/Phosphatase inhibitors cocktail. Then, lysates were transferred into Eppendorf tubes, sonicated and stored at -80°C.

Lysates were analyzed by Western blot for VEGFR phosphorylation (Tyrosine 1175) and total receptor amount determination. Densitometric analysis of sample lanes were performed by ImageQuant TL software (GE Healthcare Life Sciences). Each sample value was normalized by respective actin; resulting data were used to calculate inhibitory effect of compounds with respect to stimuli control (absence of inhibitor). Compound 5 showed a maximal inhibitory activity on VEGFR-phosphorylation starting from the concentration of 1 µM.

### Example 48:

### Cell viability after treatment with the compounds of invention on different cell lines:

U87MG: a human glioblastoma astrocytoma from ATCC. Grown in DMEM medium supplemented with 10% FBS at 37°C with CO₂.

U87MG vIII (4.12): a permanent line established from U87MG cell line. U87MG vIII (4.12) cell line stably expressed high levels of EGF mutant receptor vIII and G418-resistance gene introduced by plasmid's stable transfection (Celther). Grown adherent in MEM medium supplemented with 10% FBS, 1% Pen-Strep, 200 µg/ml G418 at 37°C with CO2.

U373: a human glioblastoma astrocytoma from ECACC, also known as U373 MG (Uppsala). Grown adherent in DMEM medium supplemented with 10% FBS at 37°C with CO₂.

T98G: a human glioblastoma multiforme from ATCC. Grown adherent in DMEM medium supplemented with 10% FBS at 37°C with CO₂.

Cell viability and total cell count was performed with ViaCount Assay on Guava® Systems, which was able to distinguish viable and non-viable cells based on differential permeabilities of two DNA-binding dyes in the Guava ViaCount® Reagent. 2x10⁴ cancer cells were plated in 24well in DMEM 10%. After 72h cells were treated with the different compounds for 24, 72h or 7 days, with a medium change and a second treatment with the product on day 4. At the end of the incubation period the analysis was done following the kit instructions.

The results obtained are summarized in the following Table 21.

**Table 21: cell viability**

| **Compounds** | **U87MG µM** | **U373 µM** |
|---|---|---|
| **5** | 1-3 | 1-3 |
| **17** | 1-10 | 1-10 |
| **20** | 1-10 | 1-10 |
| **23** | 1-5 | 1-5 |
| **24** | 1-10 | 1-10 |
| **28** | 1-10 | 1-10 |
| **29** | 1-10 | 1-10 |
| **30** | 1-10 | 1-10 |
| **32** | 1-10 | 1-10 |
| **48** | 1-5 | 1-5 |

As Table 18 shows, some compounds of the invention, particularly the compounds of the example 5, 23 and 28 are active in reducing cell viability of the tested cell lines.

### Example 49:

### Determination of PK of the invention compounds

The pharmacokinetics of compounds 17 and 5, were studied CD1 mice. The mice were treated intravenously and orally (n=3 for each dose route) with compounds formulated as solutions. The rats were fitted with a jugular cannula for serial sampling. A full profile was acquired from each rat. Plasma extracts were quantitatively analyzed using a specific and sensitive LC-MS/MS bioanalytical method. Inter-individual variations between the three rats in each group were limited. After intravenous injection all compounds showed moderate volume of distribution (Vss). A range of clearance values were obtained for the different structure, ranging from low to moderate values. After oral administration, absorption was quite fast with a clear maximum concentration reached by the first sampling time-point of 15 minutes for all compounds. The absolute oral bioavailability was good for both compounds with F% around or in excess of 50%. The brain penetration of compound 5 was relevant, with a measured B/P (brain to plasma ratio) in CD1 mice of 1.2 calculated on AUC iv and 1.4 calculated on AUC po.

**Table 22: PK parameters of selected compounds**

| Cmpds | Route | Dose (mg/kg) | CLp (ml/min/Kg ) | Vz (L/Kg ) | t1/2* (h) | AUC 0-t (ng.h/ml) | AUC inf (ng.h/ml) | F% | Cmax (µM) | Tmax (h) | Tlast (h) |
|---|---|---|---|---|---|---|---|---|---|---|---|
| 17 | iv | 3 | 47.7 | 22.8 | 5.5 | 1349 | 1395 | | | | 24 |
| | po | 3 | | | 4.5 | 712 | 726 | 53 | 0.178 | 1 | 24 |
| 5 | iv | 3 | 30.4 | 10.2 | 3.9 | 1624 | 1643 | | | | 24 |
| | po | 3 | | | 4.0 | 1167 | 1183 | 72 | 0.331 | 2 | 24 |

### Example 50:

### Reduction of tumor growth in U87 Glioblastoma bearing mice treated with compound 5 of invention

Human U87MG cells (Glioblastoma Cancer cell line) were engineered to express the luciferase gene (U87MG-luc). Cultured U87MG-luc tumor cells were intracranially injected in athymic Nude mice. Eight days from injection mice were randomised on the basis of luminescence values emitted by luciferase-expressing U87M-luc tumor cells and divided into 2 treatment groups. The treatment started on day 9 and lasted 10 days. Mice were orally dosed with vehicle or with compound 5 of invention (50mg/kg, once a day). The tumor growth was evaluated by real-time Bioluminescence Imaging at the end of the treatments period (day 19) and on day 26 and 33, form the cell injection. The tumour growth was reduced in mice treated with compound 5 compared to mice treated with vehicle alone. The inhibition of tumour growth (percentage of radiance inhibition) evaluated at each time points was 24.9%, 34.4%, and 60.4% on day 19, 26, and 33, respectively.

### Example 51:

### Increased survival time of Glioblastoma bearing mice treated with compound 5 of invention

Human U87MG cancer cell line were implanted in athymic Nude mice by intracranial injection. Mice, divided into two groups, were orally dosed for 10 days either with vehicle or with Compound 5 (50mg/kg, once a day) from the day 9 until day 19 after injection of tumour cells. The animal survival was tracked over time, mice were monitored for mortality and sacrificed at appearance of signs of distress, neurological deficits, seizure, or significant body weight loss (e.g.>20%). The percent survival in U87MG xenograft model is provided in Figure 1. Mice treated with vehicle had a median survival time of 56 days from tumour cell injection. Mice treated with compound 5 had a median survival time of 70 days from tumour cell injection. The group treated with Compound 5 was significantly different (p<0.05) from vehicle control group.

## Claims

1. A compound of Formula (I): 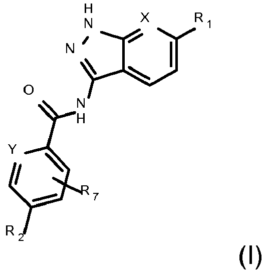 or a salt thereof,
wherein:
X is C or N;
Y is C or N;
R₁ is a substituent selected from the group consisting of: 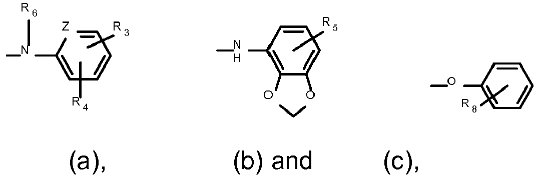
Where
Z is C or N
R₃ and R₄ are, independently from each other, selected from the group consisting of hydrogen, halogen, cyano, (C₁-C₃)alkyl, (C₁-C₃)alkoxy and trifluoromethyl,
R₅ is hydrogen or fluorine,
R₆ is hydrogen or (C₁-C₃)alkyl,
R₈ is hydrogen or fluorine,
R₂ is a heterocyclic substituent selected from the group consisting of:
piperidinyl, piperazinyl, morpholinyl, 4-piperidinyloxy and 3-piperidinyloxy wherein said heterocyclic substituent is
optionally N-substituted with a substituent independently selected from (C₁-C₃) alkyl, acetyl, 2-methoxyethyl and 3-oxethanyl, and
optionally C-substituted with one or more fluorine;
R₇ is H, 4-tetrahydropyranylamino or 3-oxethanylmethylenoxy.

2. The compound of claim 1 wherein X is C.

3. The compound of claim 1 wherein X is N.

4. The compound of claim 2 or 3 wherein Y is C.

5. The compound of anyone of claims 1-4, wherein R₁ is the substituent (a) wherein Z is carbon or the substituent (b), preferably the substituent (a) wherein Z is carbon.

6. The compound of anyone of claims 1-5, wherein R₂ is piperidinyl, piperazinyl or 4-piperidinyloxy, preferably piperidinyl.

7. The compound of claim 1, selected from the group consisting of:
N-(6-((2-chloro-6-fluorophenyl)amino)-1H-indazol-3-yl)-4-(1-methylpiperidin-4-yl)benzamide;
N-(6-((2-ethyl-6-fluorophenyl)amino)-1H-indazol-3-yl)-4-(1-methylpiperidin-4-yl)benzamide;
4-((3S,4R)-3-fluoro-1-methylpiperidin-4-yl)-N-(6-((2-fluorophenyl)amino)-1H-indazol-3-yl)benzamide;
4-((3R,4S)-3-fluoro-1-methylpiperidin-4-yl)-N-(6-((2-fluorophenyl)amino)-1H-indazol-3-yl)benzamide;
N-(6-((2-fluorophenyl)amino)-1H-pyrazolo[3,4-b]pyridin-3-yl)-4-(1-methylpiperidin-4-yl)benzamide;
N-(6-((4-cyano-2-fluorophenyl)amino)-1H-indazol-3-yl)-4-(1-methylpiperidin-4-yl)benzamide;
N-(6-((2-fluorophenyl)amino)-1H-indazol-3-yl)-4-(1-(oxetan-3-yl)piperidin-4-yl)benzamide;
N-(6-((2-fluoro-4-methylphenyl)amino)-1H-indazol-3-yl)-4-(1-methylpiperidin-4-yl)benzamide;
N-(6-((2-chloro-4-fluorophenyl)amino)-1H-indazol-3-yl)-4-(1-methylpiperidin-4-yl)benzamide;
4-(1-methylpiperidin-4-yl)-N-(6-((2-(trifluoromethyl)phenyl)amino)-1H-indazol-3-yl)benzamide;
N-(6-((2-fluoro-6-methylphenyl)amino)-1H-indazol-3-yl)-4-(1-methylpiperidin-4-yl)benzamide;
N-(6-((4-chloro-2-fluorophenyl)amino)-1H-indazol-3-yl)-4-(1-methylpiperidin-4-yl)benzamide;
N-(6-((2-chloro-6-methylphenyl)amino)-1H-indazol-3-yl)-4-(1-methylpiperidin-4-yl) benzamide;
N-(6-((2-chlorophenyl)amino)-1H-indazol-3-yl)-4-(1-methylpiperidin-4-yl)benzamide;
N-(6-((5-fluorobenzo[d][1,3]dioxol-4-yl)amino)-1H-indazol-3-yl)-4-(1-methylpiperidin-4-yl)benzamide;
N-(6-((2,4-difluorophenyl)amino)-1H-indazol-3-yl)-4-(1-methylpiperidin-4-yl)benzamide;
N-(6-((2-fluorophenyl)amino)-1H-indazol-3-yl)-4-(1-methylpiperidin-4-yl)benzamide;
4-(1-methylpiperidin-4-yl)-N-(6-(p-tolylamino)-1H-indazol-3-yl)benzamide;
N-(6-((3-fluorophenyl)amino)-1H-indazol-3-yl)-4-(1-methylpiperidin-4-yl)benzamide;
N-(6-((2,3-difluorophenyl)amino)-1H-pyrazolo[3,4-b]pyridin-3-yl)-4-(1-methylpiperidin-4-yl)benzamide;
N-(6-((2-fluoro-6-methylphenyl)amino)-1H-pyrazolo[3,4-b]pyridin-3-yl)-4-(1-methylpiperidin-4-yl)benzamide;
N-(6-((2,4-difluorophenyl)amino)-1H-pyrazolo[3,4-b]pyridin-3-yl)-4-(1-methylpiperidin-4-yl)benzamide;
4-(1-methylpiperidin-4-yl)-N-(6-(p-tolylamino)-1H-pyrazolo[3,4-b]pyridin-3-yl) benzamide;
N-(6-((2-fluorophenyl)amino)-1H-pyrazolo[3,4-b]pyridin-3-yl)-4-(1-isopropylpiperidin-4-yl)benzamide;
N-(6-((2-fluorophenyl)amino)-1H-pyrazolo[3,4-b]pyridin-3-yl)-4-(1-(2-methoxyethyl) piperidin-4-yl)benzamide;
N-(6-((2-chlorophenyl)amino)-1H-pyrazolo[3,4-b]pyridin-3-yl)-4-(1-methylpiperidin-4-yl)benzamide;
N-(6-((2-methoxyphenyl)amino)-1H-pyrazolo[3,4-b]pyridin-3-yl)-4-(1-methylpiperidin-4-yl)benzamide;
N-(6-(benzo[d][1,3]dioxol-5-ylamino)-1H-pyrazolo[3,4-b]pyridin-3-yl)-4-(1-methylpiperidin-4-yl)benzamide;
4-(1-methylpiperidin-4-yl)-N-(6-(phenylamino)-1H-pyrazolo[3,4-b]pyridin-3-yl) benzamide;
N-(6-((2-fluoro-4-methylphenyl)amino)-1H-pyrazolo[3,4-b]pyridin-3-yl)-4-(1-methylpiperidin-4-yl)benzamide;
N-(6-((4-methoxyphenyl)amino)-1H-pyrazolo[3,4-b]pyridin-3-yl)-4-(1-methylpiperidin-4-yl)benzamide;
N-(6-((4-cyanophenyl)amino)-1H-pyrazolo[3,4-b]pyridin-3-yl)-4-(1-methylpiperidin-4-yl)benzamide;
N-(6-((3-fluoro-4-methoxyphenyl)amino)-1H-pyrazolo[3,4-b]pyridin-3-yl)-4-(1-methylpiperidin-4-yl)benzamide;
N-(6-((2-fluorophenyl)amino)-1 H-indazol-3-yl)-4-(4-methylpiperazin-1-yl)benzamide;
N-(6-((2-fluorophenyl)amino)-1H-indazol-3-yl)-4-((1-methylpiperidin-4-yl)oxy)benzamide;
N-(6-((2-fluorophenyl)amino)-1H-indazol-3-yl)-4-morpholinobenzamide;
N-(6-(2-fluorophenoxy)-1H-indazol-3-yl)-4-((1-methylpiperidin-4-yl)oxy)benzamide;
N-(6-((2-fluorophenyl)amino)-1H-indazol-3-yl)-4-((1-methylpiperidin-3-yl)oxy)benzamide;
N-(6-((2-fluorophenyl)amino)-1H-indazol-3-yl)-5-(1-methylpiperidin-4-yl)picolinamide;
rel-(R)-N-(6-((2-fluorophenyl)amino)-1H-indazol-3-yl)-4-((1-methylpiperidin-3-yl) oxy)benzamide;
rel-(S)-N-(6-((2-fluorophenyl)amino)-1H-indazol-3-yl)-4-((1-methylpiperidin-3-yl) oxy)benzamide;
N-(6-((2-fluorophenyl)amino)-1H-indazol-3-yl)-4-(1-methylpiperidin-4-yl)-3-(oxetan-3-ylmethoxy)benzamide;
N-(6-((3,5-difluoropyridin-2-yl)amino)-1H-pyrazolo[3,4-b]pyridin-3-yl)-4-(1-methylpiperidin-4-yl)benzamide;
N-(6-((2,5-difluorophenyl)amino)-1H-pyrazolo[3,4-b]pyridin-3-yl)-4-(1-methylpiperidin-4-yl)benzamide;
rel-(R)-4-(3,3-difluoro-1-methylpiperidin-4-yl)-N-(6-((2-fluorophenyl)amino)-1H-pyrazolo[3,4-b]pyridin-3-yl)benzamide;
rel-(S)-4-(3,3-difluoro-1-methylpiperidin-4-yl)-N-(6-((2-fluorophenyl)amino)-1 H-pyrazolo[3,4-b]pyridin-3-yl)benzamide;
N-(6-((2-fluorophenyl)amino)-1H-pyrazolo[3,4-b]pyridin-3-yl)-4-((1-methylpiperidin-4-yl)oxy)benzamide;
N-(6-((2-fluorophenyl)amino)-1H-pyrazolo[3,4-b]pyridin-3-yl)-4-(4-methylpiperazin-1-yl)benzamide;
N-(6-((5-fluorobenzo[d][1,3]dioxol-4-yl)amino)-1H-pyrazolo[3,4-b]pyridin-3-yl)-4-(1-methylpiperidin-4-yl)benzamide;
N-(6-((2-fluorophenyl)(methyl)amino)-1H-pyrazolo[3,4-b]pyridin-3-yl)-4-(1-methylpiperidin-4-yl)benzamide;
N-(6-((3,5-difluorophenyl)amino)-1H-pyrazolo[3,4-b]pyridin-3-yl)-4-(1-methylpiperidin-4-yl)benzamide;
4-(4-acetylpiperazin-1-yl)-N-(6-((2-fluorophenyl)amino)-1H-pyrazolo[3,4-b]pyridin-3-yl)benzamide; and
N-(6-((2-fluorophenyl)amino)-1H-indazol-3-yl)-4-(1-methylpiperidin-4-yl)-2-((tetrahydro-2H-pyran-4-yl)amino)benzamide.

8. The compound according to claim 7 selected from the group consisting of:
N-(6-((2-chloro-6-fluorophenyl)amino)-1H-indazol-3-yl)-4-(1-methylpiperidin-4-yl) benzamide
N-(6-((2-fluorophenyl)amino)-1H-pyrazolo[3,4-b]pyridin-3-yl)-4-(1-methylpiperidin-4-yl)benzamide
N-(6-((2,3-difluorophenyl)amino)-1H-pyrazolo[3,4-b]pyridin-3-yl)-4-(1-methylpiperidin-4-yl)benzamide
N-(6-(benzo[d][1,3]dioxol-5-ylamino)-1H-pyrazolo[3,4-b]pyridin-3-yl)-4-(1-methylpiperidin-4-yl)benzamide
N-(6-((2-fluoro-4-methylphenyl)amino)-1H-pyrazolo[3,4-b]pyridin-3-yl)-4-(1-methylpiperidin-4-yl)benzamide, and
N-(6-((2-fluorophenyl)amino)-1H-pyrazolo[3,4-b]pyridin-3-yl)-4-((1-methylpiperidin-4-yl)oxy)benzamide.
4-(1-methylpiperidin-4-yl)-N-(6-(6-tolylamino)-1H-pyrazolo[3,4-b]pyridin-3-yl) benzamide

9. A pharmaceutical composition comprising a compound of anyone of claims 1-8 or its salt and a pharmaceutically acceptable carrier.

10. A compound of Formula (I) or its salt of anyone of claims 1-8 for use as a medicament.

11. A compound of Formula (I) or its salt of anyone of claims 1-8 for the use in the inhibition of at least one of PTK (protein tyrosine kinase).

12. The compound for use of claim 11, wherein the least one of PTKs (protein tyrosin kinase) is selected from FYN, EGFR, VEGFR2 (KDR) and YES1.

13. A compound of Formula (I) or its salt of anyone of claims 1-8 for use in the treatment of at least one of PTK (protein tyrosine kinase)-mediated disease or disorder.

14. The compound for use of claim 13 wherein the at least one of PTK (protein tyrosine kinase)-mediated disease or disorder is a tumour.

15. The compound for use of claim 14, wherein the tumour is glioblastoma.
